# EUROPEAN PATENT APPLICATION

(11) **EP 4 799 630 A1**
(43) Date of publication of application: **02.09.2026**
(21) Application number: 25161174.5
(22) Date of filing: 01.03.2025
(51) Int. Cl.: A61K 31/7088, A61P 27/02, A61K 31/713

(54) **MEANS AND METHODS FOR THE TREATMENT OF A SUBJECT SUFFERING FROM OR BEING AT RISK OF SUFFERING FROM BACK OF THE EYE DISEASES**

(71) Applicant: TME Pharma AG, 10589 Berlin (DE); Singapore Health Services Pte Ltd, Singapore 168582 (SG); National University of Singapore, Singapore 119077 (SG)
(72) Inventor: WONG, Tina Tzee Ling, 168582 Singapore (SG); WANG, Xiaomeng, 169857 Singapore (SG); LIM, Seok Ting, 169857 Singapore (SG); EULBERG, Dirk, 10247 Berlin (DE); FRÖMMING, Anna, 14621 Schönwalde-Glien (DE)
(74) Representative: Bohmann, Armin K.

(57) **Abstract**

The present invention is related to an inhibitor of Monocyte Chemoattractant Protein-1 (CCL2) activity, for use in a method for treating a subject suffering from or being at risk of suffering from an eye disease, wherein the eye disease is a back of the eye disease.

## Description

The present invention is related to an inhibitor of Monocyte Chemoattractant Protein-1 (CCL2, also referred to as MCP-1) activity for use in a method for treating a subject suffering from or being at risk of suffering from an eye disease, and a method for the treatment of back of the eye disease.

The human eye is a complex organ with a structure designed to capture light and convert it into neural signals that the brain interprets as vision. The general structure of the eye can be broken down into several key components, each of which can be involved in various disorders or pathologies (Kaplan 2007, Levin 2024). These elements are (i) the cornea, a transparent, dome-shaped surface that covers the front of the eye, functioning as a barrier to dirt, germs, and other particles and also helps focus incoming light; (ii) the aqueous humor, the clear fluid found between the cornea and the iris which provides nutrients to the avascular cornea and lens and maintains intraocular pressure; (iii) the iris, which controls the size of the pupil to regulate the amount of light entering the eye; (iv) the lens, a transparent, flexible structure that focuses light onto the retina and that, together with the cornea, refracts the light and ensure it is properly focused on the retina; (v) the vitreous humor, a clear gel-like substance that fills the space between the lens and retina which helps maintain the eye's shape and allows light to pass through to the retina; (vi) the retina, a thin layer of light-sensitive cells (photoreceptors) at the back of the eye which converts light into electrical signals that are sent to the brain via the optic nerve; (vii) the optic nerve which transmits visual information from the retina to the brain; (viii) the macula, a small central area of the retina responsible for focused, central vision, which also contains the fovea, which is the point of clearest vision; (ix) the sclera, the white, outer protective layer of the eye which maintains the eye's shape and provides protection; and (x) the choroid, a layer between the retina and sclera which is rich in blood vessels that provide oxygen and nutrients to the retina.

Disorders and pathologies associated with the eye (Salmon 2024) include refractive errors which can be subclassified into (i) myopia (nearsightedness), i.e. difficulty seeing distant objects clearly due to an elongated eyeball or overly curved cornea; (ii) hyperopia (farsightedness), i.e. difficulty seeing close objects clearly, typically due to a shorter eyeball or flatter cornea; (iii) astigmatism, i.e. irregular curvature of the cornea or lens causing blurred vision; (iv) presbyopia, i.e. age-related loss of near vision due to hardening of the lens; (v) cataract, clouding of the lens that can cause blurred or dimmed vision, a condition which is commonly age-related but can also be caused by trauma or other factors; (vi) glaucoma, a group of eye conditions characterized by increased intraocular pressure that can damage the optic nerve, leading to vision loss, where the two most frequent types are open-angle glaucoma (POAG) and angle-closure glaucoma (PACG); (vii) macular degeneration, which can be subclassified into (a) dry age-related macular degeneration (AMD) - the most common form of macular degeneration, accounting for about 85-90% of cases which occurs when the macula gradually thins and breaks down due to the accumulation of waste products called drusen (yellow deposits beneath the retina), a process that leads to a gradual loss of central vision; and (b) neovascular or wet or exudative age-related macular degeneration (nAMD) which occurs when abnormal blood vessels grow beneath the retina, leaking fluid and blood into the macula, causing rapid damage to the central vision due to the resulting inflammation which finally leads to scarring (fibrosis) and distortion of the macula and which can result in more rapid vision loss compared to dry AMD; (viii) diabetic retinopathy, a damage to retinal blood vessels caused by prolonged high blood sugar levels in diabetics which can lead to vision impairment or blindness if untreated; (ix) retinal detachment which occurs when the retina separates from the underlying layer of support tissue and which can lead to vision loss; (x) conjunctivitis, an inflammation of the conjunctiva, the thin layer covering the front of the eye which is caused by infections or allergic reactions; (xi) keratitis, an inflammation of the cornea, often caused by infection or trauma; (xii) strabismus, a misalignment of the eyes (crossed or wandering eyes), leading to double vision or poor depth perception, caused by neurological, muscular, or refractive issues; (xiii) blepharitis, an inflammation of the eyelids, often near the eyelashes, typically caused by bacterial infection or seborrheic dermatitis; (xiv) retinopathy of prematurity, abnormal growth of retinal blood vessels in premature infants, potentially leading to retinal detachment and blindness; (xv) dry eye syndrome, insufficient tear production or poor-quality tears, leading to discomfort, redness, and potential damage to the cornea; (xvi) optic neuritis, inflammation of the optic nerve, often linked with multiple sclerosis, causing vision loss or pain with eye movement; and (xvii) uveitis, an inflammation of the uvea which can lead to pain, light sensitivity, and vision loss.

The problem underlying the present invention is the provision of means and methods for treating diseases of the eye.

A further problem underlying the present invention is the provision of means and methods for treating a subject suffering from age-related macular degeneration (AMD).

A still further problem underlying the present invention is the provision of means and methods for treating a subject suffering from or being at risk of suffering from diabetic retinopathy (DR).

Another problem underlying the present invention is the provision of means and methods for treating a subject suffering from or being at risk of suffering from diabetic retinopathy (PVR).

A still further problem underlying the present invention is the provision of means and method suitable as adjunct therapy for PVR.

These and other problems underlying the present invention are solved by the subject matter of the attached independent claims. Preferred embodiments may be taken from the attached dependent claims.

Similarly, these and other problems underlying the present invention are solved by the subject matter of the Embodiments disclosed in the following. Additionally, these and other problems underlying the present invention are solved by the subject matter of the various aspect of the present invention, including any embodiment thereof, as disclosed herein.

These and other problems underlying the present invention are solved by the subject matter of the attached independent claim. Preferred embodiments may be taken from the attached dependent claims.

Embodiment 1. An inhibitor of Monocyte Chemoattractant Protein-1 (CCL2) activity, for use in a method for treating a subject suffering from or being at risk of suffering from an eye disease, wherein the eye disease is a back of the eye disease selected from the group consisting of age-related macular degeneration (AMD), diabetic retinopathy (DR) and proliferative vitreoretinopathy (PVR).

Embodiment 2. The inhibitor of Monocyte Chemoattractant Protein-1 (CCL2) activity for use of claim 1, wherein the inhibitor of CCL2 activity is locally administered to the subject.

Embodiment 3. The inhibitor of Monocyte Chemoattractant Protein-1 (CCL2) activity for use of any one of Embodiments 1 and 2, wherein the inhibitor of CCL2 activity is systemically administered to the subject.

Embodiment 4. The inhibitor of Monocyte Chemoattractant Protein-1 (CCL2) activity for use of any one of Embodiments 1 to 3, wherein the inhibitor of CCL2 activity is locally and systemically administered to the subject.

Embodiment 5. The inhibitor of Monocyte Chemoattractant Protein-1 (CCL2) activity for use of any one of Embodiments 2 to 4, wherein local administration is intravitreal administration, suprachoroidal administration, subretinal administration or a combination thereof.

Embodiment 6. The inhibitor of Monocyte Chemoattractant Protein-1 (CCL2) activity for use of Embodiment 5, wherein local administration is intravitreal administration.

Embodiment 7. The inhibitor of Monocyte Chemoattractant Protein-1 (CCL2) activity for use of any one of Embodiments 3 to 6, wherein systemic administration is selected from the group comprising subcutaneous administration and intravenous administration.

Embodiment 8. The inhibitor of Monocyte Chemoattractant Protein-1 (CCL2) activity for use of any one of Embodiments 1 to 7, wherein the subject is suffering from or at risk of suffering from AMD.

Embodiment 9. The inhibitor of Monocyte Chemoattractant Protein-1 (CCL2) activity for use of Embodiment 8, wherein AMD is early AMD, intermediate AMD or advanced AMD.

Embodiment 10. The inhibitor of Monocyte Chemoattractant Protein-1 (CCL2) activity for use of any one of Embodiments 8 to 9, wherein AMD, preferably advanced AMD, is selected from the group consisting of neovascular or wet AMD and atrophic dry AMD.

Embodiment 11. The inhibitor of Monocyte Chemoattractant Protein-1 (CCL2) activity for use of any one of Embodiments 8 to 10, wherein AMD is polypoidal choroidal vasculopathy or retinal angiomatous proliferation.

Embodiment 12. The inhibitor of Monocyte Chemoattractant Protein-1 (CCL2) activity for use of any one of Embodiments 8 to 11, wherein the subject has drusen underneath the retinal pigment epithelium, preferably soft and/or large drusen.

Embodiment 13. The inhibitor of Monocyte Chemoattractant Protein-1 (CCL2) activity for use of any one of Embodiment 8 to 12, wherein the subject is at risk of suffering from AMD or is suffering from AMD because of a risk factor selected from the group comprising increased age, smoking, uncontrolled hypertension and a body mass index > 25 kg/m².

Embodiment 14. The inhibitor of Monocyte Chemoattractant Protein-1 (CCL2) activity for use of any one of Embodiments 8 to 13, wherein the subject is of Caucasian ancestry.

Embodiment 15. The inhibitor of Monocyte Chemoattractant Protein-1 (CCL2) activity for use of any one of Embodiments 8 to 14, wherein an agent is administered to the subject prior to, concomitantly with or subsequent to the administration of the inhibitor of CCL2 activity, wherein the agent is selected from the group comprising a VEGF inhibitor, an angiopoietin-2 inhibitor, a C3 complement inhibitor, a C5 complement inhibitor and a steroid.

Embodiment 16. The inhibitor of Monocyte Chemoattractant Protein-1 (CCL2) activity for use of any one of Embodiments 1 to 7, wherein the subject is suffering from or at risk of suffering from DR.

Embodiment 17. The inhibitor of Monocyte Chemoattractant Protein-1 (CCL2) activity for use of Embodiment 16, wherein DR is nonproliferative diabetic retinopathy (NPDR) or proliferative diabetic retinopathy (PDR).

Embodiment 18. The inhibitor of Monocyte Chemoattractant Protein-1 (CCL2) activity for use of any one of Embodiments 16 and 17, wherein DR goes along with or is associated with diabetic macular edema (DME).

Embodiment 19. The inhibitor of Monocyte Chemoattractant Protein-1 (CCL2) activity for use of Embodiment 18, wherein the subject is suffering from or at risk of suffering from both nonproliferative diabetic retinopathy (NPDR) and diabetic macular edema (DME).

Embodiment 20. The inhibitor of Monocyte Chemoattractant Protein-1 (CCL2) activity for use of Embodiment 19, wherein the subject is suffering from or at risk of suffering from both proliferative diabetic retinopathy (PDR) and diabetic macular edema (DME).

Embodiment 21. The inhibitor of Monocyte Chemoattractant Protein-1 (CCL2) activity for use of any one of Embodiments 16 to 20, wherein prior to the administration of the inhibitor of CCL2 activity the subject underwent a therapy selected from the group comprising anti-VEGF therapy, laser photoagglutination, vitreoretinal surgery or a combination thereof.

Embodiment 22. The inhibitor of Monocyte Chemoattractant Protein-1 (CCL2) activity for use of any one of Embodiments 16 to 21, wherein an agent is administered to the subject prior to, concomitantly with or subsequent to the administration of the inhibitor of CCL2 activity, wherein the agent is selected from the group comprising an angiotensin converting enzyme inhibitor, a VEGF inhibitor, a CTGF inhibitor, a statin and a steroid.

Embodiment 23. The inhibitor of Monocyte Chemoattractant Protein-1 (CCL2) activity for use of Embodiment 22, wherein the agent is concomitantly administered with the inhibitor of CCL2 activity.

Embodiment 24. The inhibitor of Monocyte Chemoattractant Protein-1 (CCL2) activity for use of any one of Embodiments 1 to 7, wherein the subject is suffering from or at risk of suffering from PVR.

Embodiment 25. The inhibitor of Monocyte Chemoattractant Protein-1 (CCL2) activity for use of Embodiment 24, wherein the subject is at risk of suffering from PVR.

Embodiment 26. The inhibitor of Monocyte Chemoattractant Protein-1 (CCL2) activity for use of any one of Embodiments 24 to 25, wherein macrophages are present in a vitreous of the subject.

Embodiment 27. The inhibitor of Monocyte Chemoattractant Protein-1 (CCL2) activity for use of any one of Embodiments 24 to 26, wherein the subject is at risk of suffering from PVR if macrophages are present in a vitreous of the subject.

Embodiment 28. The inhibitor of Monocyte Chemoattractant Protein-1 (CCL2) activity for use of any one of Embodiments 24 to 27, wherein the subject is suffering from an ocular trauma.

Embodiment 29. The inhibitor of Monocyte Chemoattractant Protein-1 (CCL2) activity for use of Embodiment 28, wherein the ocular trauma is retinal detachment repair surgery, wherein the subject undergoes the retinal detachment repair surgery prior to, concomitant with or subsequent to administration of the inhibitor of CCL2 activity.

Embodiment 30. The inhibitor of Monocyte Chemoattractant Protein-1 (CCL2) activity for use of any one of Embodiments 24 to 29, wherein administering an inhibitor of CCL2 is an adjunct therapy for the treatment of PVR, preferably as a consequence of or resulting from ocular trauma.

Embodiment 31. The inhibitor of Monocyte Chemoattractant Protein-1 (CCL2) activity for use of Embodiment 30, wherein ocular trauma is as surgery for treating RD.

Embodiment 32. The inhibitor of Monocyte Chemoattractant Protein-1 (CCL2) activity for use of any one of Embodiment 24 to 31, wherein an agent is administered to the subject prior to, concomitant with or subsequent to the administration of the inhibitor of CCL2 activity, wherein the agent is selected from the group comprising an anti-inflammatory agent, an agent inhibiting cell proliferation and an anti-fibrotic agent.

Embodiment 33. The inhibitor of Monocyte Chemoattractant Protein-1 (CCL2) activity for use of Embodiment 32, wherein the agent is selected from the group comprising a corticosteroid and methotrexate.

Embodiment 34. The inhibitor of Monocyte Chemoattractant Protein-1 (CCL2) activity for use of any one of Embodiments 1 to 33, wherein the inhibitor of CCL2 activity is anti-fibrotic.

Embodiment 35. The inhibitor of Monocyte Chemoattractant Protein-1 (CCL2) activity for use of any one of Embodiments 1 to 34, wherein the inhibitor of CCL2 activity is a compound selected from the group comprising a compound binding to CCL2, a compound binding to CCR2, a compound inhibiting activity of CCR2 and a compound inhibiting signaling of CCR2.

Embodiment 36. The inhibitor of Monocyte Chemoattractant Protein-1 (CCL2) activity for use of Embodiment 35, wherein the compound inhibiting activity of CCR2 is a compound binding to CCR2 or the compound inhibiting activity of CCR2 is a compound binding to CCL2.

Embodiment 37. The inhibitor of Monocyte Chemoattractant Protein-1 (CCL2) activity for use of and one of Embodiments 1 to 36, wherein the inhibitor of CCL2 is a compound inhibiting signaling of CCR2, preferably the compound is binding to CCL2 or is binding to CCR2.

Embodiment 38. The inhibitor of Monocyte Chemoattractant Protein-1 (CCL2) activity for use of any one of Embodiments 1 to 37, wherein the inhibitor of CCL2 activity is a compound selected from the group comprising an aptamer binding to CCL2, an aptamer binding to CCR2, an aptamer inhibiting signaling of CCR2, preferably by binding to CCL2 or to CCR2, a Spiegelmer binding to CCL2, a Spiegelmer binding to CCR2, a Spiegelmer inhibiting signaling of CCR2, preferably by binding to CCL2 or to CCR2, an antibody binding to CCL2, an antibody binding to CCR2, an antibody inhibiting signaling of CCR2, preferably by binding to CCL2 or to CCR2, an antibody fragment binding to CCL2, an antibody fragment binding to CCR2, an antibody fragment inhibiting signaling of CCR2, preferably by binding to CCL2 or to CCR2, a protein binding to CCL2, a protein binding to CCR2, a protein inhibiting signaling of CCR2, preferably by binding to CCL2 or to CCR2, an anticalin binding to CCL2, an anticalin binding to CCR2, an anticalin inhibiting signaling of CCR2, preferably by binding to CCL2 or to CCR2, a small molecule binding to CCL2, a small molecule binding to CCR2 and a small molecule inhibiting signaling of CCR2, preferably by binding to CCL2 or to CCR2.

Embodiment 39. An inhibitor of Monocyte Chemoattractant Protein-1 (CCL2) activity for use of any one of Embodiments 1 to 38, wherein the inhibitor of CCL2 activity is an L-nucleic acid, preferably binding to MCP-1, selected from the group comprising type 1A nucleic acids, type 1B nucleic acids, type 2 nucleic acids, type 3 nucleic acids, type 4 nucleic acids and nucleic acids having a nucleic acid sequence according to any of SEQ.ID.No. 87 to 115.

Embodiment 40. The inhibitor of Monocyte Chemoattractant Protein-1 (CCL2) activity for use of Embodiment 39, wherein the type 1A nucleic acid comprises in 5'->3' direction a first stretch Box B1A, a second stretch Box B2, a third stretch Box B3, a fourth stretch Box B4, a fifth stretch Box B5, a sixth stretch Box B6 and a seventh stretch Box B1B, wherein
the first stretch Box B1A and the seventh stretch Box B1B optionally hybridize with each other, wherein upon hybridization a double-stranded structure is formed,
the first stretch Box B1A comprises a nucleotide sequence of AGCRUG,
the second stretch Box B2 comprises a nucleotide sequence of CCCGGW,
the third stretch Box B3 comprises a nucleotide sequence of GUR,
the fourth stretch Box B4 comprises a nucleotide sequence of RYA,
the fifth stretch Box B5 comprises a nucleotide sequence of GGGGGRCGCGAYC
the sixth stretch Box B6 comprises a nucleotide sequence of UGCAAUAAUG or URYAWUUG, and
the seventh stretch Box B1B comprises a nucleotide sequence of CRYGCU.

Embodiment 41. The inhibitor of Monocyte Chemoattractant Protein-1 (CCL2) activity for use of Embodiment 40, wherein the first stretch Box B1A comprises a nucleotide sequence of AGCGUG.

Embodiment 42. The inhibitor of Monocyte Chemoattractant Protein-1 (CCL2) activity for use of Embodiments 40 or 41, wherein the second stretch Box B2 comprises a nucleotide sequence of CCCGGU.

Embodiment 43. The inhibitor of Monocyte Chemoattractant Protein-1 (CCL2) activity for use of any of Embodiments 40 to 42, wherein the third stretch Box B3 comprises a nucleotide sequence of GUG.

Embodiment 44. The inhibitor of Monocyte Chemoattractant Protein-1 (CCL2) activity for use of any of Embodiments 40 to 43, wherein the fourth stretch Box B4 comprises a nucleotide sequence of GUA.

Embodiment 45. The inhibitor of Monocyte Chemoattractant Protein-1 (CCL2) activity for use of any of Embodiments 40 to 44, wherein the fifth stretch Box B5 comprises a nucleotide sequence of GGGGGGCGCGACC.

Embodiment 46. The inhibitor of Monocyte Chemoattractant Protein-1 (CCL2) activity for use of any of Embodiments 40 to 45, wherein the sixth stretch Box B6 comprises a nucleotide sequence of UACAUUUG.

Embodiment 47. The inhibitor of Monocyte Chemoattractant Protein-1 (CCL2) activity for use of any of Embodiments 40 to 46, wherein the seventh stretch Box B1B comprises a nucleotide sequence of CACGCU.

Embodiment 48. The inhibitor of Monocyte Chemoattractant Protein-1 (CCL2) activity for use of any of Embodiments 40 to 47, wherein the nucleic acid comprises a nucleic acid sequence according to SEQ.ID. No 21.

Embodiment 49. The inhibitor of Monocyte Chemoattractant Protein-1 (CCL2) activity for use of Embodiment 39, wherein the type 1B nucleic acid comprises in 5'->3' direction a first stretch Box B1A, a second stretch Box B2, a third stretch Box B3, a fourth stretch Box B4, a fifth stretch Box B5, a sixth stretch Box B6 and a seventh stretch Box B1B, wherein
the first stretch Box B1A and the seventh stretch Box B1B optionally hybridize with each other, wherein upon hybridization a double-stranded structure is formed,
the first stretch Box B1A comprises a nucleotide sequence of AGYRUG,
the second stretch Box B2 comprises a nucleotide sequence of CCAGCU or CCAGY,
the third stretch Box B3 comprises a nucleotide sequence of GUG,
the fourth stretch Box B4 comprises a nucleotide sequence of AUG,
the fifth stretch Box B5 comprises a nucleotide sequence of GGGGGGCGCGACC
the sixth stretch Box B6 comprises a nucleotide sequence of CAUUUUA or CAUUUA, and
the seventh stretch Box B1B comprises a nucleotide sequence of CAYRCU.

Embodiment 50. The inhibitor of Monocyte Chemoattractant Protein-1 (CCL2) activity for use of Embodiment 49, wherein the first stretch Box B1A comprises a nucleotide sequence of AGCGUG.

Embodiment 51. The inhibitor of Monocyte Chemoattractant Protein-1 (CCL2) activity for use of Embodiments 49 or 50, wherein the second stretch Box B2 comprises a nucleotide sequence of CCAGU.

Embodiment 52. The inhibitor of Monocyte Chemoattractant Protein-1 (CCL2) activity for use of any of Embodiments 49 to 51, wherein the sixth stretch Box B6 comprises a nucleotide sequence of CAUUUUA.

Embodiment 53. The inhibitor of Monocyte Chemoattractant Protein-1 (CCL2) activity for use of any of Embodiments 49 to 53, wherein the seventh stretch Box B1B comprises a nucleotide sequence of CACGCU.

Embodiment 54. The inhibitor of Monocyte Chemoattractant Protein-1 (CCL2) activity for use of any of Embodiments 49 to 53, wherein the nucleic acid comprises a nucleic acid sequence according to SEQ.ID.No 28 and SEQ.ID.No 27.

Embodiment 55. The inhibitor of Monocyte Chemoattractant Protein-1 (CCL2) activity for use of Embodiment 39, wherein the type 2 nucleic acid comprises in 5'->3' direction a first stretch Box B1A, a second stretch Box B2, and a third stretch Box B1B, wherein
the first stretch Box B1A and the third stretch Box B1B optionally hybridize with each other, wherein upon hybridization a double-stranded structure is formed,
the first stretch Box B1A comprises a nucleotide sequence selected from the group comprising ACGCA, CGCA and GCA,
the second stretch Box B2 comprises a nucleotide sequence of CSUCCCUCACCGGUGCAAGUGAAGCCGYGGCUC, and
the third stretch Box B1B comprises a nucleotide sequence selected from the group comprising UGCGU, UGCG and UGC.

Embodiment 56. The inhibitor of Monocyte Chemoattractant Protein-1 (CCL2) activity for use of Embodiment 55, wherein the second stretch Box B2 comprises a nucleotide sequence of CGUCCCUCACCGGUGCAAGUGAAGCCGUGGCUC.

Embodiment 57. The inhibitor of Monocyte Chemoattractant Protein-1 (CCL2) activity for use of any of Embodiments 55 to 56, wherein
a) the first stretch Box B1A comprises a nucleotide sequence of ACGCA,
   and
   the third stretch Box B1B comprises a nucleotide sequence of UGCGU; or
b) the first stretch Box B1A comprises a nucleotide sequence of CGCA,
   and
   the third stretch Box B1B comprises a nucleotide sequence of UGCG; or
c) the first stretch Box B 1A comprises a nucleotide sequence of GCA,
   and
   the third stretch Box B1B comprises a nucleotide sequence of UGC or UGCG.

Embodiment 58. The inhibitor of Monocyte Chemoattractant Protein-1 (CCL2) activity for use of any of Embodiments 55 to 57, wherein the first stretch Box B1A comprises a nucleotide sequence of GCA.

Embodiment 59. The inhibitor of Monocyte Chemoattractant Protein-1 (CCL2) activity for use of any of Embodiments 55 to 58 and preferably Embodiment 58, wherein the third stretch Box B1B comprises a nucleotide sequence of UGCG.

Embodiment 60. The inhibitor of Monocyte Chemoattractant Protein-1 (CCL2) activity for use of any of Embodiments 55 to 59, wherein the nucleic acid comprises a nucleic acid sequence according to SEQ.ID.No 37, SEQ.ID.No 116, SEQ.ID.No 117 and SEQ.ID.No 278.

Embodiment 61. The inhibitor of Monocyte Chemoattractant Protein-1 (CCL2) activity for use of Embodiment 39 wherein the type 3 nucleic acid comprises in 5'->3' direction a first stretch Box B1A, a second stretch Box B2A, a third stretch Box B3, a fourth stretch Box B2B, a fifth stretch Box B4, a sixth stretch Box B5A, a seventh stretch Box B6, an eighth stretch Box B5B and a ninth stretch Box B1B, wherein
the first stretch Box B1A and the ninth stretch Box B1B optionally hybridize with each other, wherein upon hybridization a double-stranded structure is formed,
the second stretch Box B2A and the fourth Box B2B optionally hybridize with each other, wherein upon hybridization a double-stranded structure is formed,
the sixth stretch Box B5A and the eighth Box B5B optionally hybridize with each other, wherein upon hybridization a double-stranded structure is formed,
the first stretch Box B1A comprises a nucleotide sequence which is selected from the group comprising GURCUGC, GKSYGC, KBBSC and BNGC,
the second stretch Box B2A comprises a nucleotide sequence of GKMGU,
the third stretch Box B3 comprises a nucleotide sequence of KRRAR,
the fourth stretch Box B2B comprises a nucleotide sequence of ACKMC,
the fifth stretch Box B4 comprises a nucleotide sequence selected from the group comprising CURYGA, CUWAUGA, CWRMGACW and UGCCAGUG,
the sixth stretch Box B5A comprises a nucleotide sequence selected from the group comprising GGY and CWGC,
the seventh stretch Box B6 comprises a nucleotide sequence selected from the group comprising YAGA, CKAAU and CCUUUAU,
the eighth stretch Box B5B comprises a nucleotide sequence selected from the group comprising GCYR and GCWG, and
the ninth stretch Box B1B comprises a nucleotide sequence selected from the group comprising GCAGCAC, GCRSMC, GSVVM and GCNV.

Embodiment 62. The inhibitor of Monocyte Chemoattractant Protein-1 (CCL2) activity for use of Embodiment 61, wherein the third stretch Box B3 comprises a nucleotide sequence of GAGAA or UAAAA

Embodiment 63. The inhibitor of Monocyte Chemoattractant Protein-1 (CCL2) activity for use of Embodiments 61 or 62, wherein the fifth stretch Box B4 comprises a nucleotide sequence of CAGCGACU or CAACGACU.

Embodiment 64. The inhibitor of Monocyte Chemoattractant Protein-1 (CCL2) activity for use of any of Embodiments 61 to 63, wherein the fifth stretch Box B4 comprises a nucleotide sequence of CAGCGACU and Box B3 comprises a nucleotide sequence of UAAAA.

Embodiment 65. The inhibitor of Monocyte Chemoattractant Protein-1 (CCL2) activity for use of any of Embodiments 61 to 64, wherein the fifth stretch Box B4 comprises a nucleotide sequence of CAACGACU and the third stretch Box B3 comprises a nucleotide sequence of GAGAA.

Embodiment 66. The inhibitor of Monocyte Chemoattractant Protein-1 (CCL2) activity for use of any of Embodiments 61 to 65, wherein the seventh stretch Box B6 comprises a nucleotide sequence of UAGA.

Embodiment 67. The inhibitor of Monocyte Chemoattractant Protein-1 (CCL2) activity for use of any of Embodiments 61 to 66, wherein
a) the first stretch Box B1A comprises a nucleotide sequence of GURCUGC,
   and
   the ninth stretch Box B1B comprises a nucleotide sequence of GCAGCAC; or
b) the first stretch Box B1A comprises a nucleotide sequence of GKSYGC,
   and
   the ninth stretch Box B1B comprises a nucleotide sequence of GCRSMC; or
c) the first stretch Box B1A comprises a nucleotide sequence of KBBSC,
   and
   the ninth stretch Box B1B comprises a nucleotide sequence of GSVVM; or
d) the first stretch Box B1A comprises a nucleotide sequence of BNGC,
   and
   the ninth stretch Box B1B comprises a nucleotide sequence of GCNV.

Embodiment 68. The inhibitor of Monocyte Chemoattractant Protein-1 (CCL2) activity for use of Embodiment 63, wherein
a) the first stretch Box B1A comprises a nucleotide sequence of GUGCUGC,
   and
   the ninth stretch Box B1B comprises a nucleotide sequence of GCAGCAC; or
b) the first stretch Box B1A comprises a nucleotide sequence of GUGCGC,
   and
   the ninth stretch Box B1B comprises a nucleotide sequence of GCGCAC; or
c) the first stretch Box B1A comprises a nucleotide sequence of KKSSC,
   and
   the ninth stretch Box B1B comprises a nucleotide sequence of GSSMM; or
d) the first stretch Box B1A comprises a nucleotide sequence of SNGC,
   and
   the ninth stretch Box B1B comprises a nucleotide sequence of GCNS.

Embodiment 69. The inhibitor of Monocyte Chemoattractant Protein-1 (CCL2) activity for use of Embodiment 68, wherein
the first stretch Box B 1A comprises a nucleotide sequence of GGGC,
   and
the ninth stretch Box B1B comprises a nucleotide sequence of GCCC.

Embodiment 70. The inhibitor of Monocyte Chemoattractant Protein-1 (CCL2) activity for use of any of Embodiments 61 to 69, wherein the second stretch Box B2A comprises a nucleotide sequence of GKMGU and the fourth stretch Box B2B comprises a nucleotide sequence of ACKMC.

Embodiment 71. The inhibitor of Monocyte Chemoattractant Protein-1 (CCL2) activity for use of Embodiment 70, wherein the second stretch Box B2A comprises a nucleotide sequence of GUAGU and the fourth stretch Box B2B comprises a nucleotide sequence of ACUAC.

Embodiment 72. The inhibitor of Monocyte Chemoattractant Protein-1 (CCL2) activity for use of any of Embodiments 61 to 61, wherein
a) the sixth stretch Box B5A comprises a nucleotide sequence of GGY,
   and
   the eighth stretch Box B5B comprises a nucleotide sequence of GCYR; or
b) the sixth stretch Box B5A comprises a nucleotide sequence of CWGC,
   and
   the eighth stretch Box B5B comprises a nucleotide sequence of GCWG.

Embodiment 73. The inhibitor of Monocyte Chemoattractant Protein-1 (CCL2) activity for use of Embodiment 72, wherein
the sixth stretch Box B5A comprises a nucleotide sequence of GGC,
   and
the eighth stretch Box B5B comprises a nucleotide sequence of GCCG.

Embodiment 74. The inhibitor of Monocyte Chemoattractant Protein-1 (CCL2) activity for use of any of Embodiments 61 to 73, wherein the sixth stretch Box B5A hybridizes with the nucleotides GCY of the eighth stretch Box B5B.

Embodiment 75. The inhibitor of Monocyte Chemoattractant Protein-1 (CCL2) activity for use of any of Embodiments 61 to 64 and 66 to 74, wherein the nucleic acid comprises a nucleic acid sequence according to SEQ.ID.No 56.

Embodiment 76. The inhibitor of Monocyte Chemoattractant Protein-1 (CCL2) activity for use of any of Embodiments 61 to 64 and 166 to 74, wherein the nucleic acid comprises a nucleic acid sequence selected from the group comprising the nucleic acid sequences according to SEQ.ID.No 57 to 61, SEQ.ID.No 67 to 71 and SEQ.ID.No 73.

Embodiment 77. The inhibitor of Monocyte Chemoattractant Protein-1 (CCL2) activity for use of Embodiment 39, wherein the type 4 nucleic acid comprises in 5'->3' direction a first stretch Box B1A, a second stretch Box B2, a third stretch Box B1B wherein
the first stretch Box B1A and the third stretch Box B1B optionally hybridize with each other, wherein upon hybridization a double-stranded structure is formed,
the first stretch Box B1A comprises a nucleotide sequence selected from the group comprising AGCGUGDU, GCGCGAG, CSKSUU, GUGUU, and UGUU;
the second stretch Box B2 comprises a nucleotide sequence selected from the group comprising AGNDRDGBKGGURGYARGUAAAG, AGGUGGGUGGUAGUAAGUAAAG and CAGGUGGGUGGUAGAAUGUAAAGA, and
the third stretch Box B1B comprises a nucleotide sequence selected from the group comprising GNCASGCU, CUCGCGUC, GRSMSG, GRCAC, and GGCA.

Embodiment 78. The inhibitor of Monocyte Chemoattractant Protein-1 (CCL2) activity for use of Embodiment 77, wherein
a) the first stretch Box B 1A comprises a nucleotide sequence of GUGUU,
   and
   the third stretch Box B1B comprises a nucleotide sequence of GRCAC;
b) the first stretch Box B1A comprises a nucleotide sequence of GCGCGAG,
   and
   the third stretch Box B1B comprises a nucleotide sequence of CUCGCGUC; or
c) the first stretch Box B1A comprises a nucleotide sequence of CSKSUU,
   and
   the third stretch Box B1B comprises a nucleotide sequence of GRSMSG, or
d) the first stretch Box B1A comprises a nucleotide sequence of UGUU,
   and
   the third stretch Box B1B comprises a nucleotide sequence of GGCA, or
e) the first stretch Box B1A comprises a nucleotide sequence of AGCGUGDU,
   and
   the third stretch Box B1B comprises a nucleotide sequence of GNCASGCU.

Embodiment 79. The inhibitor of Monocyte Chemoattractant Protein-1 (CCL2) activity for use of Embodiment 78, wherein the first stretch Box B1A comprises a nucleotide sequence of CSKSUU and the third stretch Box B1B comprises a nucleotide sequence of GRSMSG.

Embodiment 80. The inhibitor of Monocyte Chemoattractant Protein-1 (CCL2) activity for use of Embodiments 79, wherein the first stretch Box B1A comprises a nucleotide sequence of CCGCUU and the third stretch Box B1B comprises a nucleotide sequence of GGGCGG.

Embodiment 81. The inhibitor of Monocyte Chemoattractant Protein-1 (CCL2) activity for use of any of Embodiments 77 to 80, wherein
the second stretch Box B2 comprises a nucleotide sequence of AGGUGGGUGGUAGUAAGUAAAG.

Embodiment 82. The inhibitor of Monocyte Chemoattractant Protein-1 (CCL2) activity for use of any of Embodiments 77 to 81, wherein the nucleic acid comprises a nucleic acid sequence according to SEQ.ID.No 80.

Embodiment 83. The inhibitor of Monocyte Chemoattractant Protein-1 (CCL2) activity for use of any of Embodiments 39 to 82, wherein the nucleic acid is capable of binding human MCP-1.

Embodiment 84. The inhibitor of Monocyte Chemoattractant Protein-1 (CCL2) activity for use of any of Embodiments 39 to 83, preferably Embodiment 176, wherein the MCP-1 has an amino acid sequence according to SEQ ID No. 1.

Embodiment 85. The inhibitor of Monocyte Chemoattractant Protein-1 (CCL2) activity for use of any of Embodiments 39 to 83, wherein the nucleic acid comprises a modification, wherein the modification is preferably a high molecular weight moiety and/or wherein the modification preferably allows to modify the characteristics of the nucleic acid according to any of Embodiments 1 to 46 in terms of residence time in the animal or human body, preferably the human body.

Embodiment 86. The inhibitor of Monocyte Chemoattractant Protein-1 (CCL2) activity for use of Embodiment 85, wherein the modification is selected from the group comprising a HES moiety and a PEG moiety.

Embodiment 87. The inhibitor of Monocyte Chemoattractant Protein-1 (CCL2) activity for use of Embodiment 86, wherein the modification is a PEG moiety consisting of a straight or branched PEG, wherein the molecular weight of the PEG moiety is preferably from about 20 to 120 kD, more preferably from about 30 to 80 kD and most preferably about 40 kD.

Embodiment 88. The inhibitor of Monocyte Chemoattractant Protein-1 (CCL2) activity for use of Embodiment 86, wherein the modification is a HES moiety, wherein preferably the molecular weight of the HES moiety is from about 10 to 130 kD, more preferably from about 30 to 130 kD and most preferably about 100 kD.

Embodiment 89. The inhibitor of Monocyte Chemoattractant Protein-1 (CCL2) activity for use of any of Embodiments of 85 to 88, wherein the modification is coupled to the nucleic acid via a linker.

Embodiment 90. The inhibitor of Monocyte Chemoattractant Protein-1 (CCL2) activity for use of any of Embodiments of 88 to 89, wherein the modification is coupled to the nucleic acid at its 5'-terminal nucleotide and/or its 3'-terminal nucleotide and/or to a nucleotide of the nucleic acid between the 5'-terminal nucleotide and the 3'-terminal nucleotide.

Embodiment 91. The inhibitor of Monocyte Chemoattractant Protein-1 (CCL2) activity for use of any of Embodiments 39 to 90, wherein the nucleotides of or the nucleotides forming the nucleic acid are L-nucleotides.

Embodiment 92. The inhibitor of Monocyte Chemoattractant Protein-1 (CCL2) activity for use of any of Embodiments 39 to 91, wherein the moiety of the nucleic acid capable of binding MCP-1 consists of L-nucleotides.

Embodiment 93. The inhibitor of Monocyte Chemoattractant Protein-1 (CCL2) activity for use of any one of Embodiments 39, 55 to 59 and 83 to 92, wherein the type 2 nucleic acid comprises in 5'->3' direction a first stretch Box B1A, a second stretch Box B2, and a third stretch Box B1B, wherein
the first stretch Box B1A comprises a nucleotide sequence selected from the group comprising ACGCA, CGCA and GCA,
the second stretch Box B2 comprises a nucleotide sequence of CSUCCCUCACCGGUGCAAGUGAAGCCGYGGCUC, and
the third stretch Box B1B comprises a nucleotide sequence selected from the group comprising UGCGU, UGCG and UGC.

Embodiment 94. The inhibitor of Monocyte Chemoattractant Protein-1 (CCL2) activity for use of any one of Embodiments 39, 55 to 60 and 83 to 94, wherein the nucleic acid comprises a nucleotide sequence of SEQ IQ NO: 37 or a nucleotide sequence having an identity of at least 85 % to the nucleotide sequence of SEQ ID NO: 37.

Embodiment 95. The inhibitor of Monocyte Chemoattractant Protein-1 (CCL2) activity for use of any one of Embodiments 39, 55 to 60 and 83 to 94, wherein the nucleic acid comprises a nucleotide sequence of SEQ ID NO: 37.

Embodiment 96. The inhibitor of Monocyte Chemoattractant Protein-1 (CCL2) activity for use any one of Embodiments 93 to 95, wherein the nucleic acid comprises a modification.

Embodiment 97. The inhibitor of Monocyte Chemoattractant Protein-1 (CCL2) activity for use of Embodiment 96, wherein the modification is coupled to the nucleic acid via a linker.

Embodiment 98. The inhibitor of Monocyte Chemoattractant Protein-1 (CCL2) activity for use of any one of Embodiments 96 to 97, wherein the modification comprises a polyethylene glycol (PEG) molecule.

Embodiment 99. The inhibitor of Monocyte Chemoattractant Protein-1 (CCL2) activity for use of Embodiments 98, wherein the PEG molecule is at the 5' terminus of the nucleic acid.

Embodiment 100. The inhibitor of Monocyte Chemoattractant Protein-1 (CCL2) activity for use of Embodiment 98, wherein the PEG molecule is at the 3' terminus of the nucleic acid.

Embodiment 101. The inhibitor of Monocyte Chemoattractant Protein-1 (CCL2) activity for use of any one of Embodiments 98 to 100, wherein the PEG molecule is straight chain or branched.

Embodiment 102. The inhibitor of Monocyte Chemoattractant Protein-1 (CCL2) activity for use of any one of Embodiments 98 to 101, wherein the PEG molecule has a molecular weight from about 20 kDa to about 120 kDa, preferably from about 30 kDa to about 80 kDa.

Embodiment 103. The inhibitor of Monocyte Chemoattractant Protein-1 (CCL2) activity for use of any one of Embodiments 98 to 102, wherein the PEG molecule has a molecular weight of about 40 kDa.

Embodiment 104 The inhibitor of Monocyte Chemoattractant Protein-1 (CCL2) activity for use of any one of Embodiments 98 to 103, wherein the PEG is a polydisperse PEG or a monodisperse PEG.

Embodiment 105. The inhibitor of Monocyte Chemoattractant Protein-1 (CCL2) activity for use of any one of Embodiments 1 to 104, wherein the inhibitor of CCL2 activity is compound NOX-E36.

Embodiment 106. The inhibitor of Monocyte Chemoattractant Protein-1 (CCL2) activity for use of any one of Embodiments 1 to 104, wherein the inhibitor of CCL2 activity is selected from the group comprising a compound targeting CCL2, wherein the compound is selected from the group comprising Bindarit, a small molecule CCL2 inhibitor; Carlumab (CNTO888), a fully-human anti-CCL2 Mab; ABN-912, a fully-human anti-CCL2 Mab; CGEN-54 and a recombinant CCL2-inhibiting protein.

Embodiment 107. The inhibitor of Monocyte Chemoattractant Protein-1 (CCL2) activity for use of any one of Embodiments 1 to 105, wherein the inhibitor of CCL2 activity is selected from the group comprising a compound targeting CCR2, wherein the compound is selected from the group comprising AZ-889, a small molecule CCR2 antagonist; AZD-2423 a small molecule CCR2 antagonist, a small molecule CCR2 antagonist; BL-2030, a soluble CCR2 receptor fused to a human antibody Fc component; BMS-741672, a small molecule CCR2 antagonist; BMS-753426, a small molecule CCR2 antagonist; BMS-813160, a small molecule CCR2 antagonist; CCX-140, a small molecule CCR2 inhibitor; CCX-598, a small molecule CCR2 inhibitor; CCX-872, a small molecule CCR2 inhibitor; CCX-915, a small molecule CCR2 inhibitor; cenicriviroc, a small molecule CCR2/CCR5 antagonist; CNTX-6970, a small molecule CCR2 antagonist; CPD-B, a small molecule CCR2 antagonist; EPX-102216, a small molecule CCR2 antagonist; INCB-3344, a small molecule CCR2 inhibitor; INCB-3284, a small molecule CCR2 inhibitor; INCB-8696, a small molecule CCR2 inhibitor; LF-0376, a small molecule CCR2/5 antagonist; MK-812, a small molecule CCR2 inhibitor; NIBR-6465, a small molecule CCR2/5 antagonist; OB-004 a small molecule CCR2 antagonist; OPL-CCL2-LPM, a human CCL2 chemokine fusion protein with cytotoxic payload; PD-172084, a small molecule CCR2 antagonist; PF-04634817, a small molecule CCR2/5 antagonist; PF-4136309, a small molecule CCR2 antagonist; Plozalizumab (MLN-1202), a humanized anti-CCR2 mAb; R-103, an oral Dalal-peptide T-amide analogue targeting CCR2, 5 and 8; RAP-103; RAP-310, both small molecule CCR2/5 antagonists; SB-380732, a small molecule CCR2 antagonist; SPR-3, a small molecule CCR2 antagonist; STI-B0201, a fully human anti-CCR2 mAb; STI-B0211, a fully human anti-CCR2 mAb; STI-B0221, a fully human anti-CCR2 mAb; STI-B0234, a fully human anti-CCR2 mAb; TAK-779, a small molecule CCR2/5 antagonist; and TLK-19705, small molecule CCR2 antagonist.

The problem underlying the present invention is solved in a first aspect by an inhibitor of Monocyte Chemoattractant Protein-1 (CCL2) activity, for use in a method for treating a subject suffering from or being at risk of suffering from an eye disease, wherein the eye disease is a back of the eye disease selected from the group consisting of age-related macular degeneration (AMD), diabetic retinopathy (DR) and proliferative vitreoretinopathy (PVR).

The problem underlying the present invention is solved in a second aspect by a method for treating a subject suffering from or being at risk of suffering from an eye disease, wherein the eye disease is a back of the eye disease selected from the group consisting of age-related macular degeneration (AMD), diabetic retinopathy (DR) and proliferative vitreoretinopathy (PVR) and wherein the method comprises administering the inhibitor of CCL2 activity to the subject.

The problem underlying the present invention is solved in a third aspect by the use of an inhibitor of CCL2 activity for the manufacture of a medicament for the prevention or treatment of a disease, wherein disease is a back of the eye disease selected from the group comprising of age-related macular degeneration (AMD), diabetic retinopathy (DR) and proliferative vitreoretinopathy (PVR).

In an embodiment of each and any aspect of the present invention, the inhibitor of CCL2 activity is preferably an L-nucleic acid molecules binding to CCL2 disclosed herein and also described, for example, in WO 2007/093409, and more preferably compound NOX-E36.

Anti-angiogenic therapies and VEGF inhibitors are known in the art. The following agents represent examples thereof.

In a preferred embodiment of the present invention, the anti-angiogenic compound is an anti-VEGF antibody. The anti-VEGF antibody may be a human, humanized or chimeric anti-VEGF antibody. The term anti-VEGF antibody as preferably used herein, also encompassed any fragment of such anti-VEGF antibody, whereby such fragment is still binding or still capable of binding to VEGF.

In a preferred embodiment of the present invention, the anti-angiogenic antibody comprises the following light chain CDRS: CDR1: QDISNY (SEQ ID NO: 226), CDR2: FTS, and CDR3: QQYSTVPWT (SEQ ID NO: 227); or the following heavy chain CDRS: CDR1: GYTFTNYG (SEQ ID NO: 228), CDR2: INTYTGEP (SEQ ID NO: 229), and CDR3: AKYPHYYGSSHWYFDV (SEQ ID NO: 230), or a combination of the following light chain CDRS: CDR1: QDISNY (SEQ ID NO: 226), CDR2: FTS, and CDR3: QQYSTVPWT (SEQ ID NO: 227); and the following heavy chain CDRS: CDR1: GYTFTNYG (SEQ ID NO: 228), CDR2: INTYTGEP (SEQ ID NO: 229), and CDR3: AKYPHYYGSSHWYFDV (SEQ ID NO: 230).

In another preferred embodiment of the present invention, the anti-angiogenic antibody comprises a light chain comprising the following amino acid sequence: 
DIQMTQSPSSLSASVGDRVTITCSASQDISNYLNWYQQKPGKAPKVLIYFT
SSLHSGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 239),
a heavy chain comprising the following amino acid sequence: 
EVQLVESGGGLVQPGGSLRLSCAASGYTFTNYGMNWVRQAPGKGLEWVGWINTYTGE
PTYAADFKRRFTFSLDTSKSTAYLQMNSLRAEDTAVYYCAKYPHYYGSSHWYFDVWG
QGTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGK (SEQ ID NO: 240), or
 a combination of a light chain comprising the following amino acid sequence: 
DIQMTQSPSSLSASVGDRVTITCSASQDISNYLNWYQQKPGKAPKVLIYFT
SSLHSGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 239, and
a heavy chain comprising the following amino acid sequence:

EVQLVESGGGLVQPGGSLRLSCAASGYTFTNYGMNWVRQAPGKGLEWV
GWINTYTGEPTYAADFKRRFTFSLDTSKSTAYLQMNSLRAEDTAVYYCAKYPHYYGSS
HWYFDVWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTP

PVLDSDGSFFLYSKLTVDKSRWQQGNVFSCSVMHEALHNHYTQKSLSLSPGK (SEQ ID
NO: 240).

In a preferred embodiment of the present invention, the anti-VEGF-antibody is Bevacizumab. Bevacizumab is a recombinant humanized monoclonal antibody to VEGF; USAN/BAN/JAN Name: Bevacizumab; Laboratory Code: RO487-6646; CAS Registry Number: 216974-75-3; Bevacizumab is also referred to as rhuMAb VEGF, and anti-VEGF.

Bevacizumab is an inhibitor of angiogenesis. As such, it prevents the formation of new vasculature. Bevacizumab binds selectively with high affinity to all isoforms of human VEGF, the key driver of angiogenesis, and thereby inhibits the binding of VEGF to its receptors (Flt-1, VEGFR1 and KDR, VEGFR2) on the surface of endothelial cells. Neutralizing the biological activity of VEGF through a steric blocking of the binding of VEGF to its receptors regresses the vascularization of tumors, normalizes remaining tumor vasculature, and inhibits the formation of new tumor vasculature, thereby inhibiting tumor growth. Receptors activation normally induces their tyrosine phosphorylation and the subsequent series of signal transduction events elicit mitogenic and pro-survival activity signals for the vascular endothelial cells. Since there is a very low or undetectable expression of VEGF receptors in most normal tissues (with exception of renal glomeruli) but a significant up-regulation in the vasculature of many tumors, the neutralization of VEGF by bevacizumab provides the rationale a relative specific inhibition of the tumor angiogenesis and thereby inhibition of tumor growth and metastasizing.

Bevacizumab is a recombinant humanized monoclonal IgG1κ isotype antibody that contains human framework regions (93%) and murine complementarity-determining regions (7%). The antibody is composed of two identical light chains (214 amino acid residues) and two heavy chains (453 residues) with a total molecular weight of 149 kDa. The heavy chains demonstrate C-terminal heterogeneity (lysine variants) and also contain one N-linked glycosylation site at asparagine 303. The oligosaccharides are of complex biantennary structures with a core fucose and with the two branches terminating mainly with zero (G0), one (G1) or two (G2) galactose residues. The G0 glycoform predominates at approximately 80 % relative abundance. Each light chain is covalently coupled through a disulfide bond at cysteine 214 to a heavy chain at cysteine 226. The two heavy chains are covalently coupled to each other through two inter-chain disulfide bonds, which is consistent with the structure of a human IgG1. Bevacizumab was generated by humanization of the murine parent antibody A4.6.1 which was produced at Genentech using hybridomas generated from mice immunized with the 165-residue-form of recombinant human vascular endothelial growth factor (rhuVEGF165) conjugated with keyhole limpet hemocyanin.

The humanization of the A4.6.1 antibody involved insertion of the six CDRs of A4.6.1, in place of those of a selected human antibody Fab framework (pEMX1), which has a consensus human kappa subgroup I light chain (domains VL-CL) and a truncated human subgroup III immunoglobulin gamma (IgG1) heavy chain (domains VH-CH1). A series of framework residue substitutions were made to produce the final humanized version, Fab-12, which contains eight substitutions of the human framework outside of the CDRs. The VH and VL domains of Fab-12 were combined with human IgG1 constant domains CH1-CH2-CH3 and CL, respectively, to produce bevacizumab. The expression plasmid pSVID5.ID.LLnspeV.xvegf36HC.LC encoding bevacizumab was introduced into Chinese hamster ovary parental cells CHO DP-12 by lipofection and cells were selected in the presence of increasing concentrations of methotrexate (MTX). Isolates were selected for secretion of active bevacizumab. Isolated subclone 107N was used for the production of phase I and phase II clinical materials and as the starting point for the development of the more highly productive G7 cell line, which was used for the production of phase III clinical materials and which will be used for the production of drug substance intended for marketing.

In another embodiment of the present invention, the anti-VEGF antibody is ranibizumab (Lucentis; also Roche). The CDRs of bevacizumab and ranibizumab are very similar but have few changes (see IMGT Repertoire (IG and TR obtainable from https://www.imgt.org/IMGTrepertoire/GenesClinical/humanized/bevacizumab/bevacizumab_Pro teinDisplay.html). Ranibizumab is the Fab fragment of bevacizumab. In ranibizumab, 6 amino acids are modified thereof 4 in the CDRs. Ranibizumab is not glycosylated. More specifically, ranibizumab has the following amino acid changes compared to bevacizumab: in the VH (CDR1-IMGT: T29→D, N36→H, CDR3-IMGT: H109→Y, S112→T) and one amino acid change in V-KAPPA (M4→L). There is also an additional change in the hinge (T10>L) of bevacizumab.

In a still other embodiment of the present invention, the anti-angiogenic compound binding to VEGF is a VEGF-binding protein. Preferably such VEGF-binding protein is a functional derivative of a VEGF receptor, more preferably of VEGFR1 and/or VEGFR2. In a particularly preferred embodiment, the anti-angiogenic compound is ziv-aflibercept (Zaltrap), a 'VEGF trap' that inhibits VEGF-A, VEGF-B and PIGF-2. Zaltrap which is also referred to as aflibercept or also known as VEGF TRAP in the scientific literature, is a recombinant fusion protein consisting of VEGF-binding portions from the extracellular domains of human VEGF receptors 1 and 2 fused to the Fc portion of the human IgG1. Aflibercept is produced by recombinant DNA technology in a Chinese hamster ovary (CHO) K-1 mammalian expression system. Aflibercept is a dimeric glycoprotein with a protein molecular weight of 97 kilodaltons (kDa) and contains glycosylation, constituting an additional 15 % of the total molecular mass, resulting in a total molecular weight of 115 kDa. Aflibercept acts as a soluble decoy receptor that binds to VEGF-A, with higher affinity than its native receptors, as well as the related ligands PlGF and VEGF-B. By acting as a ligand trap, aflibercept prevents binding of endogenous ligands to their cognate receptors and thereby blocks receptor mediated signaling. Aflibercept blocks the activation of VEGF receptors and the proliferation of endothelial cells, thereby inhibiting the growth of new vessels that supply tumours with oxygen and nutrients. Aflibercept binds to human VEGF-A (equilibrium dissociation constant KD of 0.5 pM for VEGF-A165 and 0.36 pM for VEGF-A121), to human PlGF (KD of 39 pM for PlGF-2), and to human VEGF-B (KD of 1.92 pM) to form a stable, inert complex which has no detectable biological activity (see, European Medicines Agency, entry related to zaltrap: https://www.ema.europa.eu/en/medicines/human/EPAR/zaltrap).

In an embodiment of the present invention, the anti-angiogenic compound is a compound binding to a VEGFR. VEGFR may be VEGFR1, VEGFR2 or a combination thereof. In a more preferred embodiment, the anti-angiogenic compound binding to a VEGFR is an anti-VEGFR antibody.

In an embodiment of the present invention, the anti-VEGFR antibody comprises the following light chain CDRs CDR1: RASQGIDNWLG (SEQ ID NO: 233), CDR2: DASNLDT (SEQ ID NO: 234), and CDR3: QQAKAFPPT (SEQ ID NO: 235), or the following heavy chain CDRs CDR1: GFTFSSYSMN (SEQ ID NO: 236), CDR2: SISSSSSYIYYADSVKG (SEQ ID NO: 237) and CDR3: VTDAFDI (SEQ ID NO: 238), or the following light chain CDRs CDR1: RASQGIDNWLG (SEQ ID NO: 233), CDR2: DASNLDT (SEQ ID NO: 234), and CDR3: QQAKAFPPT (SEQ ID NO: 235), and the following heavy chain CDRs CDR1: GFTFSSYSMN (SEQ ID NO: 236), CDR2: SISSSSSYIYYADSVKG (SEQ ID NO: 237) and CDR3: VTDAFDI (SEQ ID NO: 238).

In an embodiment of the present invention, the anti-VEGFR antibody comprises the following light chain DIQMTQSPSS VSASIGDRVT ITCRASQGID NWLGWYQQKP GKAPKLLIYD ASNLDTGVPS RFSGSGSGTY FTLTISSLQA EDFAVYFCQQ AKAFPPTFGG GTKVDIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC (SEQ ID NO: 239), or the following EVQLVQSGGG LVKPGGSLRL SCAASGFTFS SYSMNWVRQA PGKGLEWVSS ISSSSSYIYY ADSVKGRFTI SRDNAKNSLY LQMNSLRAED TAVYYCARVT DAFDIWGQGT MVTVSSASTK GPSVLPLAPS SKSTSGGTAA LGCLVKDYFP EPVTVSWNSG ALTSGVHTFP AVLQSSGLYS LSSVVTVPSS SLGTQTYICN VNHKPSNTKV DKRVEPKSCD KTHTCPPCPA PELLGGPSVF LFPPKPKDTL MISRTPEVTC VVVDVSHEDP EVKFNWYVDG VEVHNAKTKP REEQYNSTYR VVSVLTVLHQ DWLNGKEYKC KVSNKALPAP IEKTISKAKG QPREPQVYTL PPSREEMTKN QVSLTCLVKG FYPSDIAVEW ESNGQPENNY KTTPPVLDSD GSFFLYSKLT VDKSRWQQGN VFSCSVMHEA LHNHYTQKSL SLSPGK (SEQ ID NO: 240), or a combination of the following light chain CDRs CDR1: RASQGIDNWLG (SEQ ID NO: 233), CDR2: DASNLDT (SEQ ID NO: 234), and CDR3: QQAKAFPPT (SEQ ID NO: 235), or the following heavy chain CDRs CDR1: GFTFSSYSMN (SEQ ID NO: 236), CDR2: SISSSSSYIYYADSVKG (SEQ ID NO: 237) and CDR3: VTDAFDI (SEQ ID NO: 238), or the following light chain CDRs CDR1: RASQGIDNWLG (SEQ ID NO: 233), CDR2: DASNLDT (SEQ ID NO: 234), and CDR3: QQAKAFPPT (SEQ ID NO: 235), and the following heavy chain CDRs CDR1: GFTFSSYSMN (SEQ ID NO: 236), CDR2: SISSSSSYIYYADSVKG (SEQ ID NO: 237) and CDR3: VTDAFDI (SEQ ID NO: 238).

In an embodiment of the present invention, the anti-VEGFR antibody comprises the following light chain DIQMTQSPSS VSASIGDRVT ITCRASQGID NWLGWYQQKP GKAPKLLIYD ASNLDTGVPS RFSGSGSGTY FTLTISSLQA EDFAVYFCQQ AKAFPPTFGG GTKVDIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC (SEQ ID NO: 239), and the following EVQLVQSGGG LVKPGGSLRL SCAASGFTFS SYSMNWVRQA PGKGLEWVSS ISSSSSYIYY ADSVKGRFTI SRDNAKNSLY LQMNSLRAED TAVYYCARVT DAFDIWGQGT MVTVSSASTK GPSVLPLAPS SKSTSGGTAA LGCLVKDYFP EPVTVSWNSG ALTSGVHTFP AVLQSSGLYS LSSVVTVPSS SLGTQTYICN VNHKPSNTKV DKRVEPKSCD KTHTCPPCPA PELLGGPSVF LFPPKPKDTL MISRTPEVTC VVVDVSHEDP EVKFNWYVDG VEVHNAKTKP REEQYNSTYR VVSVLTVLHQ DWLNGKEYKC KVSNKALPAP IEKTISKAKG QPREPQVYTL PPSREEMTKN QVSLTCLVKG FYPSDIAVEW ESNGQPENNY KTTPPVLDSD GSFFLYSKLT VDKSRWQQGN VFSCSVMHEA LHNHYTQKSL SLSPGK (SEQ ID NO: 240).

In another embodiment of the present invention, the anti-angiogenic compound binding to VEGF is an aptamer, i.e. a D-nucleic acid, more specifically pegaptanib (Macugen). Pegaptanib (Macugen) is an anti-VEGF aptamer, developed as an anti-angiogenic medicine for the treatment of neovascular (wet) age-related macular degeneration. Pegaptanib is a pegylated aptamer that specifically binds to the 165 isoform of VEGF.

More preferably, the anti-VEGFR antibody is Ramucirumab (Cyramza), which is a specific VEGFR2 inhibitor. The amino acid sequence of the heavy chain of ramucirumab is 
EVQLVQSGGG LVKPGGSLRL SCAASGFTFS SYSMNWVRQA
PGKGLEWVSS ISSSSSYIYYADSVKGRFTI SRDNAKNSLY LQMNSLRAED
TAVYYCARVT DAFDIWGQGT MVTVSSASTKGPSVLPLAPS SKSTSGGTAA
LGCLVKDYFP EPVTVSWNSG ALTSGVHTFP AVLQSSGLYSLSSVVTVPSS
SLGTQTYICN VNHKPSNTKV DKRVEPKSCD KTHTCPPCPA
PELLGGPSVFLFPPKPKDTL MISRTPEVTC VVVDVSHEDP EVKFNWYVDG
VEVHNAKTKP REEQYNSTYRVVSVLTVLHQ DWLNGKEYKC KVSNKALPAP
IEKTISKAKG QPREPQVYTL PPSREEMTKNQVSLTCLVKG FYPSDIAVEW
ESNGQPENNY KTTPPVLDSD GSFFLYSKLT VDKSRWQQGNVFSCSVMHEA
LHNHYTQKSL SLSPGK (SEQ ID NO: 241);
 and the light chain of ramucirumab is

DIQMTQSPSS VSASIGDRVT ITCRASQGID NWLGWYQQKP
GKAPKLLIYD ASNLDTGVPSRFSGSGSGTY FTLTISSLQA EDFAVYFCQQ
AKAFPPTFGG GTKVDIKRTV AAPSVFIFPPSDEQLKSGTA SVVCLLNNFY
PREAKVQWKV DNALQSGNSQ ESVTEQDSKD STYSLSSTLTLSKADYEKHK
VYACEVTHQG LSSPVTKSFN RGEC (SEQ ID NO: 242).

These sequences may be taken from the information provided by the following link: https://www.genomejp/entry/D09371.

In another embodiment of the present invention, the anti-angiogenic compound is a compound which inhibits signaling of a VEGFR, preferably VEGFR1 and VEGFR2. It is, however, also within the present invention that the signaling of one or more of the following receptors is inhibited: platelet-derived growth factor receptor (PDGFR), fibroblast growth factor receptor (FGFR) and angiopoietin-1 receptor (Tie-2).

In a preferred embodiment of the present invention, the anti-angiogenic compound inhibits the signal cascade triggered upon binding of VEGF to the VEGFR. Assays used for determining whether a compound inhibits said signal cascade, are known in the art. More specifically, cell culture assays have been used for such purpose and the characterization of inhibitors of VEGFR. The assays detect activation of intracellular signaling pathways, which are in the case of VEGFR the Ras/MAPK pathway, regulating cell proliferation and gene expression; the FAK/paxillin pathway, involved in the rearrangement of the cytoskeleton; the PI3K/AKT pathway, regulating cell survival; the PLCγ pathway, controlling vascular permeability.

More preferably, the anti-angiogenic compound inhibits a tyrosine kinase activity of the VEGFR. In such embodiment, the anti-angiogenic compound is a tyrosine kinase inhibitor, more preferably a multi-tyrosine kinase inhibitor.

In a more preferred embodiment of the present invention, the anti-angiogenic compound being a tyrosine kinase inhibitor is selected from the group comprising the tyrosine kinase inhibitor is selected from the group comprising pazopanib (Votrient inhibiting VEGFR1-3, PDGFRα and β, FGFR1 and 3, KIT, ITK, LCK, c-FMS), sunitinib (Sutent inhibiting VEGFR1-3, PDGFRα and β, KIT, FLT-3, CSF-1R, RET,), sorafenib (Nexavar inhibiting intracellular c-RAF, BRAF and mutant BRAF) and cell surface kinases (KIT, FLT-3, RET, RET/PCT, VEGFR1-3, PDGFR-β), axitinib (Inlyta inhibiting VEGFR1-3), ponatinib (Iclusig inhibiting VEGFR, PDGFR, FGFR, EPH receptors, SCR family of kinases, KIT, RET, TIE2 and FLT3), cabozantinib (Cometriq/Cabometyx inhibiting RET, MET, VEGFR1-3, KIT, TKRB, FLT-3, AXL, ROS1, TYRO3, MER, TIE-2), regorafenib (Stivarga inhibiting RET, VEGFR1-3, KIT, PDGFRα and β, FGFR1-2, TIE2, DDR2, TRKA, EPH2A, RAF-1, BRAF, BRAF V600E, SAPK2, PTKS, ABL, CSF1R), vandetanib (Caprelsa inhibiting EGFR and VEGFR families, RET, BRK, TIE-2, members of EPHR and Src kinase families) and lenvatinib (Lenvima inhibiting VEGFR1-3, FGFR1-4, PDGFRα, KIT and RET).

Age-related macular degeneration (AMD) is a leading cause of blindness in adults 60 years and older in the industrialized world. The estimated global prevalence of AMD is 8.7% and in 2020, it was estimated that over 190 million worldwide and over 11 million individuals in the US were affected by AMD (Apte 2021), and as prevalence is expected to increase as the population ages, it is predicted to affect around 288 million people in 2040 (Wong, Su et al. 2014).

AMD is a progressive, neurodegenerative disease of the retina which primarily affects the macula, which is the cone photoreceptor-rich central part of the retina and is initially characterized by focal or diffuse lipoprotein-rich deposits called drusen that form underneath the retinal pigment epithelium or sub-retinal drusenoid deposits that accumulate under the neurosensory retina (Rudolf, Malek et al. 2008, Sene, Chin-Yee et al. 2015).

While the natural history of AMD is one of relentless progression, treatments are available that reduce disease progression or vision loss. Micronutrient supplementation with a formulation containing vitamin C, zinc, vitamin E, copper, and beta carotene helps to reduce the probability of progression to advanced AMD (Apte 2021), in addition patients should be counseled regarding smoking cessation and adequate control of blood pressure in those with hypertension, given reported associations between these factors and AMD risk.

In patients with neovascular AMD, VEGF-targeted therapies have revolutionized care by the ability to prevent catastrophic vision loss (Apte, Chen et al. 2019). Multiple randomized clinical trials have demonstrated that intravitreal injections of anti-VEGF agents to treat choroidal neovascularization in neovascular AMD reduced the risk of moderate vision loss to less than 10% over a 24-month period, compared to about 50% in the control group (Heier, Brown et al. 2012).

There are several VEGF-A antagonists currently used in clinical practice; all require multiple, intravitreal injections for sustained therapeutic benefit. On average, patients require 7-8 injections during the initial 12 months of therapy; the need for continued injections is determined on clinical examination and by optical coherence tomography (Comparison of Age-related Macular Degeneration Treatments Trials Research, Martin et al. 2012).

Treatment goals include resolution of fluid and hemorrhage associated with neovascular AMD. Although sustained benefit is often achieved with anti-VEGF pharmacotherapy, in some cases choroidal neovascularization persists or recurs, and partial regression in the initial visual acuity gains have been observed at or after 5 years of treatment. (Bhisitkul, Mendes et al. 2015, Sene, Chin-Yee et al. 2015, Apte, Chen et al. 2019). In addition to the challenges associated with treating choroidal neovascularization, substantial evidence also implicates atrophy and retinal neurodegeneration in the pathogenesis of long term vision loss (Bhisitkul, Mendes et al. 2015, Grunwald, Pistilli et al. 2015).

Other treatments of AMD include inhibitors of angiopoietin-2 such as the antibody faricimab which in addition to VEGF also targets angiopoietin-2, inhibitors of factors of the complement system components C3 such as pegcetacoplan and C5 such as avancincaptad pegol, and steroids such as triamcinolone (Bandello, Preziosa et al. 2014, Heier, Khanani et al. 2022, Wilke and Apte 2024).

Diabetic retinopathy (DR) is the most common microvascular complication of diabetes remains a leading cause of vision loss globally (Antonetti, Klein et al. 2012). DR currently affects almost 100 million people worldwide and is set to become an ever-increasing health burden, with estimates between 1990 and 2010 showing that DR-related visual impairment and blindness increased by 64% and 27%, respectively (Leasher, Bourne et al. 2016).

The etiology and pathology of diabetic retinopathy have been extensively studied for half a century, yet there are disappointingly few therapeutic options. Intraocular treatment modalities for diabetic eye disease include laser photocoagulation, intravitreous injections of anti-VEGF and steroid agents, and vitreoretinal surgery (Duh, Sun et al. 2017).

Proliferative vitreoretinopathy (PVR) is the main cause of failure after retinal detachment (RD) surgical repair and can also occur after ocular trauma (Idrees, Sridhar et al. 2019) and a demanding challenge for vitreoretinal surgeons. PVR is caused by the formation and contraction of proliferative cellular membranes in the vitreous cavity on both retinal sides, leading to the possibility of a tractional RD with fixed retinal folds (Pastor, Rojas et al. 2016).

In the last years, despite the improvements in vitreoretinal surgery techniques, the incidence of PVR has remained stable, ranging from 5 to 10% of the cases after RD repair surgery. It has been reported that almost 77% of cases of PVR occurs within 1 month after RD surgery and 95% of them within 3 months (Claes and Lafeta 2014).

Surgical repair currently represents the mainstay for the treatment of PVR (Coffee, Jiang et al. 2014). Although there have been recent advances in the surgical management of RD with PVR, the recurrence rate of RD due to PVR itself continues to be a significant limitation to success (Charteris 2020). In this regard, the Silicone Oil Study showed a limited success rate in PVR surgery (35-42%) and unsatisfactory visual outcomes (1992). In series with established PVR at presentation, the primary overall success rate has been reported between 43 and 69%; although cases can be treated with good anatomical results, visual outcomes are often less favorable, and more reoperations are required compared with standard retinal detachments (Anguita, Roth et al. 2021). Studies have referred a best-corrected visual acuity (BCVA) of ≥ 5/200 in 40-80% of the patients following to RD repair with PVR (Pastor, de la Rua et al. 2002). Other clinical studies showed that eyes that developed PVR resulting in worse visual outcomes (Wickham, Ho-Yen et al. 2011).

PVR remains a challenging condition and the main cause of RD failure for vitreoretinal specialists. Although several different advantages in vitreoretinal surgery techniques have been obtained, to date, functional and anatomical outcomes in patients with PVR are still unsatisfactory (Charteris 2020). Due to this unmet need, various adjunctive treatment options have been investigated for the management of PVR. However, to date, no pharmaceutical agents have been approved as adjunctive treatment in combination with surgery for treating PVR.

It will be understood that according to the present invention including each and any aspect thereof, including any embodiment of such each and any aspect, the inhibitor of CCL2 activity is administered to an eye of the subject to be treated for a back of the eye disease, or treated for prevention of such back of the eye disease, preferably an eye of the subject suffering from AMD, DR or PVR.

Fibrosis is a reaction observed in back of the eye diseases and AMD, DR and PVR in particular.

In general, fibrosis is a histopathological definition in which there is permanent deposition of extracellular matrix (ECM) components that include collagen, fibronectin, elastin, and others, as a result of tissue injury. Fibrosis is a stereotypical physiological response aimed at restoring anatomical integrity with minimal or no return of function (Armendariz and Chakravarthy 2024). The functional impairment caused by permanent fibrotic tissue deposition is seen in a spectrum of diseases including interstitial lung disease, end-stage liver and kidney diseases, heart failure and nAMD (Franceschi, Bonafe et al. 2000). Fibrosis is characterized by four stages: inflammatory, proliferative, matrix remodelling and wound quiescence (Singer and Clark 1999). In the proliferative phase, there is formation of provisional matrix that functions as a scaffold for permanent matrix deposition, i.e. a scar or fibrotic tissue.

In the context of an aging disease like nAMD, the different components that contribute to the early pathophysiology (e.g. neovascularization and leakage, disruption of the blood-retina- barrier, etc) are also instrumental in eventual fibrosis development (Ishikawa, Kannan et al. 2016). This alteration of the homeostasis and the hostile microenvironment may induce epithelial-mesenchymal transition (EMT), a process by which epithelial cells de-differentiate into myofibroblasts and that constitutes a key step in the development of fibrosis (Shu, Butcher et al. 2020, Liu, Zhang et al. 2023). Blood constituents, an essential component of the provisional matrix, create the permissive environment for fibroblast proliferation and ultimately collagen deposition (Singer and Clark 1999). Thus, fibrosis could be considered an inevitable consequence of nAMD: ultimately, fibrosis is the attempt by the body to restore the outer blood retinal barrier.

Fibrosis has, however, a direct impact on vision. Fibrosis in the outer retina arising secondary to neovascularization in AMD has profound effects on central retinal function even though the exudative process may be controlled by anti VEGF treatments. Subretinal and subfoveal fibrosis have larger impact on vision loss than fibrosis located underneath the RPE or outside of the fovea. However, any alteration of the structure of the eye and the correct functioning of its cells may lead to vision impairment (Armendariz and Chakravarthy 2024). The pathological changes of particular note are the replacement by fibrocytes and collagen of photoreceptor matrix, retinal pigment epithelium and choriocapillaris. In addition, the contraction of the fibrous components leads to disorganisation and distortion of any residual neural and retinal pigment epithelium elements. The consequence of such pathological processes is progressive visual impairment. In fact, AMD leads to blindness when left untreated, due to the development of atrophy and/or disciform scar. And even with intensive therapy, the main reasons for vision loss are atrophy and macular scar formation (Hogg, Curry et al. 2003, Daniel, Pan et al. 2018, Jaffe, Ying et al. 2019), underscoring the impact of fibrosis in functional outcomes. Whether fibrosis impacts vision or not is determined by the status of the overlying photoreceptors and outer retinal layers. Fibrosis may be associated, thus, with atrophic processes. In fact, patients with fibrosis showed a higher percentage of retinal pigment epithelium atrophy and Ellipsoid Zone disruption (Querques, Parravano et al. 2020), and reduced retinal sensitivity assessed with microperimetry (Roh, Lains et al. 2019, Querques, Parravano et al. 2020, Schranz, Sacu et al. 2024). Fibrosis in the back of the eye can be assessed using fluorescence angiography (FA) and colour fundus photography (CFP), with optical coherence tomography (OCT) introduced more recently. All have their advantages and limitations, which is the reason why a multimodal imaging approach is often preferred (Bachmeier, Armendariz et al. 2023).

AMD is a progressive, neurodegenerative disease of the retina which primarily affects the macula, which is the cone photoreceptor-rich central part of the retina and is initially characterized by focal or diffuse lipoprotein-rich deposits called drusen that form underneath the retinal pigment epithelium or sub-retinal drusenoid deposits that accumulate under the neurosensory retina (Rudolf, Malek et al. 2008, Sene, Chin-Yee et al. 2015). Drusen are also associated with macular pigmentary changes and thickening of the acellular Bruch's membrane lamina underneath the retinal pigment epithelium. Soft, large drusen are associated with increased risk of disease progression. Based on macular drusen area and size, AMD at this stage is classified as early or intermediate (Davis, Gangnon et al. 2005). Longitudinal data from the Age-Related Eye Disease Studies demonstrated that patients with intermediate AMD can progress to advanced stages of disease classified as; (i) neovascular or 'wet' AMD characterized by proliferative neovascularization underneath the neurosensory retina, also called choroidal neovascularization and (ii) atrophic dry AMD characterized by atrophy of the retinal pigment epithelium and the overlying neurosensory retina, called geographic atrophy (Davis, Gangnon et al. 2005, Ferris, Davis et al. 2005). These studies also demonstrated that AMD progresses relentlessly without treatment and provided a simplified clinical scale for stratifying AMD risk at diagnosis and during follow-up.

In addition to choroidal neovascularization, two other forms of AMD-associated neovascularization have been described; polypoidal choroidal vasculopathy that is more prevalent in Asia, and retinal angiomatous proliferation, where the neovascularization is initiated within the retina, rather than in the choroid (Yannuzzi, Negrao et al. 2001). Choroidal neovascularization and geographic atrophy may develop in the same eye, as they represent a continuum of the same disease process, and vision loss ultimately occurs from secondary neurodegeneration associated with photoreceptor loss whether from proliferative neovascularization and fibrosis or atrophy. The wet form of AMD accounts for only about 10-15% of AMD patients but is responsible for the majority of cases of severe vision loss.

Fibrosis in the back of the eye is a significant problem because the human visual system is based on an exquisite interplay between the structure of the eye and the correct functioning of its cells. Thus, any alteration of these features may lead to vision impairment. The pathological changes of particular note are the replacement by fibrocytes and collagen of photoreceptor matrix, retinal pigment epithelium (RPE) and choriocapillaris. In addition, the contraction of the fibrous components leads to disorganisation and distortion of any residual neural and RPE elements. The consequence of such pathological processes is progressive visual impairment (Armendariz and Chakravarthy 2024). In fact, AMD leads to blindness when left untreated, due to the development of atrophy and/or disciform scar. And even with intensive therapy, the main reasons for vision loss are atrophy and macular scar formation (Hogg, Curry et al. 2003, Daniel, Pan et al. 2018, Jaffe, Ying et al. 2019), underscoring the impact of fibrosis in functional outcomes.

The impact of fibrosis on vision depends on its location within the retina: when there is deposition of material immediately external to the neurosensory retina and internal to the RPE, the interaction and communication between the photoreceptors and the RPE may be altered and can lead to the degeneration of the former with consequent visual decline. However, sometimes this fibrotic material can accumulate externally to the RPE (i.e. in the subRPE space). Interestingly, the effects on best corrected visual acuity (BCVA) are not so severe in these cases as compared with subretinal fibrosis (Ryu, Al-Humaid et al. 2016).

Since the advent of anti-VEGF therapies, which are very effective for controlling exudation, large disciform scars are rarely encountered in the clinic. However, long term studies show that smaller and less severe fibrotic scars are not uncommon and develop over time despite optimal treatment. In fact, despite the potentially devastating effects in vision of fibrosis in nAMD, there is no treatment approved yet (Armendariz and Chakravarthy 2024).

AMD is more common in people of European and North American ancestry compared to Asian, Hispanic or African ancestry, although regional differences and genetic predisposition contribute to high variability. The incidence of AMD is similar in women and men. Increasing age is the strongest risk factor, and with the exponential growth in the aging population, it is estimated that the global prevalence will increase to 288 million in 2040 (Wong, Su et al. 2014). Numerous studies have linked smoking, uncontrolled hypertension, and higher body mass index (>25 kg/m²) to more severe AMD (Seddon, Willett et al. 1996, Seddon, Cote et al. 2003, Seddon, George et al. 2006).

Based on their obvious manifestations during DR progression, microvascular lesions have been utilized as the major criteria for evaluating and classifying the retina in DR. However, diabetes-induced changes also occur in nonvascular cell types that play an important role in the development and progression of DR, albeit in unison with the vasculature. DR falls into 2 broad categories: the earlier stage of nonproliferative diabetic retinopathy (NPDR) and the advanced stage of proliferative diabetic retinopathy (PDR). Classification of NPDR is based on clinical findings manifested by visible features, including microaneurysms, retinal hemorrhages, intraretinal microvascular abnormalities, and venous caliber changes, while PDR is characterized by the hallmark feature of pathologic preretinal neovascularization (Stitt, Curtis et al. 2016, Joachim 2022). An important additional categorization in DR is diabetic macular edema (DME), which is an important manifestation of DR that occurs across all DR severity levels of both NPDR and PDR and represents the most common cause of vision loss in patients with DR. DME arises from diabetes-induced breakdown of the blood-retinal barrier (BRB), with consequent vascular leakage of fluid and circulating proteins into the neural retina (Stitt, Curtis et al. 2016). The extravasation of fluid into the neural retina leads to abnormal retinal thickening and often cystoid edema of the macula.

The hallmark microvascular features of NPDR include intraretinal hemorrhages, microaneurysms, venous caliber abnormalities, formation of intraretinal microvascular abnormalities, lipid exudates from the damaged vasculature, capillary nonperfusion with accompanying neuronal infarcts represented as cotton-wool spots, and retinal neovascularization. Retinal vascular lesions, which have been well-characterized histologically in postmortem human eyes and in some long-term preclinical models, are preceded and/or accompanied by vascular basement membrane thickening, an early loss of pericytes, and eventual death of endothelial cells that underpin the vasodegenerative pathology that is a feature of early DR (Curtis, Gardiner et al. 2009). Several retinal vascular pathologic processes in DR have a direct impact on vision. In NPDR, gradual nonperfusion of the retinal vascular bed, characterized by loss of vessel integrity, ultimately leads to occlusion or degeneration of capillaries (Stitt, Curtis et al. 2016). Localized capillary nonperfusion results in regions of ischemia and impaired oxygenation of the metabolically demanding retinal neurons. Progressive capillary nonperfusion and resultant ischemia underpin progression to PDR, which is driven by hypoxia and expression of proangiogenic growth factors, which stimulate the aberrant formation of new blood vessels in the retina that protrude into the preretinal space. Retinal neovascularization can result in severe vision loss when it leads to vitreous hemorrhage or tractional retinal detachment. Another major pathologic process is DME, which is characterized by overt breakdown of the BRB that leads to macular edema and swelling of the neuropile, which frequently leads to vision loss.

A number of processes are key steps in the development and progression of PDR: cell proliferation, extracellular matrix expansion, and neovascularization (Ban and Twigg 2008). These events are typically growth factor-driven in response to hypoxia and inflammatory insults, and they promote the formation of fibrotic tissue on the retinal surface or in the vitreous cavity (Friedlander 2007, Ban and Twigg 2008). Ultimately, the tractional forces generated by the fibrotic process during neovascularization result in the separation of the inner neurosensory retina from the outer retinal pigment epithelium (RPE) resulting in retinal detachment (Yang, Su et al. 2008). Although it is unclear which cell types participate in generating tractional force contributing to retinal detachment in PDR, Müller cells are known to produce stress fibers that are well established in providing mechanical strength to the retinal detachment process (Guidry, Bradley et al. 2003). Two types of fibrotic tissues, fibrovascular proliferative tissue and avascular proliferative tissue, can develop in patients with PDR and contribute to retinal detachment (McMeel 1971). Fibrovascular proliferative tissue is formed when abnormal new vessels grow during PDR on the retinal surface. Avascular proliferative tissue is less common and consists of amorphous avascular membranes. Of the three types of retinal detachment, rhegmatogenous, traction, and exudative, PDR is most often associated with traction retinal detachment. During traction retinal detachment, the scar tissue on the retinal surface "pulls" at the retina, detaching it from the underlying layer, whereas during rhegmatogenous retinal detachment, retinal tears develop allowing fluid accumulation in the subretinal space (Humphrey, Constable et al. 1993). This fluid together with the tractional force of the vitreous on the retina can promote retinal detachment.

While retinal neovessels are the primary target of clinical interventions in PDR, retinal fibrosis is another confounding factor for which therapeutic strategies are limited (Roy, Amin et al. 2016).

For patients with proliferative diabetic retinopathy (PDR), laser photocoagulation remains a mainstay therapy, even though it is an inherently destructive procedure (Stitt, Curtis et al. 2016). As the proangiogenic factors in DR lead almost exclusively to pathologic preretinal neovascularization rather than beneficial revascularization, extensive efforts have been made over decades to identify the major proangiogenic growth factors in PDR, such as VEGF (Aiello, Avery et al. 1994). As a result, anti-VEGF treatments have emerged as an effective approach for treatment of this condition and it is highly effective in regressing retinal neovascularization in eyes with PDR (Avery, Pearlman et al. 2006). Recent data suggest that anti-VEGF is a viable treatment alternative to laser photocoagulation in eyes with PDR, especially for individuals with coexisting DME that already necessitates anti-VEGF therapy. Eyes treated with anti-VEGF for PDR have equivalent visual acuity outcomes at the 2-year endpoint of the study, compared with those treated with laser photocoagulation. In addition, eyes treated with anti-VEGF exhibited better average visual acuity over the entire course of the 2-year study period (Writing Committee for the Diabetic Retinopathy Clinical Research, Gross et al. 2015). Additional benefits of anti-VEGF as compared with laser photocoagulation include significantly less peripheral visual field loss, decreased rates of DME onset, and fewer vitrectomies over 2 years. Despite these benefits, anti-VEGF therapy may not be optimal for patients who cannot comply with the near-monthly follow-up and injection regimen required for adequate treatment and prevention of PDR recurrences.

Vitreoretinal surgery is utilized for cases of nonclearing vitreous hemorrhage from PDR or cases of PDR with tractional retinal detachment to relieve fibrous attachments that may be distorting the retina and causing vision loss or metamorphopsia (Berrocal, Acaba et al. 2016). Vitrectomy with or without peeling of the internal limiting membrane can also be performed to treat DME, particularly when there is an epiretinal membrane or element of vitreoretinal traction leading to retinal thickening. Although retinal thickening is often improved after vitrectomy for DME, visual outcomes are less certain, with approximately a third of patients experiencing substantial visual improvement, but between 20%-30% experiencing substantial visual loss after surgery. Although current therapies are effective at preventing vision loss and frequently result in visual gain for patients with both PDR and DME, unmet treatment needs still exist.

Retinal fibrosis is closely associated with the development of PDR and is characterized by the presence of newly formed blood vessels often accompanied by a fibrous tissue spreading along the inner surface of the retina, across the optic disc, or extending into the vitreous cavity. Clinical studies have identified fibrotic membranes in PDR to exhibit uncontrolled growth and cause serious complications including retinal detachment, thereby contributing to vision loss. While retinal neovessels are the primary target of clinical interventions in PDR, retinal fibrosis is another confounding factor for which therapeutic strategies are limited. A variety of potential inhibitory modalities are being investigated for prevention of proliferative fibrotic membranes associated with PDR, including angiotensin converting enzyme inhibitors, VEGF inhibitors, CTGF inhibitors such as pamrevlumab, statins such as simvastatin, and steroids such as triamcinolone (Roy, Amin et al. 2016).

Proliferative vitreoretinopathy (PVR) is the main cause of failure after retinal detachment (RD) surgical repair (Idrees, Sridhar et al. 2019) and a demanding challenge for vitreoretinal surgeons. PVR is caused by the formation and contraction of proliferative cellular membranes in the vitreous cavity on both retinal sides, leading to the possibility of a tractional RD with fixed retinal folds (Pastor, Rojas et al. 2016). The Retina Society Terminology Committee first introduced the definition of PVR, suggesting a staging classification (A, B, C, D) in relation to disease severity, with stage A, minimal; B, moderate with surface retinal wrinkling, rolled edges of the retinal, retinal stiffness, and vessel tortuosity; C marked with full-thickness retinal folds; and D massive PVR with fixed retinal folds in four quadrants (1983); however, several limitations were still present with this definition, and further modifications were subsequently proposed, including one in 1989 by the Silicone Study Group, describing the location (anterior or posterior) and the type of contraction; this was followed by a further classification in 1991, in which a more detailed description of the anterior and posterior PVR was provided (1983, Machemer, Aaberg et al. 1991).

The pathogenesis of PVR is complex and relies on numerous interactions that are not yet fully understood. Various cell types are involved, such as glial cells, retinal pigment epithelial cells (RPE), inflammatory cells, and fibroblasts, which are regulated by numerous cytokines, inflammatory factors, and growth factors (Charteris, Hiscott et al. 1993, Pastor-Idoate, Rodriguez-Hernandez et al. 2015, Eastlake, Banerjee et al. 2016). Cell death pathways, including apoptosis and programmed necrosis, have also been found to be involved in retinal photoreceptor degeneration and cell death. These processes may play an important role in the development of proliferative vitreoretinopathy and provide clues to the imbalance in fibrosis formation during wound healing (Pastor-Idoate, Rodriguez-Hernandez et al. 2015). In recent years, it has been found that the influence of Müller glia on other retinal cells and their involvement in the biochemical cascade during PVR are more important than previously thought. The response of the Müller glia during PVR resembles the response to a pathogenic stimulus, resulting in hypertrophy, increased proliferation, and upregulation of intermediate filaments nestin, vimentin, and glial fibrillary acidic protein (Sethi, Lewis et al. 2005).

Preclinical studies have highlighted that macrophages are important players in the pathogenesis of PVR. Their presence in the vitreous has been related to a higher risk to develop PVR (Campochiaro 1997). In fact, these cells are more frequent in the vitreous body of those patients developing PVR after surgery in comparison with those who will not develop PVR (Martin, Pastor et al. 2003). Moreover, macrophages not only display a pro-inflammatory activity, but they can also mediate the apoptosis of photoreceptor cells through MCP-1(Nakazawa, Hisatomi et al. 2007). The complex interplay between different cell types (RPE cells glial cells and macrophages), cytokines, and other mediators involved in the pathogenesis of PVR creates a feedback loop maintaining the disease process.

Considering the pathogenetic mechanism of the disease, several different drugs targeting inflammation, cell proliferation, and fibrosis processes have been examined. Among others, corticosteroids such as intravitreal triamcinolone acetonide or systemic prednisolone or dexamethasone, different anti-proliferative agents such as 5-fluorouracil, mitomycin C, daunomycin and other substances like low-molecular weight heparin have shown promising preliminary results in preclinical models and clinical trials (Charteris 2020).

According to the present invention including each and any aspect thereof, including any embodiment of such each and any aspect, the inhibitor of CCL2 activity is administered locally. Such local administration includes but is not limited to intravitreal, suprachoroidal and subretinal administration.

It will be understood that according to the present invention including each and any aspect thereof, including any embodiment of such each and any aspect, systemic administration is selected from the group comprising subcutaneous administration, intravenous administration and any combination thereof. It will be equally understood that system administration encompasses any administration of an inhibitor of CCL2 activity to a subject where the inhibitor of CCL2 activity is administered to the body of the subject so that the inhibitor of CCL2 activity is transported in the vasculature of the body or the lymphatic system of the body.

It will be understood that according to the present invention including each and any aspect thereof, including any embodiment of such each and any aspect, the subject is suffering from or at risk of suffering from a back of the eye disease, preferably the subject is suffering from AMD, DR, preferably PDR, or PVR.

In embodiments of each and any aspect, including any embodiment of each and any aspect, and as preferably used herein an inhibitor of CCL2 activity is a compound which binds to CCL2 or a compound which binds to the receptor of CCL2. Preferably the receptor of CCL2 is CCR2. Preferable, the binding of the inhibitor of CCL2 activity to CCL2 inhibits the binding to CCR2 of CCL2 bound by the inhibitor of CCL2 activity. Also preferably, the binding of the inhibitor of CCL2 activity to CCR2 inhibits the binding of CCL2 to CCR2 bound by the inhibitor of CCL2 activity. In these embodiments, activity of the inhibitor of CCL2 activity is its activity of inhibiting the binding of CCL2 to its receptor CCR2 and, respectively, the binding of CCR2 to its ligand CCL2.

In an alternative embodiment of each and any aspect, including any embodiment of each and any aspect, and as preferably used herein, an inhibitor of CCL2 activity is a compound that binds to or is capable of binding to CCL2 (both terms are used herein synonymously), whereby CCL2 bound by the inhibitor of CCL2 activity shows or results in reduced activity of CCR2 signaling. Such reduced activity of CCR2 can be determined in or by means of a Ca²⁺-release assay described in Example 7 herein or can be determined in or by means of a chemotaxis assay as described in Example 8 herein. In this embodiment, activity of the inhibitor of CCL2 activity is its activity of reducing the activity of CCR2, preferably resulting from inhibiting or reducing the binding of CCL2 to CCR2, wherein the inhibitor of CCL2 activity is bound to CCL2. It will be appreciated by a person skilled in the art that, preferably, the term inhibiting is a generic term, and that, preferably, the term reducing is a generic term, whereby the term reducing refers to a generic numerical concept of inhibition.

In a further alternative embodiment of each and any aspect, including any embodiment of each and any aspect, and as preferably used herein, an inhibitor of CCL2 activity is a compound that binds to CCR2, whereby CCR2 bound by the inhibitor of CCL2 activity shows or results in reduced activity of CCR2, preferably because the binding of CCL2 to CCR2 is blocked or reduced. Such reduced activity of CCR2 can be determined in or by means of a Ca²⁺-release assay described in Example 7 herein or can be determined in or by means of a chemotaxis assay as described in Example 8 herein. In this embodiment, activity of the inhibitor of CCL2 activity is its activity of reducing the activity of CCR2, preferably resulting from inhibiting or reducing the binding of CCL2 to CCR2, wherein the inhibitor of CCL2 activity is bound to CCR2. It will be appreciated by a person skilled in the art that, preferably, the term inhibiting is a generic term, and that, preferably, the term reducing is a generic term, whereby the term reducing refers to a generic numerical concept of inhibition.

In an embodiment of each and any aspect, including any embodiment of each and any aspect, the inhibitor of CCL2 activity is selected from the group comprising an aptamer binding to CCL2, an aptamer binding to CCR2, a Spiegelmer binding to CCL2, a Spiegelmer binding to CCR2, an antibody binding to CCL2, an antibody binding to CCR2, an antibody fragment binding to CCL2, an antibody fragment binding to CCR2, a protein binding to CCL2, a protein binding to CCR2, an anticalin binding to CCL2, an anticalin binding to CCR2, a small molecule binding to CCL2 and a small molecule binding to CCR2.

In an embodiment of each and any aspect, including any embodiment of each and any aspect, a compound which may be used as an inhibitor of CCL2 activity is selected from the group comprising a compound targeting CCL2, wherein the compound is selected from the group comprising Bindarit, a small molecule CCL2 inhibitor; Carlumab (CNTO888), a fully-human anti-CCL2 Mab; ABN-912, a fully-human anti-CCL2 Mab; CGEN-54 and a recombinant CCL2-inhibiting protein.

In an embodiment of each and any aspect, including any embodiment of each and any aspect, a compound which may be used as an inhibitor of CCL2 activity is selected from the group comprising a compound targeting CCR2, wherein the compound is selected from the group comprising AZ-889, a small molecule CCR2 antagonist; AZD-2423 a small molecule CCR2 antagonist, a small molecule CCR2 antagonist; BL-2030, a soluble CCR2 receptor fused to a human antibody Fc component; BMS-741672, a small molecule CCR2 antagonist; BMS-753426, a small molecule CCR2 antagonist; BMS-813160, a small molecule CCR2 antagonist; CCX-140, a small molecule CCR2 inhibitor; CCX-598, a small molecule CCR2 inhibitor; CCX-872, a small molecule CCR2 inhibitor; CCX-915, a small molecule CCR2 inhibitor; cenicriviroc, a small molecule CCR2/CCR5 antagonist; CNTX-6970, a small molecule CCR2 antagonist; CPD-B, a small molecule CCR2 antagonist; EPX-102216, a small molecule CCR2 antagonist; INCB-3344, a small molecule CCR2 inhibitor; INCB-3284, a small molecule CCR2 inhibitor; INCB-8696, a small molecule CCR2 inhibitor; LF-0376, a small molecule CCR2/5 antagonist; MK-812, a small molecule CCR2 inhibitor; NIBR-6465, a small molecule CCR2/5 antagonist; OB-004 a small molecule CCR2 antagonist; OPL-CCL2-LPM, a human CCL2 chemokine fusion protein with cytotoxic payload; PD-172084, a small molecule CCR2 antagonist; PF-04634817, a small molecule CCR2/5 antagonist; PF-4136309, a small molecule CCR2 antagonist; plozalizumab (MLN-1202), a humanized anti-CCR2 mAb; R-103, an oral Dala1-peptide T-amide analogue targeting CCR2, 5 and 8; RAP-103; RAP-310, both small molecule CCR2/5 antagonists; SB-380732, a small molecule CCR2 antagonist; SPR-3, a small molecule CCR2 antagonist; STI-B0201, a fully human anti-CCR2 mAb; STI-B0211, a fully human anti-CCR2 mAb; STI-B0221, a fully human anti-CCR2 mAb; STI-B0234, a fully human anti-CCR2 mAb; TAK-779, a small molecule CCR2/5 antagonist; and TLK-19705, small molecule CCR2 antagonist.

Aptamers are nucleic acid molecules made of D-nucleotides as building blocks which specifically bind to a target molecule through a mechanism different from Watson Crick base pairing. Spiegelmers are nucleic acid molecules made of L-nucleotides as building blocks which specifically bind to a target molecule through a mechanism different from Watson Crick base pairing. Both aptamers and spiegelmers can be made of ribonucleotides, of deoxyribonucleotides are a combination of both ribonucleotides and deoxyribonucleotides. Aptamers and spiegelmers as such are known to a person skilled in the art and are, among others, described in 'The Aptamer Handbook' (eds. Klussmann, 2006) and spiegelmers comprising both ribonucleotides and deoxyribonucleotides are disclosed in international patent application WO 2012/095303.

Anticalines are a class of target binding polypeptides is, among others, described in German patent application DE 197 42 706.

The generation of antibodies, including antibody fragments against targets such as CCL2 and CCR2 is within the skills of an ordinary person of the art.

In accordance with the present invention and in an embodiment of each and any aspect of the present invention, including any embodiment of each and any aspect, the inhibitor of CCL2 activity is an L-nucleic acid molecule which is binding to MCP-1, wherein the L-nucleic acid molecule is selected from the group comprising type 1A nucleic acids, type 1B nucleic acids, type 2 nucleic acids, type 3 nucleic acids, type 4 nucleic acids and nucleic acids having a nucleic acid sequence according to any of SEQ.ID.NOs. 87 to 115. These L-nucleic acid molecules are, for example, disclosed in WO 2007/093409 the disclosure of which is incorporated herein by reference.

As outlined in more detail in the embodiments and example 4, these CCL2 binding nucleic acid molecules can be characterised in terms of stretches of nucleotide which are also referred to herein as Boxes. The various CCL2 binding nucleic acid molecules can be categorised based on said Boxes and some structural features and elements, respectively. The various categories thus defined are also referred to herein as types and more specifically as type 1A, type 1B, type 2, type 3 and type 4.

These nucleic acids molecules also comprise nucleic acids which are essentially identical or homologous to the particular sequences disclosed herein. The term substantially identical or homologous shall be understood such that the identity and homology, respectively, is at least 75%, preferably 85%, more preferably 90% and most preferably more than 95 %, 96 %, 97 %, 98 % or 99%. Both terms identity and homology are used interchangeably and synonymously herein, unless indicated otherwise. Also, the terms identical and homologous are used interchangeably and synonymously herein, unless indicated otherwise.

The actual percentage of identical nucleotides present in the nucleic acid according to the present invention will depend on the total number of nucleotides present in the nucleic acid. The percent modification can be based upon the total number of nucleotides present in the nucleic acid.

The homology or identity can be determined as known to the person skilled in the art. More specifically, a sequence comparison algorithm then calculates the percent sequence identity for the test sequence(s) relative to the reference sequence, based on the designated program parameters. The test sequence is preferably the sequence or nucleic acid molecule which is said to be or to be tested whether it is homologous, and if so, to what extent, to another nucleic acid molecule, whereby such another nucleic acid molecule is also referred to as the reference sequence. In an embodiment, the reference sequence is a nucleic acid molecule as described herein, more preferably a nucleic acid molecule having a sequence according to any of SEQ. ID. NOs. 10 to 129, 132 to 256 and 278 - 282. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul et al. 1990 and Altschul et al, 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis et al, 2004).

In an embodiment of each and any aspect, including any embodiment thereof, the terms nucleic acid and nucleic acid molecule are used interchangeably and synonymously herein, unless indicated otherwise.

If not indicated to the contrary, any nucleotide sequence is set forth herein in 5' → 3' direction.

The nucleic acid molecules binding to CCL2 or CCR2 may be modified. Such modifications may be related to the single nucleotide of the nucleic acid and are well known in the art. Examples for such modification are described in, among others, Venkatesan (2003); Kusser (2000); Aurup (1994); Cummins (1995); Eaton (1995); Green (1995); Kawasaki (1993); Lesnik (1993); and Miller (1993). Such modification can be a H atom, a F atom or O-CH3 group or NH2-group at the 2' position of the individual nucleotide of which the nucleic acid consists. Also, the nucleic acid according to the present invention can comprises at least one LNA nucleotide. In an embodiment the nucleic acid according to the present invention consists of LNA nucleotides.

The nucleic acid molecules suitable for the practicing of the present invention preferably exhibit a very favourable K_{D} value range.

A possibility to determine the binding constant is the use of the so called biacore device, which is also known to the one skilled in the art. Affinity as used herein was also measured by the use of the "pull-down assay" as described in the example part herein. An appropriate measure in order to express the intensity of the binding between the nucleic acid according to the target which is in the present case CCL2, is the so-called K_{D} value which as such as well the method for its determination are known to the one skilled in the art.

The nucleic acids nucleic acid molecules suitable for the practicing of the present invention are characterized by a certain K_{D} value. Preferably, the K_{D} value shown by the nucleic acid molecules is below 1 µM. A K_{D} value of about 1 µM is said to be characteristic for a nonspecific binding of a nucleic acid molecule to a target. As will be acknowledged by the ones in the art, the K_{D} value of a group of compounds such as the nucleic acid molecule suitable for the practicing of the present invention are within a certain range. The above-mentioned K_{D} of about 1 µM is a preferred upper limit for the K_{D} value. The preferred lower limit for the K_{D} of target binding nucleic acid molecules can be about 10 picomolar or higher. It is within the present invention that the K_{D} values of individual nucleic acids binding to CCL2 is preferably within this range. Preferred ranges can be defined by choosing any first number within this range and any second number within this range. Preferred upper values are 250 nM and 100 nM, preferred lower values are 50 nM, 10 nM, 1 nM, 100 pM and 10 pM.

The nucleic acid molecules suitable for the practicing of the present invention may have any length provided that they are still able to bind to the target molecule. It will be acknowledged in the art that there are preferred lengths of the nucleic acid molecules suitable for the practicing of the present invention. Typically, the length is between 15 and 120 nucleotides. It will be acknowledged by the ones skilled in the art that any integer between 15 and 120 is a possible length for the nucleic acid molecules suitable for the practicing of the present invention. More preferred ranges for the length of such nucleic acid molecules are lengths of about 20 to 100 nucleotides, about 20 to 80 nucleotides, about 20 to 60 nucleotides, about 20 to 50 nucleotides and about 30 to 50 nucleotides.

In an embodiment of each and any aspect of the invention, including any embodiment thereof, nucleic acid molecules comprise a moiety which preferably is a high molecular weight moiety and/or which preferably allows to modify the characteristics of the nucleic acid molecule in terms of, among others, residence time in the animal body, preferably the human body. A particularly preferred embodiment of such modification is PEGylation and HESylation of the nucleic acid molecules suitable for the practicing of the invention. As used herein PEG stands for poly(ethylene glycole) and HES for hydroxyethly starch. PEGylation as preferably used herein is the modification of said nucleic acid molecules, whereby such modification consists of a PEG moiety which is attached to said nucleic acid molecule. HESylation as preferably used herein is the modification of said nucleic acid molecule, whereby such modification consists of a HES moiety which is attached to the nucleic acid molecule. These modifications as well as the process of modifying a nucleic acid using such modifications, is described in European patent application EP 1 306 382, the disclosure of which is herewith incorporated in its entirety by reference.

Preferably, the molecular weight of a modification consisting of or comprising a high molecular weight moiety is about from 2,000 to 200,000 Da, preferably 20,000 to 120,000 Da, particularly in case of PEG being such high molecular weight moiety, and is preferably about from 3,000 to 180,000 Da, more preferably from 5,000 to 130,000 Da, particularly in case of HES being such high molecular weight moiety. The process of HES modification is, e.g., described in German patent application DE 1 2004 006 249.8 the disclosure of which is herewith incorporated in its entirety by reference.

PEG and HES may be used as either a linear or branched from as further described in the patent applications WO2005074993 and PCT/EP02/11950. Such modification can, in principle, be made to the nucleic acid molecules at any position thereof. Preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the nucleic acid molecule suitable for the practicing of the present invention either directly or through a linker. It will be understood by a person skilled in the art that the nucleic acid molecule may comprise one or more modifications, preferably one or more PEG and/or HES moiety. In an embodiment, the individual linker molecule attaches more than one PEG moiety or HES moiety to the nucleic acid molecule. The linker used for attaching the modification to the nucleic acid molecule can itself be either linear or branched. This kind of linkers are known to the ones skilled in the art and are further described in the patent applications WO2005074993 and PCT/EP02/11950.

In an embodiment of each and any aspect, including any embodiment of each and any aspect, a compound which may be used as an inhibitor of CCL2 activity is selected from the group comprising a compound targeting CCL2, wherein the compound is selected from the group comprising Bindarit, a small molecule CCL2 inhibitor; Carlumab (CNTO888), a fully-human anti-CCL2 Mab; ABN-912, a fully-human anti-CCL2 Mab; CGEN-54 and a recombinant CCL2-inhibiting protein.

In an embodiment of each and any aspect, including any embodiment of each and any aspect, a compound which may be used as an inhibitor of CCL2 activity is selected from the group comprising a compound targeting CCR2, wherein the compound is selected from the group comprising AZ-889, a small molecule CCR2 antagonist; AZD-2423 a small molecule CCR2 antagonist, a small molecule CCR2 antagonist; BL-2030, a soluble CCR2 receptor fused to a human antibody Fc component; BMS-741672, a small molecule CCR2 antagonist; BMS-753426, a small molecule CCR2 antagonist; BMS-813160, a small molecule CCR2 antagonist; CCX-140, a small molecule CCR2 inhibitor; CCX-598, a small molecule CCR2 inhibitor; CCX-872, a small molecule CCR2 inhibitor; CCX-915, a small molecule CCR2 inhibitor; Cenicriviroc, a small molecule CCR2/CCR5 antagonist; CNTX-6970, a small molecule CCR2 antagonist; CPD-B, a small molecule CCR2 antagonist; EPX-102216, a small molecule CCR2 antagonist; INCB-3344, a small molecule CCR2 inhibitor; INCB-3284, a small molecule CCR2 inhibitor; INCB-8696, a small molecule CCR2 inhibitor; LF-0376, a small molecule CCR2/5 antagonist; MK-812, a small molecule CCR2 inhibitor; NIBR-6465, a small molecule CCR2/5 antagonist; OB-004 a small molecule CCR2 antagonist; OPL-CCL2-LPM, a human CCL2 chemokine fusion protein with cytotoxic payload; PD-172084, a small molecule CCR2 antagonist; PF-04634817, a small molecule CCR2/5 antagonist; PF-4136309, a small molecule CCR2 antagonist; Plozalizumab (MLN-1202), a humanized anti-CCR2 mAb; R-103, an oral Dalal-peptide T-amide analogue targeting CCR2, 5 and 8; RAP-103; RAP-310, both small molecule CCR2/5 antagonists; SB-380732, a small molecule CCR2 antagonist; SPR-3, a small molecule CCR2 antagonist; STI-B0201, a fully human anti-CCR2 mAb; STI-B0211, a fully human anti-CCR2 mAb; STI-B0221, a fully human anti-CCR2 mAb; STI-B0234, a fully human anti-CCR2 mAb; TAK-779, a small molecule CCR2/5 antagonist; and TLK-19705, small molecule CCR2 antagonist.

In an embodiment of each and any aspect, including any embodiment of each and any aspect, an inhibitor of CCL2 activity is characterized by slow elimination from the surgical site after subconjunctival administration to ensure durable inhibition of CCL2-mediated (i) attraction of monocytes/macrophages from peripheral blood to the surgical site and (ii) crosstalk between fibroblasts and macrophages, with both processes promoting fibrosis. At the same time, as the combination of local and systemic administration has been shown to be very efficient, the inhibitor of CCL2 activity also has pharmacokinetic properties in the blood plasma compartment that allow sustained pharmacological inhibition of CCL2, i.e. a plasma half-life of at least 24 h. Preferably, the agent should be stable in biological fluids and tissues.

In an embodiment of each and any aspect, including any embodiment of each and any aspect, an inhibitor of CCL2 activity is characterized by slow elimination of the CCL2 binding agent from the vitreous or subretinal compartment after intravitreal, suprachoroidal or subretinal administration would be advantageous to ensure durable inhibition of CCL2-mediated (i) attraction of monocytes/ macrophages from peripheral blood into retinal tissue or tissue that surrounds the retina and (ii) crosstalk between fibroblasts and macrophages, with both processes promoting fibrosis. The agent should be stable in biological fluids and tissues, and in particular in the vitreous.

It will be acknowledged by a person skilled in the art given the disclosure presented herein, possible administration schemes can be perceived by a person skilled in the art as a matter of routine. For example, in case of AMD or DR, the inhibitor of CCL2 activity is locally and in particular intravitreally administered to the subjectin intervals of 2, 4, 6, 8, 10, 12, 14 or 16 weeks. It will be acknowledged by a person skilled in the art that the inhibitor of CCL2 activity may be administered as long as such treatment is therapeutically necessary and tolerated by the subject. In the case of PVR, the inhibitor of CCL2 activity is locally and in particular intravitreally administered to the subject first on the day of ocular trauma or DR repair surgery and one or several times thereafter in intervals of 1, 2, 3 or 4 weeks. It will be acknowledged by a person skilled in the art that the inhibitor of CCL2 activity may be administered as long as such treatment is therapeutically necessary and tolerated by the subject. It will be equally acknowledged by a person skilled in the art that, in principle, any of the above schemes for local administration of the inhibitor of CCL2 activity may be combined with systemic administration of the inhibitor of CCL2 activity.

It will be acknowledged by a person skilled in the art given the disclosure presented herein, possible formulation schemes can be perceived by a person skilled in the art as a matter of routine. For example, if the inhibitor of CCL2 activity is compound NOX-E36, NOX-E36 is formulated at a concentration of 20 mg/ml formulation or less. In an embodiment, the concentration of NOX-E-36 is 13 mg/ml formulation. In a further embodiment, 100 µl of such formulation are locally, preferably intravitreally administered to the subject. In an embodiment, systemic, preferably subcutaneous administration of the inhibitor of CCL2 activity comprises administration of 40 mg NOX-E36 as a flat fixed dose. In connection with any concentration expressed as mg/ml, it is to be noted that in case the inhibitor of CCL2 activity is a compound comprising both a nucleic acid moiety which is formed by a sequence of nucleotides, and a non-nucleic acid moiety such as a PEG moiety, the milligram refer to the weight of the nucleic acid moiety of the inhibitor of CCL2 activity only.

It will be acknowledged by a person skilled in the art given the disclosure presented herein, possible administration schemes can be perceived by a person skilled in the art as a matter of routine. For example, the inhibitor of CCL2 activity is locally and in particular subconjunctivally administered to the subject on the day of glaucoma filtration surgery and then until week 8 after glaucoma filtration surgery. Within these 8 weeks, the inhibitor of CCL2 activity may be locally and in particular subconjunctivally administered to the subject in week 1, 2, 3, 4, 5, 6, 7 and 8; alternatively, the inhibitor of CCL2 activity may be locally and in particular subconjunctivally administered to the subject in week 1, 2, 4, 6 and 8. The inhibitor of CCL2 activity is systemically and in particular subcutaneously administered to the subject on the day of glaucoma filtration surgery and several times within the two weeks subsequent to glaucoma filtration surgery. Within these two weeks, the inhibitor of CCL2 activity may be administered in week 1 and in week 2. It will be equally acknowledged by a person skilled in the art that, in principle, any of the above schemes for local administration of the inhibitor of CCL2 activity may be combined with systemic administration of the inhibitor of CCL2 activity.

It will be acknowledged by a person skilled in the art given the disclosure presented herein, possible formulation schemes can be perceived by a person skilled in the art as a matter of routine. For example, if the inhibitor of CCL2 activity is compound NOX-E36, NOX-E36 is formulated at a concentration of 20 mg/ml formulation or less. In an embodiment, the concentration of NOX-E-36 is 13 mg/ml formulation. In a further embodiment, 200 µl of such formulation are locally, preferably subconjunctivally administered to the subject. In an embodiment, systemic, preferably subcutaneous administration of the inhibitor of CCL2 activity comprises administration of 40 mg NOX-E36 as a flat fixed dose. In connection with any concentration expressed as mg/ml, it is to be noted that in case the inhibitor of CCL2 activity is a compound comprising both a nucleic acid moiety which is formed by a sequence of nucleotides, and a non-nucleic acid moiety such as a PEG moiety, the milligram refer to the weight of the nucleic acid moiety of the inhibitor of CCL2 activity only.

In an embodiment of each and any aspect, including any embodiment thereof, the terms surgical procedure to reduce intraocular pressure and surgery for reducing intraocular pressure are used interchangeably and synonymously herein, unless indicated otherwise.

In an embodiment of each and any aspect, including any embodiment thereof, the terms surgical procedure to reduce intraocular pressure and surgery for reducing intraocular pressure are used interchangeably and synonymously herein, unless indicated otherwise.

In an embodiment of each and any aspect, including any embodiment thereof, the terms inhibitor of Monocyte Chemoattractant Protein-1 (CCL2) activity and inhibitor of CCL2 activity are used interchangeably and synonymously herein, unless indicated otherwise.

In an embodiment of each and any aspect, including any embodiment thereof, the terms CCL2, CCL-2 and MCP-1 are used interchangeably and synonymously herein, unless indicated otherwise.

Any reference to the invention or to the present invention refers in particular to each and any aspect of the invention, including any embodiment of such each and any aspect. Also, the terms aspect and aspect of the invention or aspect of the present invention are used interchangeably and synonymously herein, unless indicated otherwise.

In an embodiment of each and any aspect, including any embodiment thereof, the terms systemically administering and systemic administration have an interchangeable and synonymous meaning herein, unless indicated otherwise.

In an embodiment of each and any aspect, including any embodiment thereof, the terms topically administering and topical administration have an interchangeable and synonymous meaning herein, unless indicated otherwise. It will be appreciated by a person skilled in the art that a similar interchangeable and synonymous meaning exists for other routes of administration disclosed herein, whereby such administration is described by the noun and the characterizing adjective on the one hand and by the verb and the characterizing adverb on the other hand.

In an embodiment of each and any aspect, including any embodiment thereof, the term anti-fibrotic means that fibrosis is inhibited, reduced or avoided.

In an embodiment of each and any aspect, including any embodiment thereof, the term anti-fibrotic means that fibrosis is reduced.

In an embodiment of each and any aspect, including any embodiment thereof, the term anti-fibrotic means that fibrosis is avoided.

In an embodiment of each and any aspect, including any embodiment thereof, the term anti-fibrotic means that fibrosis is inhibited.

In an embodiment of each and any aspect, including any embodiment of such each and any aspect, an anti-angiogenic therapy such as a VEGF inhibitor is not applied if the subject is suffering from or is at risk of suffering from atrophic dry AMD.

In a preferred embodiment of each and any aspect, including any embodiment of such each and any aspect, an anti-angiogenic therapy such as a VEGF inhibitor is administered to the subject prior to, concomitantly with or subsequent to the administration of the inhibitor of CCL2 activity, wherein the subject is suffering from nAMD or PDR.

In an embodiment of each and any aspect, including any embodiment of such each and any aspect, an anti-angiogenic therapy such as a VEGF inhibitor is not applied if the subject is suffering from or is at risk of suffering from PVR.

In an embodiment of each and any aspect, including any embodiment of such each and any aspect, an ocular trauma may result from traumatic accidents, exposure to chemicals or foreign objects in an eye, preferably such ocular trauma results in fibrosis or could result in fibrosis.

If reference is made to an embodiment of the invention or an embodiment of the present invention, such embodiment constitutes an embodiment of each and any aspect of the invention, including any embodiment of each and any aspect.

If reference is made herein to "any of Embodiments X to Y", this means that backreference is made to each and any individual embodiment contained within the range defined by X and Y, including the embodiments numbered as X and Y, respectively. In accordance therewith, all of said embodiments are disclosed individually and back-referenced. For example, if back-reference is to any one of embodiments 1 to 4, such back-reference means any one of embodiment 1, embodiment 2, embodiment 3 and embodiment 4.

The various SEQ.ID. Nos., the chemical nature of the nucleic acid molecules according to the present invention and the target molecules MCP-1 as used herein, the actual sequence thereof and the internal reference number is summarized in the following table.

| **Seq.-ID** | ***RNA*/*Peptide*** | **Sequence** | ***Internal Reference*** |
|---|---|---|---|
| 1 | L-protein | QPDAINAPVTCCYNFTNRKISVQRLASYRRITSSKCPKEAVIFKTIVAKEICADPKQKWV QDSMDHLDKQTQTPKT | human MCP-1, huMCP-1, CCL2 |
| 2 | L-protein | QPDAVNAPLTCCYSFTSKMIPMSRLESYKRITSSRCPKEAVVFVTKLKREVCADPKKEWV QTYIKNLDRNQMRSEPTTLFKTASALRSSAPLNVKLTRKSEANASTTFSTTTSSTSVGVT SVTVN | mouse MCP-1, mCCL2, mMCP-1, murine MCP-1 (Mus musculus) |
| 3 | L-protein | QPDAINAPVTCCYNFTNRKISVQRLASYRRITSSKCPKEAVIFKTIVAKEICADPKQKWV QDSMDHLDKQIQTPKP | monkey MCP-1 (Macaca mulatta) |
| 4 | L-protein | QPDAINSPVTCCYTLTSKKISMQRLMSYRRVTSSKCPKEAVIFKTIAGKEICAEPKQKWV QDSISHLDKKNQTPKP | pig MCP-1 (Sus scrofa) |
| 5 | L-protein | QPDAIISPVTCCYTLTNKKISIQRLASYKRVTSSKCPKEAVIFKTVLNKEICADPKQKWV QDSMAHLDKKSQTQTA | dog MCP-1 (Canis familiaris) |
| 6 | L-protein | QPDAVNSPVTCCYTFTNKTISVKRLMSYRRINSTKCPKEAVIFMTKLAKGICADPKQKWV QDAIANLDKKMQTPKTLTSYSTTQEHTTNLSSTRTPSTTTSL | rabbit MCP-1 (Oryctolagus cuniculus) |
| 7 | L-protein | QPVGINTSTTCCYRFINKKIPKQRLESYRRTTSSHCPREAVIFKTKLDKEICADPTQKWV QDFMKHLDKKTQTPKL | human MCP-3, CCL7, huMCP-3 |
| 8 | L-protein | GPASVPTTCCFNLANRKIPLQRLESYRRITSGKCPQKAVIFKTKLAKDICADPKKKWVQD SMKYLDQKSPTPKP | human eotaxin/CCL11 |
| 9 | L-protein | QPDSVSIPITCCFNVINRKIPIQRLESYTRITNIQCPKEAVIFKTKRGKEVCADPKERWV RDSMKHLDQIFQNLKP | human MCP-2, CCL8, huMCP-2 |
| 10 | L-RNA | AGCGUGCCCGGAGUGGCAGGGGGACGCGACCUGCAAUAAUGCACGCU | 169-B1trc |
| 11 | L-RNA | AGCGUGCCCGGAGUGGCAGGGGGACGCGACCUGCAAUUGCACGCU | 169-F3trc |
| 12 | L-RNA | AGCGUGCCCGGAGUGGCAGGGGGACGCGACCUGUAAUAAUGCACGCU | 169-C1trc |
| 13 | L-RNA | AGCGUGCCCGGUGUGGCAGGGGGACGCGACCUGCAAUAAUGCGCGCU | 169-A3trc |
| 14 | L-RNA | AGCGUGCCCGGAGUAGCAGGGGGGCGCGACCUGCAAUAAUGCACGCU | 169-B2trc |
| 15 | L-RNA | AGCGUGCCCGGUGUGGUAGGGGGGCGCGAUCUACAAUUGCACGCU | 176-B12trc |
| 16 | L-RNA | AGCGUGCCCGGUGUGACAGGGGGGCGCGACCUGCAUUUGCACGCU | 176-D9trc |
| 17 | L-RNA | AGCGUGCCCGGUGUGGCAGGGGGGCGCGACCUGUAUUUGCACGCU | 176-B10trc |
| 18 | L-RNA | AGCGUGCCCGGAGUGGCAGGGGGGCGCGACCUGCAAUAAUGCACGCU | 169-F2trc |
| 19 | L-RNA | AGCGUGCCCGGUGUGGCAGGGGGGCGCGACCUGCAAUUGCACGCU | 176-B9trc |
| 20 | L-RNA | AGCAUGCCCGGUGUGGCAGGGGGGCGCGACCUGCAUUUGCAUGCU | 176-H9trc |
| 21 | L-RNA | AGCGUGCCCGGUGUGGUAGGGGGGCGCGACCUACAUUUGCACGCU | 176-E10trc |
| 22 | L-RNA | AGUGUGCCAGCUGUGAUGGGGGGGCGCGACCCAUUUUACACACU | 176-G9trc |
| 23 | L-RNA | AGUGUGCCAGCGUGAUGGGGGGGCGCGACCCAUUUUACACACU | 176-F9trc |
| 24 | L-RNA | AGUGUGCGAGCGUGAUGGGGGGGCGCGACCCAUUUUACAUACU | 176-C11trc |
| 25 | L-RNA | AGUGUGCCAGCGUGAUGGGGGGGCGCGACCCAUUUUACAUACU | 176-E11trc |
| 26 | L-RNA | AGUAUGCCAGCGUGAUGGGGGGGCGCGACCCAUUUACAUACU | 176-D10trc |
| 27 | L-RNA | AGUGUGCCAGUGUGAUGGGGGGGCGCGACCCAUUUUACACACU | 176-H10trc |
| 28 | L-RNA | AGCGUGCCAGUGUGAUGGGGGGGCGCGACCCAUUUUACACGCU | 176-C9trc |
| 29 | L-RNA | ACGCACGUCCCUCACCGGUGCAAGUGAAGCCGCGGCUCUGCGU | 180-B1-001 |
| 30 | L-RNA | ACGCACCUCCCUCACCGGUGCAAGUGAAGCCGUGGCUCUGCGC | 180-A4-002 |
| 31 | L-RNA | ACGCACGUCCCUCACCGGUGCAAGUGAAGCCGUGGCUCUGCGU | 180-D1-002 |
| 32 | L-RNA | GCACGUCCCUCACCGGUGCAAGUGAAGCCGUGGCUCUGCGU | 180-D1-011 |
| 33 | L-RNA | ACGCACGUCCCUCACCGGUGCAAGUGAAGCCGUGGCUCUGC | 180-D1-012 |
| 34 | L-RNA | GCACGUCCCUCACCGGUGCAAGUGAAGCCGUGGCUCUGC | 180-D1-018 |
| 35 | L-RNA | CGCACGUCCCUCACCGGUGCAAGUGAAGCCGUGGCUCUGCGU | 180-D1-034 |
| 36 | L-RNA | CGCACGUCCCUCACCGGUGCAAGUGAAGCCGUGGCUCUGCG | 180-D1-035 |
| 37 | L-RNA | GCACGUCCCUCACCGGUGCAAGUGAAGCCGUGGCUCUGCG | 180-D1-036 = NOX-E36 |
| 38 | L-RNA | GUGCUGCGUAGUGGAAGACUACCUAAUGACAGCCGAAUGCUGGCAGCAC | 178-A8 |
| 39 | L-RNA | GUGCUGCGUAGUGGAAGACUACCUAAUGACAGCCUAAUGCUGGCAGCAC | 178-F7 |
| 40 | L-RNA | GUGCUGCGUAGUGGAAGACUACCUUAUGACAGCCGAAUGCUGGCAGCAC | 178-G7 |
| 41 | L-RNA | GUGCUGCGUAGUGAAAAACUACUGCCAGUGGGUCAGAGCUAGCAGCAC | 178-C6 |
| 42 | L-RNA | GUGCUGCGGAGUUAAAAACUCCCUAAGACAGGCCAGAGCCGGCAGCAC | 178-E7 |
| 43 | L-RNA | GUGCUGCGGAGUUGAAAACUCCCUAAGACAGGCCAGAGCCGGCAGCAC | 178-G6 |
| 44 | L-RNA | GUGCUGCGUAGUGGAAGACUACCUAUGACAGCCUAAUGCUGGCAGCAC | 178-A7 |
| 45 | L-RNA | GUGCUGCGGAGUUAAAAACUCCCUAAGACAGGCUAGAGCCGGCAGCAC | 178-C7 |
| 46 | L-RNA | GUGCUGCGGCGUGAAAAACGCCCUGCGACUGCCCUUUAUGCAGGCAGCAC | 178-E5 |
| 47 | L-RNA | GUGCUGCGUAGUGAAAAACUACCAACGACUGGCUAGAGCCGGCAGCAC | 181-F1 |
| 48 | L-RNA | GUGCUGCGUAGUGAAAGACUACCUGUGACAGCCGAAUGCUGGCAGCAC | 181-B2 |
| 49 | L-RNA | GUACUGCGUAGUUAAAAACUACCAACGACUGGCUAGAGCCGGCAGCAC | 181-C2 |
| 50 | L-RNA | GUGCUGCGUAGUUAAAAACUACCAACGACUGGCUAGAGCCGGCAGCAC | 178-A6 |
| 51 | L-RNA | GUGCUGCGUAGUUAAAAACUACCAGCGACAGGCUAGAGCCGGCAGCAC | 178-D6 |
| 52 | L-RNA | GUGCUGCGUAGUUAAAAACUACCAGCGACUGGCUAGAGCCGGCAGCAC | 178-D5 |
| 53 | L-RNA | GUGCUGCGUAGUGAGAAACUACCAACGACUGGCUAGAGCCGGCAGCAC | 181-A2 |
| 54 | L-RNA | GGCUGCGUAGUUAAAAACUACCAGCGACUGGCUAGAGCCGGCAGCC | 178-D5-020 |
| 55 | L-RNA | GGCGCGUAGUUAAAAACUACCAGCGACUGGCUAGAGCCGGCGCC | 178-D5-027 |
| 56 | L-RNA | GUGCGCGUAGUUAAAAACUACCAGCGACUGGCUAGAGCCGGCGCAC | 178-D5-030 |
| 57 | L-RNA | GUGCGCGUAGUGAGAAACUACCAACGACUGGCUAGAGCCGGCGCAC | 181-A2-002 |
| 58 | L-RNA | GUGCCGUAGUGAGAAACUACCAACGACUGGCUAGAGCCGGGCAC | 181-A2-004 |
| 59 | L-RNA | GUGGCGUAGUGAGAAACUACCAACGACUGGCUAGAGCCGGCCAC | 181-A2-005 |
| 60 | L-RNA | GUCGCGUAGUGAGAAACUACCAACGACUGGCUAGAGCCGGCGAC | 181-A2-006 |
| 61 | L-RNA | UGCGCGUAGUGAGAAACUACCAACGACUGGCUAGAGCCGGCGCA | 181-A2-007 |
| 62 | L-RNA | GCUGCGUAGUGAGAAACUACCAACGACUGGCUAGAGCCGGCAGC | 181-A2-008 |
| 63 | L-RNA | GCUGCGUAGUGAGAAACUACCAACGACUGGCUAGAGCCGGCAGC | 181-A2-011 |
| 64 | L-RNA | GGUGCGUAGUGAGAAACUACCAACGACUGGCUAGAGCCGGCACC | 181-A2-012 |
| 65 | L-RNA | UGGCGUAGUGAGAAACUACCAACGACUGGCUAGAGCCGGC-CA | 181-A2-015 |
| 66 | L-RNA | GCGCGUAGUGAGAAACUACCAACGACUGGCUAGAGCCGGCGC | 181-A2-016 |
| 67 | L-RNA | GUGCGUAGUGAGAAACUACCAACGACUGGCUAGAGCCGGCAC | 181-A2-017 |
| 68 | L-RNA | GG-GCGUAGUGAGAAACUACCAACGACUGGCUAGAGCCGGCCC | 181-A2-018 |
| 69 | L-RNA | GAGCGUAGUGAGAAACUACCAACGACUGGCUAGAGCCGGCUC | 181-A2-019 |
| 70 | L-RNA | CGGCGUAGUGAGAAACUACCAACGACUGGCUAGAGCCGGCCG | 181-A2-020 |
| 71 | L-RNA | CCGCGUAGUGAGAAACUACCAACGACUGGCUAGAGCCGGCGG | 181-A2-021 |
| 72 | L-RNA | CAGCGUAGUGAGAAACUACCAACGACUGGCUAGAGCCGGCUG | 181-A2-022 |
| 73 | L-RNA | CUGCGUAGUGAGAAACUACCAACGACUGGCUAGAGCCGGCAG | 181-A2-023 |
| 74 | L-RNA | AGCGUGUUAGUGAAGUGGGUGGCAGGUAAAGGACACGCU | 184-B8trc |
| 75 | L-RNA | AGCGUGGUAGCGGUGUGGGUGGUAGGUAAAGGCCACGCU | 184-C6trc |
| 76 | L-RNA | AGCGUGAUAGAAGAGCGGGUGGUAGGUAAAGGUCAGGCU | 184-H5trc |
| 77 | L-RNA | AGCGUGUUAGGUAGGGUGGUAGUAAGUAAAGGACACGCU | 184-A7trc |
| 78 | L-RNA | AGCGUGUUAGGUGGGUGGUAGUAAGUAAAGGACACGCU | 187-A5trc |
| 79 | L-RNA | AGCGUGUUAGGUGGGUGGUAGUAAGUAAAGGGCACGCU | 187-H5trc |
| 80 | L-RNA | CCGCUUAGGUGGGUGGUAGUAAGUAAAGGGGCGG | 174-D4-004 |
| 81 | L-RNA | GCGCGAGCAGGUGGGUGGUAGAAUGUAAAGACUCGCGUC | 166-A4-002 |
| 82 | L-RNA | CGUGUUAGGUGGGUGGUAGUAAGUAAAGGACACG | 187-A5trc-001 |
| 83 | L-RNA | GUGUUAGGUGGGUGGUAGUAAGUAAAGGACAC | 187-A5trc-002 |
| 84 | L-RNA | CGUGUUAGGUGGGUGGUAGUAAGUAAAGGGCACG | 187-H5trc-002 |
| 85 | L-RNA | GUGUUAGGUGGGUGGUAGUAAGUAAAGGGCAC | 187-H5trc-003 |
| 86 | L-RNA | UGUUAGGUGGGUGGUAGUAAGUAAAGGGCA | 187-H5trc-004 |
| 87 | L-RNA | GGACGAGAGUGACAAAUGAUAUAACCUCCUGACUAACGCUGCGGGCGACAGG | 177-B3 |
| 88 | L-RNA | GGACCUAUCGCUAAGACAACGCGCAGUCUACGGGACAUUCUCCGCGGACAGG | 177-C1 |
| 89 | L-RNA | GGACAAUUGUUACCCCCGAGAGAGACAAAUGAGACAACCUCCUGAAGACAGG | 177-C2 |
| 90 | L-RNA | GGACGAAAGUGAGAAAUGAUACAACCUCCUGUUGCUGCGAAUCCGGACAGG | 177-E3 |
| 91 | L-RNA | GGACGUAAAAGACGCUACCCGAAAGAAUGUCAGGAGGGUAGACCGACAGG | 177-D1 |
| 92 | L-RNA | GGACUAGAAACUACAAUAGCGGCCAGUUGCACCGCGUUAUCAACGACAGG | 177-E1 |
| 93 | L-RNA | GGACUAGUCAGCCAGUGUGUAUAUCGGACGCGGGUUUAUUUACUGACAGG | 177-A1 |
| 94 | L-RNA | GGACUGUCCGGAGUGUGAAACUCCCCGAGACCGCCAGAAGCGGGGACAGG | 177-G3 |
| 95 | L-RNA | GGACUUCUAUCCAGGUGGGUGGUAGUAUGUAAAGAGAUAGAAGUGACAGG | 177-C3 |
| 96 | L-RNA | GGACGAGAGCGAACAAUGAUAUAACCUCCUGACGGAAAGAGAUCGACAGG | 177-A2 |
| 97 | L-RNA | CCUGUGCUACACGCAGUAAGAAGUGAACGUUCAGUAUGUGUGCACAGG | 170-E4trc |
| 98 | L-RNA | CGUGAGCCAGGCACCGAGGGCGUUAACUGGCUGAUUGGACACGACACG | 166-D2trc |
| 99 | L-RNA | CGUGAACAUGCAAGCUAAGCGGGGCUGUUGGUUGCUUGGCCCGCCACG | 174-A2trc |
| 100 | L-RNA | CGUGCAGAGAGAGACCAACCACGUAAAAUCAACCUAAUGGGCCGCACG | 174-E2trc |
| 101 | L-RNA | CGUGCAGAGAGAGACCAACCACGUAAAAUCAACCUAAUGGGCCGCACG | 183-G3trc |
| 102 | L-RNA | CGUGAACAUUCAAGCUAAGCGGGGCUGUUGGUUGCUUGGCCCGCCACG | 183-B2trc |
| 103 | L-RNA | CGUGCCGAGGCGGCGACCAGCGUUACUUAGAGAGGCUUUGGCACCACG | 166-B2trc |
| 104 | L-RNA | CGUGAUAACAGCCGUCGGUCAAGAAAACAAAGUUCGGGCGGCGCACG | 166-G3trc |
| 105 | L-RNA | CGUGGGUGGCGCACCGAGGGCGAAAAGCCACCAGUAAAGAUAGACCG | 166-D1trc |
| 106 | L-RNA | CGUGUGAUCUCCUUUGGGGUGAUUAGCUUAGAGACUUCCCACACG | 183-H2trc |
| 107 | L-RNA | GCACCUUCGCCUAAUACACGUGCCGGCUAGCUAAUACUCGUCCGC | 167-A7trc |
| 108 | L-RNA | GCACGACUUGGGCGACCAGUGAUACUUAGAGAGCAAGUCGUCGGC | 167-C7trc |
| 109 | L-RNA | GCGCGCGCUCAGUAAGAAAUUGAAAGUUCAGAAUGUCGUCGCGC | 167-B5trc |
| 110 | L-RNA | AGUGUGUGGCAGGCUAAGGAGAUAUUCCGAGACCACGCU | 184-D7trc |
| 111 | L-RNA | AGUGUGUGGCAGACUAUGGAUAGACUCCGAGACCACGCU | 184-D6trc |
| 112 | L-RNA | AGCGUGAGGCGACCAGCGGAUUACUUAGAGAGUCACGCU | 184-E5trc |
| 113 | L-RNA | AGCGUGAAGGGGACCAGCGUUACUUACAGAGUUCACGCU | 184-G6trc |
| 114 | L-RNA | AGCGUGUGAUGUAUGUAGCACCGUAUCAGAGGACACGCU | 184-B7trc |
| 115 | L-RNA | AGCGUGAGGCGACCCGUGUUUCGUAGAGAGUCACGCU | 184-B6trc |
| 116 | L-RNA | 5' PEG-GCACGUCCCUCACCGGUGCAAGUGAAGCCGUGGCUCUGCG | NOX-E36-5' PEG |
| 117 | L-RNA | GCACGUCCCUCACCGGUGCAAGUGAAGCCGUGGCUCUGCG-3'PEG | NOX-E36-3' PEG |
| 118 | L-RNA | GAGAUGGCGACAUUGGUUGGGCAUGAGGCGAGGCCCUUUGAUGAAUCCGCGGCCAUUC | 188-A3-001 |
| 119 | L-RNA | GAUGGCGACAUUGGUUGGGCAUGAGGCGAGGCCCUUUGAUGAAUCCGCGGCCAUUC | 188-A3-004 |
| 120 | L-RNA | GGCGACAUUGGUUGGGCAUGAGGCGAGGCCCUUUGAUGAAUCCGCGGCCAUUC | 188-A3-005 |
| 121 | L-RNA | GGCGACAUUGGUUGGGCAUGAGGCGAGGCCCUUUGAUGAAUCCGCGGCCAUU | 188-A3-006 |
| 122 | L-RNA | GGCGACAUUGGUUGGGCAUGAGGCGAGGCCCUUUGAUGAAUCCGCGGCCA | 188-A3-007 = mNOX-E36 |
| 123 | L-RNA | GCUGGUUACCGAGGGGGCGUCGUUGGAGUUUGGUUGGUUGUCACCAGC | 189-G7-001 |
| 124 | L-RNA | CUGGUUACCGAGGGGGCGUCGUUGGAGUUUGGUUGGUUGUCACCAG | 189-G7-002 |
| 125 | L-RNA | UGGUUACCGAGGGGGCGUCGUUGGAGUUUGGUUGGUUGUCACCA | 189-G7-003 |
| 126 | L-RNA | GCCGGUUACCGAGGGGGCGUCGUUGGAGUUUGGUUGGUUGUCACCGGC | 189-G7-007 |
| 127 | L-RNA | GCCGGCUACCGAGGGGGCGUCGUUGGAGUUUGGUUGGUUGUCGCCGGC | 189-G7-008 |
| 128 | L-RNA | GCGCGUACCGAGGGGGCGUCGUUGGAGUUUGGUUGGUUGUCCGCGC | 189-G7-010 |
| 129 | L-RNA | GGGCCUACCGAGGGGGCGUCGUUGGAGUUUGGUUGGUUGUCGGCCC | 189-G7-012 |
| 130 | D-protein | Biotin- QPDAINAPVTCCYNFTNRKISVQRLASYRRITSSKCPKEAVIFKTIVAKEICADPKQKWV QDSMDHLDKQTQTPKT | biotinylated human D-MCP-1 |
| 131 | D-protein | Biotin- QPDAVNAPLTCCYSFTSKMIPMSRLESYKRITSSRCPKEAVVFVTKLKREVCADPKKEWV QTYIKNLDRNQMRSEP-Biotin | biotinylated mouse D-MCP-1 |
| 132 | D-RNA | AGCGUGCCCGGAGUGGCAGGGGGACGCGACCUGCAAUAAUGCACGCU | 169-B1trc |
| 133 | D-RNA | AGCGUGCCCGGAGUGGCAGGGGGACGCGACCUGCAAUUGCACGCU | 169-F3trc |
| 134 | D-RNA | AGCGUGCCCGGAGUGGCAGGGGGACGCGACCUGUAAUAAUGCACGCU | 169-C1trc |
| 135 | D-RNA | AGCGUGCCCGGUGUGGCAGGGGGACGCGACCUGCAAUAAUGCGCGCU | 169-A3trc |
| 136 | D-RNA | AGCGUGCCCGGAGUAGCAGGGGGGCGCGACCUGCAAUAAUGCACGCU | 169-B2trc |
| 137 | D-RNA | AGCGUGCCCGGUGUGGUAGGGGGGCGCGAUCUACAAUUGCACGCU | 176-B12trc |
| 138 | D-RNA | AGCGUGCCCGGUGUGACAGGGGGGCGCGACCUGCAUUUGCACGCU | 176-D9trc |
| 139 | D-RNA | AGCGUGCCCGGUGUGGCAGGGGGGCGCGACCUGUAUUUGCACGCU | 176-B10trc |
| 140 | D-RNA | AGCGUGCCCGGAGUGGCAGGGGGGCGCGACCUGCAAUAAUGCACGCU | 169-F2trc |
| 141 | D-RNA | AGCGUGCCCGGUGUGGCAGGGGGGCGCGACCUGCAAUUGCACGCU | 176-B9trc |
| 142 | D-RNA | AGCAUGCCCGGUGUGGCAGGGGGGCGCGACCUGCAUUUGCAUGCU | 176-H9trc |
| 143 | D-RNA | AGCGUGCCCGGUGUGGUAGGGGGGCGCGACCUACAUUUGCACGCU | 176-E10trc |
| 144 | D-RNA | AGUGUGCCAGCUGUGAUGGGGGGGCGCGACCCAUUUUACACACU | 176-G9trc |
| 145 | D-RNA | AGUGUGCCAGCGUGAUGGGGGGGCGCGACCCAUUUUACACACU | 176-F9trc |
| 146 | D-RNA | AGUGUGCGAGCGUGAUGGGGGGGCGCGACCCAUUUUACAUACU | 176-C11trc |
| 147 | D-RNA | AGUGUGCCAGCGUGAUGGGGGGGCGCGACCCAUUUUACAUACU | 176-E11trc |
| 148 | D-RNA | AGUAUGCCAGCGUGAUGGGGGGGCGCGACCCAUUUACAUACU | 176-D10trc |
| 149 | D-RNA | AGUGUGCCAGUGUGAUGGGGGGGCGCGACCCAUUUUACACACU | 176-H10trc |
| 150 | D-RNA | AGCGUGCCAGUGUGAUGGGGGGGCGCGACCCAUUUUACACGCU | 176-C9trc |
| 151 | D-RNA | ACGCACGUCCCUCACCGGUGCAAGUGAAGCCGCGGCUCUGCGU | 180-B1-001 |
| 152 | D-RNA | ACGCACCUCCCUCACCGGUGCAAGUGAAGCCGUGGCUCUGCGC | 180-A4-002 |
| 153 | D-RNA | ACGCACGUCCCUCACCGGUGCAAGUGAAGCCGUGGCUCUGCGU | 180-D1-002 |
| 154 | D-RNA | GCACGUCCCUCACCGGUGCAAGUGAAGCCGUGGCUCUGCGU | 180-D1-011 |
| 155 | D-RNA | ACGCACGUCCCUCACCGGUGCAAGUGAAGCCGUGGCUCUGC | 180-D1-012 |
| 156 | D-RNA | GCACGUCCCUCACCGGUGCAAGUGAAGCCGUGGCUCUGC | 180-D1-018 |
| 157 | D-RNA | CGCACGUCCCUCACCGGUGCAAGUGAAGCCGUGGCUCUGCGU | 180-D1-034 |
| 158 | D-RNA | CGCACGUCCCUCACCGGUGCAAGUGAAGCCGUGGCUCUGCG | 180-D1-035 |
| 159 | D-RNA | GCACGUCCCUCACCGGUGCAAGUGAAGCCGUGGCUCUGCG | (D-)180-D1-036, (D-)NOX-E36 |
| 160 | D-RNA | GUGCUGCGUAGUGGAAGACUACCUAAUGACAGCCGAAUGCUGGCAGCAC | 178-A8 |
| 161 | D-RNA | GUGCUGCGUAGUGGAAGACUACCUAAUGACAGCCUAAUGCUGGCAGCAC | 178-F7 |
| 162 | D-RNA | GUGCUGCGUAGUGGAAGACUACCUUAUGACAGCCGAAUGCUGGCAGCAC | 178-G7 |
| 163 | D-RNA | GUGCUGCGUAGUGAAAAACUACUGCCAGUGGGUCAGAGCUAGCAGCAC | 178-C6 |
| 164 | D-RNA | GUGCUGCGGAGUUAAAAACUCCCUAAGACAGGCCAGAGCCGGCAGCAC | 178-E7 |
| 165 | D-RNA | GUGCUGCGGAGUUGAAAACUCCCUAAGACAGGCCAGAGCCGGCAGCAC | 178-G6 |
| 166 | D-RNA | GUGCUGCGUAGUGGAAGACUACCUAUGACAGCCUAAUGCUGGCAGCAC | 178-A7 |
| 167 | D-RNA | GUGCUGCGGAGUUAAAAACUCCCUAAGACAGGCUAGAGCCGGCAGCAC | 178-C7 |
| 168 | D-RNA | GUGCUGCGGCGUGAAAAACGCCCUGCGACUGCCCUUUAUGCAGGCAGCAC | 178-E5 |
| 169 | D-RNA | GUGCUGCGUAGUGAAAAACUACCAACGACUGGCUAGAGCCGGCAGCAC | 181-F1 |
| 170 | D-RNA | GUGCUGCGUAGUGAAAGACUACCUGUGACAGCCGAAUGCUGGCAGCAC | 181-B2 |
| 171 | D-RNA | GUACUGCGUAGUUAAAAACUACCAACGACUGGCUAGAGCCGGCAGCAC | 181-C2 |
| 172 | D-RNA | GUGCUGCGUAGUUAAAAACUACCAACGACUGGCUAGAGCCGGCAGCAC | 178-A6 |
| 173 | D-RNA | GUGCUGCGUAGUUAAAAACUACCAGCGACAGGCUAGAGCCGGCAGCAC | 178-D6 |
| 174 | D-RNA | GUGCUGCGUAGUUAAAAACUACCAGCGACUGGCUAGAGCCGGCAGCAC | 178-D5 |
| 175 | D-RNA | GUGCUGCGUAGUGAGAAACUACCAACGACUGGCUAGAGCCGGCAGCAC | 181-A2 |
| 176 | D-RNA | GGCUGCGUAGUUAAAAACUACCAGCGACUGGCUAGAGCCGGCAGCC | 178-D5-020 |
| 177 | D-RNA | GGCGCGUAGUUAAAAACUACCAGCGACUGGCUAGAGCCGGCGCC | 178-D5-027 |
| 178 | D-RNA | GUGCGCGUAGUUAAAAACUACCAGCGACUGGCUAGAGCCGGCGCAC | 178-D5-030 |
| 179 | D-RNA | GUGCGCGUAGUGAGAAACUACCAACGACUGGCUAGAGCCGGCGCAC | 181-A2-002 |
| 180 | D-RNA | GUGCCGUAGUGAGAAACUACCAACGACUGGCUAGAGCCGGGCAC | 181-A2-004 |
| 181 | D-RNA | GUGGCGUAGUGAGAAACUACCAACGACUGGCUAGAGCCGGCCAC | 181-A2-005 |
| 182 | D-RNA | GUCGCGUAGUGAGAAACUACCAACGACUGGCUAGAGCCGGCGAC | 181-A2-006 |
| 183 | D-RNA | UGCGCGUAGUGAGAAACUACCAACGACUGGCUAGAGCCGGCGCA | 181-A2-007 |
| 184 | D-RNA | GCUGCGUAGUGAGAAACUACCAACGACUGGCUAGAGCCGGCAGC | 181-A2-008 |
| 185 | D-RNA | GCUGCGUAGUGAGAAACUACCAACGACUGGCUAGAGCCGGCAGC | 181-A2-011 |
| 186 | D-RNA | GGUGCGUAGUGAGAAACUACCAACGACUGGCUAGAGCCGGCACC | 181-A2-012 |
| 187 | D-RNA | UGGCGUAGUGAGAAACUACCAACGACUGGCUAGAGCCGGC-CA | 181-A2-015 |
| 188 | D-RNA | GCGCGUAGUGAGAAACUACCAACGACUGGCUAGAGCCGGCGC | 181-A2-016 |
| 189 | D-RNA | GUGCGUAGUGAGAAACUACCAACGACUGGCUAGAGCCGGCAC | 181-A2-017 |
| 190 | D-RNA | GG-GCGUAGUGAGAAACUACCAACGACUGGCUAGAGCCGGCCC | 181-A2-018 |
| 191 | D-RNA | GAGCGUAGUGAGAAACUACCAACGACUGGCUAGAGCCGGCUC | 181-A2-019 |
| 192 | D-RNA | CGGCGUAGUGAGAAACUACCAACGACUGGCUAGAGCCGGCCG | 181-A2-020 |
| 193 | D-RNA | CCGCGUAGUGAGAAACUACCAACGACUGGCUAGAGCCGGCGG | 181-A2-021 |
| 194 | D-RNA | CAGCGUAGUGAGAAACUACCAACGACUGGCUAGAGCCGGCUG | 181-A2-022 |
| 195 | D-RNA | CUGCGUAGUGAGAAACUACCAACGACUGGCUAGAGCCGGCAG | 181-A2-023 |
| 196 | D-RNA | AGCGUGUUAGUGAAGUGGGUGGCAGGUAAAGGACACGCU | 184-B8trc |
| 197 | D-RNA | AGCGUGGUAGCGGUGUGGGUGGUAGGUAAAGGCCACGCU | 184-C6trc |
| 198 | D-RNA | AGCGUGAUAGAAGAGCGGGUGGUAGGUAAAGGUCAGGCU | 184-H5trc |
| 199 | D-RNA | AGCGUGUUAGGUAGGGUGGUAGUAAGUAAAGGACACGCU | 184-A7trc |
| 200 | D-RNA | AGCGUGUUAGGUGGGUGGUAGUAAGUAAAGGACACGCU | 187-A5trc |
| 201 | D-RNA | AGCGUGUUAGGUGGGUGGUAGUAAGUAAAGGGCACGCU | 187-H5trc |
| 202 | D-RNA | CCGCUUAGGUGGGUGGUAGUAAGUAAAGGGGCGG | 174-D4-004 |
| 203 | D-RNA | GCGCGAGCAGGUGGGUGGUAGAAUGUAAAGACUCGCGUC | 166-A4-002 |
| 204 | D-RNA | CGUGUUAGGUGGGUGGUAGUAAGUAAAGGACACG | 187-A5trc-001 |
| 205 | D-RNA | GUGUUAGGUGGGUGGUAGUAAGUAAAGGACAC | 187-A5trc-002 |
| 206 | D-RNA | CGUGUUAGGUGGGUGGUAGUAAGUAAAGGGCACG | 187-H5trc-002 |
| 207 | D-RNA | GUGUUAGGUGGGUGGUAGUAAGUAAAGGGCAC | 187-H5trc-003 |
| 208 | D-RNA | UGUUAGGUGGGUGGUAGUAAGUAAAGGGCA | 187-H5trc-004 |
| 209 | D-RNA | GGACGAGAGUGACAAAUGAUAUAACCUCCUGACUAACGCUGCGGGCGACAGG | 177-B3 |
| 210 | D-RNA | GGACCUAUCGCUAAGACAACGCGCAGUCUACGGGACAUUCUCCGCGGACAGG | 177-C1 |
| 211 | D-RNA | GGACAAUUGUUACCCCCGAGAGAGACAAAUGAGACAACCUCCUGAAGACAGG | 177-C2 |
| 212 | D-RNA | GGACGAAAGUGAGAAAUGAUACAACCUCCUGUUGCUGCGAAUCCGGACAGG | 177-E3 |
| 213 | D-RNA | GGACGUAAAAGACGCUACCCGAAAGAAUGUCAGGAGGGUAGACCGACAGG | 177-D1 |
| 214 | D-RNA | GGACUAGAAACUACAAUAGCGGCCAGUUGCACCGCGUUAUCAACGACAGG | 177-E1 |
| 215 | D-RNA | GGACUAGUCAGCCAGUGUGUAUAUCGGACGCGGGUUUAUUUACUGACAGG | 177-A1 |
| 216 | D-RNA | GGACUGUCCGGAGUGUGAAACUCCCCGAGACCGCCAGAAGCGGGGACAGG | 177-G3 |
| 217 | D-RNA | GGACUUCUAUCCAGGUGGGUGGUAGUAUGUAAAGAGAUAGAAGUGACAGG | 177-C3 |
| 218 | D-RNA | GGACGAGAGCGAACAAUGAUAUAACCUCCUGACGGAAAGAGAUCGACAGG | 177-A2 |
| 219 | D-RNA | CCUGUGCUACACGCAGUAAGAAGUGAACGUUCAGUAUGUGUGCACAGG | 170-E4trc |
| 220 | D-RNA | CGUGAGCCAGGCACCGAGGGCGUUAACUGGCUGAUUGGACACGACACG | 166-D2trc |
| 221 | D-RNA | CGUGAACAUGCAAGCUAAGCGGGGCUGUUGGUUGCUUGGCCCGCCACG | 174-A2trc |
| 222 | D-RNA | CGUGCAGAGAGAGACCAACCACGUAAAAUCAACCUAAUGGGCCGCACG | 174-E2trc |
| 223 | D-RNA | CGUGCAGAGAGAGACCAACCACGUAAAAUCAACCUAAUGGGCCGCACG | 183-G3trc |
| 224 | D-RNA | CGUGAACAUUCAAGCUAAGCGGGGCUGUUGGUUGCUUGGCCCGCCACG | 183-B2trc |
| 225 | D-RNA | CGUGCCGAGGCGGCGACCAGCGUUACUUAGAGAGGCUUUGGCACCACG | 166-B2trc |
| 226 | D-RNA | CGUGAUAACAGCCGUCGGUCAAGAAAACAAAGUUCGGGCGGCGCACG | 166-G3trc |
| 227 | D-RNA | CGUGGGUGGCGCACCGAGGGCGAAAAGCCACCAGUAAAGAUAGACCG | 166-D1trc |
| 228 | D-RNA | CGUGUGAUCUCCUUUGGGGUGAUUAGCUUAGAGACUUCCCACACG | 183-H2trc |
| 229 | D-RNA | GCACCUUCGCCUAAUACACGUGCCGGCUAGCUAAUACUCGUCCGC | 167-A7trc |
| 230 | D-RNA | GCACGACUUGGGCGACCAGUGAUACUUAGAGAGCAAGUCGUCGGC | 167-C7trc |
| 231 | D-RNA | GCGCGCGCUCAGUAAGAAAUUGAAAGUUCAGAAUGUCGUCGCGC | 167-B5trc |
| 232 | D-RNA | AGUGUGUGGCAGGCUAAGGAGAUAUUCCGAGACCACGCU | 184-D7trc |
| 233 | D-RNA | AGUGUGUGGCAGACUAUGGAUAGACUCCGAGACCACGCU | 184-D6trc |
| 234 | D-RNA | AGCGUGAGGCGACCAGCGGAUUACUUAGAGAGUCACGCU | 184-E5trc |
| 235 | D-RNA | AGCGUGAAGGGGACCAGCGUUACUUACAGAGUUCACGCU | 184-G6trc |
| 236 | D-RNA | AGCGUGUGAUGUAUGUAGCACCGUAUCAGAGGACACGCU | 184-B7trc |
| 237 | D-RNA | AGCGUGAGGCGACCCGUGUUUCGUAGAGAGUCACGCU | 184-B6trc |
| 238 | D-RNA | 5' PEG-GCACGUCCCUCACCGGUGCAAGUGAAGCCGUGGCUCUGCG | NOX-E36-5' PEG |
| 239 | D-RNA | GCACGUCCCUCACCGGUGCAAGUGAAGCCGUGGCUCUGCG-3'PEG | NOX-E36-3' PEG |
| 240 | D-RNA | GAGAUGGCGACAUUGGUUGGGCAUGAGGCGAGGCCCUUUGAUGAAUCCGCGGCCAUUC | 188-A3-001 |
| 241 | D-RNA | GAUGGCGACAUUGGUUGGGCAUGAGGCGAGGCCCUUUGAUGAAUCCGCGGCCAUUC | 188-A3-004 |
| 242 | D-RNA | GGCGACAUUGGUUGGGCAUGAGGCGAGGCCCUUUGAUGAAUCCGCGGCCAUUC | 188-A3-005 |
| 243 | D-RNA | GGCGACAUUGGUUGGGCAUGAGGCGAGGCCCUUUGAUGAAUCCGCGGCCAUU | 188-A3-006 |
| 244 | D-RNA | GGCGACAUUGGUUGGGCAUGAGGCGAGGCCCUUUGAUGAAUCCGCGGCCA | (D-)188-A3-007 = (D-)mNOX-E36 |
| 245 | D-RNA | GCUGGUUACCGAGGGGGCGUCGUUGGAGUUUGGUUGGUUGUCACCAGC | 189-G7-001 |
| 246 | D-RNA | CUGGUUACCGAGGGGGCGUCGUUGGAGUUUGGUUGGUUGUCACCAG | 189-G7-002 |
| 247 | D-RNA | UGGUUACCGAGGGGGCGUCGUUGGAGUUUGGUUGGUUGUCACCA | 189-G7-003 |
| 248 | D-RNA | GCCGGUUACCGAGGGGGCGUCGUUGGAGUUUGGUUGGUUGUCACCGGC | 189-G7-007 |
| 249 | D-RNA | GCCGGCUACCGAGGGGGCGUCGUUGGAGUUUGGUUGGUUGUCGCCGGC | 189-G7-008 |
| 250 | D-RNA | GCGCGUACCGAGGGGGCGUCGUUGGAGUUUGGUUGGUUGUCCGCGC | 189-G7-010 |
| 251 | D-RNA | GGGCCUACCGAGGGGGCGUCGUUGGAGUUUGGUUGGUUGUCGGCCC | 189-G7-012 |
| 252 | L-protein | QPDAVNAPLTCCYSFTGKMIPMSRLENYKRITSSRCPKEAVVFVTKLKREICADPNKEWVQ KYIRKLDQNQVRSET | rat MCP-1 |
| 253 | L-RNA | 5' PEG-GGCGACAUUGGUUGGGCAUGAGGCGAGGCCCUUUGAUGAAUCCGCGGCCA | mNOX-E36-5'PEG |
| 254 | L-RNA | GGCGACAUUGGUUGGGCAUGAGGCGAGGCCCUUUGAUGAAUCCGCGGCCA-3'PEG | mNOX-E36-3'PEG |
| 255 | L-DNA | 5'-GAGGGACGTGC-(Spacer18)₂-NH4⁺ -3' | NOX-E36 Capture probe |
| 256 | L-DNA | 5'- Biotin-(Spacer18)₂-CGCAGAGCC | NOX-E36 Detect (-ion) probe |
| 257 | L-Protein | KSMQVPFSRCCFSFAEQEIPLRAILCYRNTSSICSNEGLIFKLKRGKEACALDTVGWVQRH RKMLRHCPSKRK | CCL1/I-309 |
| 258 | L-Protein | SLAADTPTACCFSYTSRQIPQNFIADYFETSSQCSKPGVIFLTKRSRQVCADPSEEWVQKY VSDLELSA | CCL3/MIP-1α |
| 259 | L-Protein | APMGSDPPTACCFSYTARKLPRNFVVDYYETSSLCSQPAVVFQTKRSKQVCADPSESWVQE YVYDLELN | CCL4/MIP-1β |
| 260 | L-Protein | SPYSSDTTPCCFAYIARPLPRAHIKEYFYTSGKCSNPAVVFVTRKNRQVCANPEKKWVREY INSLEMS | CCL5/RANTES |
| 261 | L-Protein | FNPQGLAQPDALNVPSTCCFTFSSKKISLQRLKSYVITTSRCPQKAVIFRTKLGKEICADP KEKWVQNYMKHLGRKAHTLKT | CCL13/MCP-4 |
| 262 | L-Protein | TKTESSSRGPYHPSECCFTYTTYKIPRQRIMDYYETNSQCSKPGIVFITKRGHSVCTNPSD KWVQDYIKDMKEN | CCL14/HCC-1 |
| 263 | L-Protein | ASVATELRCQCLQTLQGIHPKNIQSVNVKSPGPHCAQTEVIATLKNGRKACLNPASPIVKK IIEKMLNSDKSN | CXCL1/GROα |
| 264 | L-Protein | APLATELRCQCLQTLQGIHLKNIQSVKVKSPGPHCAQTEVIATLKNGQKACLNPASPMVKK IIEKMLKNGKSN | CXCL2/GROβ |
| 265 | L-Protein | ASVVTELRCQCLQTLQGIHLKNIQSVNVRSPGPHCAQTEVIATLKNGKKACLNPASPMVQK IIEKILNKGSTN | CXCL3/GROγ |
| 266 | L-Protein | EAEEDGDLQCLCVKTTSQVRPRHITSLEVIKAGPHCPTAQLIATLKNGRKICLDLQAPLYK KIIKKLLES | CXCL4/PF4 |
| 267 | L-Protein | GPAAAVLRELRCVCLQTTQGVHPKMISNLQVFAIGPQCSKVEVVASLKNGKEICLDPEAPFL KKVIQKILDGGNKEN | CXCL5/ENA-78 |
| 268 | L-Protein | GPVSAVLTELRCTCLRVTLRVNPKTIGKLQVFPAGPQCSKVEVVASLKNGKQVCLDPEAPFL KKVIQKILDSGNKKN | CXCL6/GCP-2 |
| 269 | L-Protein | SSTKGQTKRNLAKGKEESLDSDLYAELRCMCIKTTSGIHPKNIQSLEVIGKGTHCNQVEVIA TLKDGRKICLDPDAPRIKKIVQKKLAGDESAD | CXCL7/NAP-2 |
| 270 | L-Protein | EGAVLPRSAKELRCQCIKTYSKPFHPKFIKELRVIESGPHCANTEIIVKLSDGRELCLDPKE NWVQRVVEKFLKRAENS | CXCL8/IL-8 |
| 271 | L-Protein | TPVVRKGRCSCISTNQGTIHLQSLKDLKQFAPSPSCEKIEIIATLKNGVQTCLNPDSADVKE LIKKWEKQVSQKKKQKNGKKHQKKKVLKVRKSQRSRQKKTT | CXCL9/MIG |
| 272 | L-Protein | VPLSRTVRCTCISISNQPVNPRSLEKLEIIPASQFCPRVEIIATMKKKGEKRCLNPESKAIK NLLKAVSKERSKRSP | CXCL10/IP-10 |
| 273 | L-Protein | FPMFKRGRCLCIGPGVKAVKVADIEKASIMYPSNNCDKIEVIITLKENKGQRCLNPKSKQAR LIIKKVERKNF | CXCL11/I-TAC |
| 274 | L-Protein | KPVSLSYRCPCRFFESHVARANVKHLKILNTPNCALQIVARLKNNNRQVCIDPKLKWIQEYL EKALNKRFKM | CXCL12α/SDF-1α |
| 275 | L-Protein | KPVSLSYRCPCRFFESHVARANVKHLKILNTPNCALQIVARLKNNNRQVCIDPKLKWIQEYL EKALNKRFKM | CXCL12β/SDF-1β |
| 276 | L-Protein | QHHGVTKCNITCSKMTSKIPVALLIHYQQNQASCGKRAIILETRQHRLFCADPKEQWVKDAM QHLDRQAAALTRNG | CX₃CL1/Fractalkine |
| 277 | L-Protein | VGSEVSDKRTCVSLTTQRLPVSRIKTYTITEGSLRAVIFITKRGLKVCADPQATWVRDVVRS MDRKSNTRNNMIQTKPTGTQQSTNTAVTLTG | XCL1/Lymphotactin |
| 278 | L-RNA | 5'-Biotin-GCACGUCCCUCACCGGUGCAAGUGAAGCCGUGGCUCUGCG | biotinylated NOX-E36 |
| 279 | L-RNA | 5'-UAAGGAAACUCGGUCUGAUGCGGU AGCGCUGUGCAGAGCU | POC |
| 280 | L-RNA | 5'-PEG-UAAGGAAACUCGGUCUGAUGCGGU AGCGCUGUGCAGAGCU-3' | POC-PEG |
| 281 | L-DNA | 5'-CCAATGTCGCC-(Spacer18)₂-NH4⁺ -3' | mNOX-E36 Capture probe |
| 282 | L-DNA | 5'- Biotin-(Spacer18)₂-CGCAGAGCC | mNOX-E36 Detect (-ion) probe |
| 283 | L-protein | QPDAINSPVTCCYTFTGKKISSQRLGSYKRVTSSKCPKEAVIFKTILAKEICADPEQKWVQ DAVKQLDKKAQTPKP | horse MCP-1 (Equus caballus) |
| 284 | L-protein | QPDAINSQVACCYTFNSKKISMQRLMNYRRVTSSKCPKEAVIFKTILGKELCADPKQKWVQ DSINYLNKKNQTPKP | bovine MCP-1 (Bos Taurus) |
| 285 | L-protein | QPDAVNAPLTCCYSFTGKMIPMSRLENYKRITSSRCPKEAVVFVTKLKREICADPNKEWVQ KYIRKLDQNQVRSETTVFYKIASTLRTSAPLNVNLTHKSEANASTLFSTTTSSTSVEVTSM TEN | rat MCP-1 (Rattus norvegicus) |
| 286 | L-protein | MLSTSRSRFIRNTNESGEEVTTFFDYDYGAPCHKFDVKQIGAQLLPPLYSLVFIFGFVGNM LVVLILINCKKLKCLTDIYLLNLAISDLLFLITLPLWAHSAANEWVFGNAMCKLFTGLYHI GYFGGIFFIILLTIDRYLAIVHAVFALKARTVTFGVVTSVITWLVAVFASVPGIIFTKCQK EDSVYVCGPYFPRGWNNFHTIMRNILGLVLPLLIMVICYSGILKTLLRCRNEKKRHRAVRV IFTIMIVYFLFWTPYNIVILLNTFQEFFGLSNCESTSQLDQATQVTETLGMTHCCINPIIY AFVGEKFRSLFHIALGCRIAPLQKPVCGGPGVRPGKNVKVTTQGLLDGRGKGKSIGRAPEA SLQDKEGA | Human C-C chemokine receptor type 2 |

The invention is described in the following Figures and Examples in more detail from which further embodiment, features and advantages may be taken.

In connection therewith,
Fig. 1 shows an alignment of sequences of related RNA ligands binding to human MCP-1 indicating the sequence motif ("Type 1A") that is in a preferred embodiment in its entirety essential for binding to human MCP-1;
Fig. 2 shows an alignment of sequences of related RNA ligands binding to human MCP-1 indicating the sequence motif ("Type 1B") that is in a preferred embodiment in its entirety essential for binding to human MCP-1 and derivatives of RNA ligands 180-D1-002;
Fig. 3 shows an alignment of sequences of related RNA ligands binding to human MCP-1 indicating the sequence motif ("Type 2") that is in a preferred embodiment in its entirety essential for binding to human MCP-1;
Fig. 4 shows an alignment of sequences of related RNA ligands binding to human MCP-1 indicating the sequence motif ("Type 3") that is in a preferred embodiment in its entirety essential for binding to human MCP-1;
Fig. 5 shows derivatives of RNA ligands 178-D5 and 181-A2 (human MCP-1 RNA ligands of sequence motif "Type 3");
Fig. 6 shows an alignment of sequences of related RNA ligands binding to human MCP-1 indicating the sequence motif ("Type 4") that is in a preferred embodiment in its entirety essential for binding to human MCP-1 (other sequences);
Fig. 7 shows a table of sequences of several different RNA ligands binding to human MCP-1 which cannot be related to the MCP-1 binding sequence motifs "Type 1A", "Type 1B"; "Type 2", "Type 3" or "Type 4";
Fig. 8 shows alignments of derivatives of RNA ligand 188-A3-001 and of 189-G7-001 that bind to murine MCP-1;
Fig. 9 shows the result of a binding analysis of the aptamer D-NOX-E36 to biotinylated human D-MCP-1 at room temperature and 37°C, represented as binding of the aptamer over concentration of biotinylated human D-MCP-1 ;
Fig. 10 shows the result of a binding analysis of the aptamer D-mNOX-E36 to biotinylated murine D-MCP-1 at 37°C, represented as binding of the aptamer over concentration of biotinylated murine D-MCP-1;
Fig. 11 shows MCP-1-induced Ca⁺⁺-release in THP-1 cells, whereas a dose-response curve for human MCP-1 was obtained, indicating a half effective concentration (EC₅₀) of approximately 3 nM, represented as difference in fluorescence to blank over concentration of human MCP-1;
Fig. 12 shows the efficacy of Spiegelmer NOX-E36 in a calcium release assay; cells were stimulated with 3 nM human MCP-1 preincubated at 37°C with various amounts of Spiegelmer NOX-E36, represented as percentage of control over concentration of NOX-E36;
Fig. 13 shows the efficacy of Spiegelmer mNOX-E36 in a calcium release assay; cells were stimulated with 5 nM murine MCP-1 preincubated at 37°C with various amounts of Spiegelmer mNOX-E36, represented as percentage of control over concentration of mNOX-E36;
Fig. 14 shows the human MCP-1-induced chemotaxis of THP-1 cells whereas after 3 hours migration of THP-1 cells towards various MCP-1 concentrations a dose-response curve for MCP-1 was obtained, represented as X-fold increase compared to control over concentration of human MCP-1;
Fig. 15 shows the efficacy of Spiegelmer NOX-E36 in a chemotaxis assay; cells were allowed to migrate towards 0.5 nM human MCP-1 preincubated at 37°C with various amounts of Spiegelmer NOX-E36, represented as percentage of control over concentration of Spiegelmer NOX-E36;
Fig. 16 shows the efficacy of Spiegelmer mNOX-E36 in a chemotaxis assay; cells were allowed to migrate towards 0.5 nM murine MCP-1 preincubated at 37°C with various amounts of Spiegelmer NOX-E36, represented as percentage of control over concentration of Spiegelmer mNOX-E36;
Fig. 17 shows the Biacore 2000 sensorgram indicating the K_{D} value of Spiegelmer NOX-E-36 binding to human MCP-1 which was immobilized on a PioneerF1 sensor chip by amine coupling procedure, represented as response (RU) over time;
Fig. 18 shows the Biacore 2000 sensorgram indicating binding of Spiegelmer NOX-E36 to human MCP-family proteins (huMCP-1, huMCP-2, huMCP-3) and human eotaxin, which were immobilized by amine coupling procedure on a PioneerF1 and a CM4 sensor chip, respectively, represented as response (RU) over time;
Fig. 19 shows the Biacore 2000 sensorgram indicating binding of Spiegelmer NOX-E36 to MCP-1 from different species (canine MCP-1, monkey MCP-1, human MCP-1, porcine MCP-1, rabbit MCP-1, mouse MCP-1, rat MCP-1) whereas different forms of MCP-1 were immobilized by amine coupling procedure on PioneerF1 and a CM4 sensor chips, respectively, represented as response (RU) over time;
Fig. 20 shows the Biacore 2000 sensorgram indicating the K_{D} value of Spiegelmer 181-A2-018 binding to to human MCP-1 which was immobilized on a CM4 sensor Chip by amine coupling procedure, represented as response (RU) over time;
Fig. 21 shows the Biacore 2000 sensorgram indicating binding of Spiegelmer 181-A2-018 to human MCP-family proteins (huMCP-1, huMCP-2, huMCP-3) and human eotaxin which were immobilized by amine coupling procedure on a PioneerF1 and a CM4 sensor chip, respectively, represented as response (RU) over time;
Fig. 22 shows the Biacore 2000 sensorgram indicating binding of Spiegelmer 181-A2-018 to MCP-1 from different species (canine MCP-1, monkey MCP-1, human MCP-1, porcine MCP-1, rabbit MCP-1, mouse MCP-1, rat MCP-1) whereas different forms of MCP-1 were immobilized by amine coupling procedure on PioneerF1 and a CM4 sensor chips, respectively, represented as response (RU) over time;
Fig. 23 shows a Clustal W alignment of MCP-1 from different mammalian species as well as human MCP-2, MCP-3, and eotaxin (Positions 1-76 only);
Fig. 24A shows a table summarizing the binding specificity of NOX-E36 and 181-A2-018 regarding MCP-1 from different mammalian species as well as human MCP-2, MCP-3, and eotaxin;
Fig. 24B shows a table summarizing the selectivity of NOX-E36 as determined by Biacore analysis whereby biotinylated NOX-E36 was immobilized on a sensor chip surface and binding of a panel of various CC and CXC chemokines to NOX-E36 was analyzed;
Fig. 24C shows the kinetic analysis of NOX-E36 interacting with chemokines as determined by Biacore analysis whereby the chemokines were immobilized covalently on a CM5 sensor chip surface and various concentrations of the NOX-E36 were injected and NOX-E36s binding behaviour was analyzed using the BiaEvaluation software;
Fig. 24D shows the chemotaxis dose-response curve of THP-1 cell stimulation with MIP-1α with a half- effective concentration of about 0.2 nM;
Fig. 24E shows the Inhibition of MIP-1α induced chemotaxis by NOX-E36. NOX-E36 had no influence on the MIPla induced chemotaxis of THP-1 cells;

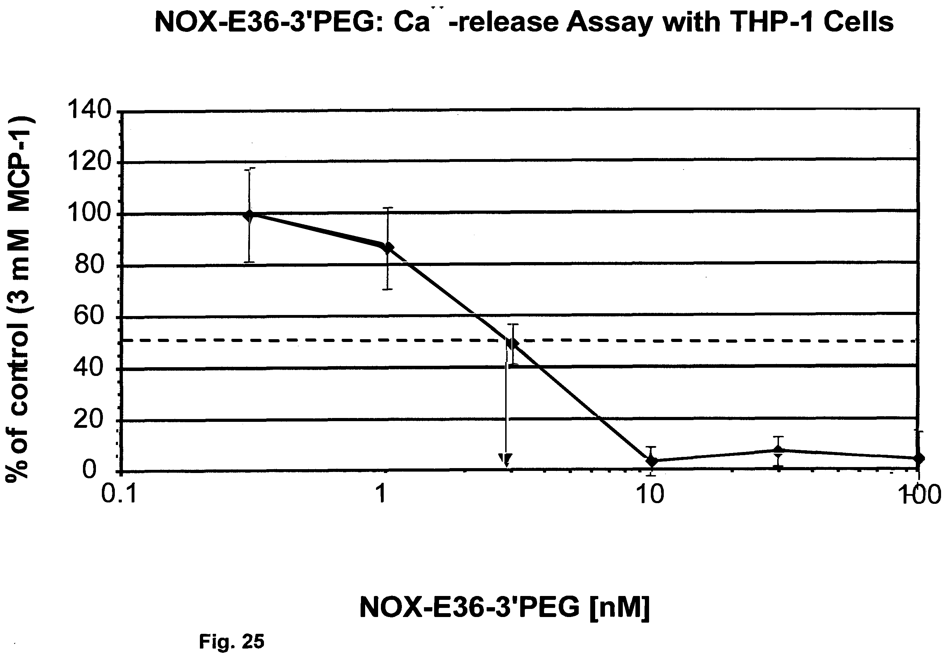

Fig. 25 shows the efficacy of Spiegelmer NOX-E36-3'-PEG in a calcium release assay; cells were stimulated with 3 nM human MCP-1 preincubated at 37°C with various amounts of Spiegelmer NOX-E36-3'-PEG, represented as percentage of control over concentration of Spiegelmer NOX-E36-3'-PEG;
Fig. 26 shows the efficacy of Spiegelmer NOX-E36-3'-PEG in a chemotaxis assay; cells were allowed to migrate towards 0.5 nM human MCP-1 preincubated at 37°C with various amounts of Spiegelmer NOX-E36-3'-PEG, represented as percentage of control over concentration of NOX-E36-3'-PEG;
Fig. 27A shows the efficacy of Spiegelmer NOX-E36-5'-PEG in a calcium release assay; cells were stimulated with 3 nM human MCP-1 preincubated at 37°C with various amounts of Spiegelmer NOX-E36-5'-PEG, represented as percentage of control over concentration of Spiegelmer NOX-E36-5'-PEG;
Fig. 27B shows the efficacy of Spiegelmer NOX-E36-5'-PEG in a chemotaxis assay; cells were allowed to migrate towards 0.5 nM human MCP-1 preincubated at 37°C with various amounts of Spiegelmer NOX-E36-5'-PEG, represented as percentage of control over concentration of Spiegelmer NOX-E36-5'-PEG;
Fig. 28 shows murine MCP-1-induced Ca⁺⁺-release in THP-1 cells, whereas a dose-response curve for murine MCP-1 was obtained, indicating a half effective concentration (EC₅₀) of approximately 5 nM, represented as difference in fluorescence to blank over concentration of murine MCP-1;
Fig. 29 shows the efficacy of anti-murine MCP-1 Spiegelmer mNOX-E36-3'-PEG in a calcium release assay; cells were stimulated with 3 nM murine MCP-1 preincubated at 37°C with various amounts of Spiegelmer mNOX-E36-3'-PEG, represented as percentage of control over concentration of Spiegelmer mNOX-E36-3'-PEG;
Fig. 30 shows the murine MCP-1-induced chemotaxis of THP-1 cells whereas after 3 hours migration of THP-1 cells towards various mMCP-1 concentrations a dose-response curve for mMCP-1 was obtained, represented as X-fold increase compared to control over concentration of murine MCP-1;
Fig. 31 shows the efficacy of anti-murine MCP-1 Spiegelmer mNOX-E36-3'-PEG in a chemotaxis assay; cells were allowed to migrate towards 0.5 nM murine MCP-1 preincubated at 37°C with various amounts of Spiegelmer mNOX-E36-3'-PEG, represented as percentage of control over concentration of anti-murine Spiegelmer mNOX-E36-3'-PEG;
Fig. 32 shows the Biacore 2000 sensorgram indicating the K_{D} value of aptamer D-mNOX-E36 binding to murine D-MCP-1 which was immobilized on a PioneerF1 sensor chip by amine coupling procedure, represented as response (RU) over time; and
Fig. 33 shows the Biacore 2000 sensorgram indicating binding of aptamer D-mNOX-E36 to human D-MCP-1 and murine D-MCP-1 whereas the two different forms of D-MCP-1 were immobilized by amine coupling procedure on PioneerF1 and a CM4 sensor chips, respectively, represented as response (RU) over time.

### Example 1: CCL2 inhibition with mNOX-E36 in mouse models of back-of-the-eye diseases

**Background:** Neovascularization processes in the back of the eye often lead to subsequent fibrosis due to wound healing processes. This is an important reason for blinding back-of-the-eye diseases such as neovascular age-related macular degeneration (nAMD) and proliferative diabetic retinopathy (PDR).

**Methods:** Studies with relevance for proliferative diabetic retinopathy and neovascular AMD are performed in *in vivo* mouse models. The studies are performed to assess the effect of CCL2 inhibition by mNOX-E36 on retinal angiogenesis, subretinal angiogenesis (choroidal neovascularization), and subretinal fibrosis.
mNOX-E36 has the nucleotide sequence: 
5’- GGCGACAUUGGUUGGGCAUGAGGCGAGGCCCUUUGAUGAAUCCGCGGCCA-
3’;
revmNOX-E36 has the nucleotide sequence:

5'-ACCGGCGCCUAAGUAGUUUCCCGGAGCGGAGUACGGGUUGGUUACAGCGG-
3'.

Doses for mNOX-E36 and revmNOX-E36 always refer to the oligonucleotide part of the molecules.

### 1. Retinal Angiogenesis

Retinal angiogenesis is assessed in the trVEGF029 (Kimba) mouse model (van Eeden, Tee et al. 2006) which transiently overexpresses human vascular endothelial growth factor (hVEGF) in photoreceptors. The expression of VEGF peaks at around 10 days postnatal and gradually decreases to an only slightly elevated level by 6 weeks postnatal. Despite transient hVEGF up-regulation, the short-term presence of elevated hVEGF induces vascular permeability, capillary nonperfusion/dropout, microaneurysms, retinal neovascularization (RNV), and occasional hemorrhages, all hallmarks of pathological angiogenesis.

The following examinations are performed:
- Posterior Segment Exam: Slit lamp and fluorescein fundus angiography/ posterior segment optical coherence tomography (PS-OCT; MICRON OCT system, Phoenix-Micron Inc, Bend, OR, USA) are performed immediately before the injection and weekly for up to 8 weeks to evaluate overall changes at the back of the eye (see study flow chart below).
- Histology: Kimba mice are sacrificed at Week 8. The eyes enucleated (16 eyes from 8 mice per treatment group) for retina flatmount are used to examine changes in retinal vasculature (CD31) and tissue apoptosis by TUNEL (Terminal deoxynucleotidyl transferase dUTP Nick End Labeling) staining (Kasagi, Gomyo et al. 1994).

The following groups are treated:
- Intravitreal mNOX-E36 group: intravitreal injection of 1 µL mNOX-E36 (10 mg/mL) in 5% (wt/vol) glucose to Kimba mice at postnatal weeks 3, 5 and 7; mice are sacrificed at postnatal week 8.
- Control group for intravitreal injection: intravitreal injection of 1 µL revmNOX-E36 control (10 mg/mL) in 5% (wt/vol) glucose to Kimba mice at postnatal weeks 3, 5 and 7; mice are sacrificed at postnatal week 8.
- Subcutaneous mNOX-E36 group: Subcutaneous injection of 20 mg/kg body weight mNOX-E36 in 5% (wt/vol) glucose is given to Kimba mice starting from postnatal week 3, then every other day (Q2D) till postnatal week 8. Mice are sacrificed at postnatal week 8.
- Control group for subcutaneous injection: Subcutaneous injection of 20 mg/kg body weight revmNOX-E36 control in 5% (wt/vol) glucose to Kimba mice starting from postnatal week 3, then every other day /Q2D till postnatal week 8. Mice are sacrificed at postnatal week 8.

### Study flowchart:

| **Timeline** | **Intravitreal groups** | **Systemic groups** |
|---|---|---|
| **Birth/ Week 0** | - | - |

| **Day 21/ Week 3** | Baseline FFA/PS-OCT imaging 1^{st} IVT injection* | Baseline FFA/PS-OCT imaging 1^{st} SC injection* Additional SC administration* Q2D until end of in-life experiment |
|---|---|---|
| **Week 4** | FFA/PS-OCT imaging | FFA/PS-OCT imaging |
| **Week 5** | FFA/PS-OCT imaging 2^{nd} IVT injection* | FFA/PS-OCT imaging |
| **Week 6** | FFA/PS-OCT imaging | FFA/PS-OCT imaging |
| **Week 7** | FFA/PS-OCT imaging 3^{rd} IVT injection* | FFA/PS-OCT imaging |
| **Up to Week 8** | FFA/PS-OCT imaging Eye enucleation, retinal flatmount | FFA/PS-OCT imaging Eye enucleation, retinal flatmount |

| | | |
|---|---|---|
| *, mNOX-E36 or revmNOX-E36 control FFA, fluorescein fundus angiography; PS-OCT, posterior segment optical coherence tomography; IVT, intravitreal; SC, subcutaneous; Q2D, every other day | | |

### Results:

Study results demonstrate that treatment with mNOX-E36 reduces pathological angiogenesis in the retina indicative of a clinically meaningful benefit.

### 2. Subretinal Angiogenesis (Choroidal Neovascularization)

Subretinal angiogenesis is assessed in the mouse model of laser-induced choroidal neovascularization (CNV) (Tobe, Ortega et al. 1998). 6-8 week-old C67BL/6 mice are subjected to laser injury which causes rupture of Bruch's membrane and abnormal blood vessel formation from the choroid through the retinal pigmented epithelium into the subretinal space, hallmarks of subretinal pathological angiogenesis. In the model, choroidal neovascularization reaches the highest level on day 14 following laser injury while fibrosis begins 7-14 days post laser and continues to expand beyond day 35 after laser injury.

The following examinations are performed:
- Posterior Segment Exam: Slit lamp and fluorescein fundus angiography/ posterior segment optical coherence tomography (PS-OCT; MICRON OCT system, Phoenix-Micron Inc, Bend, OR, USA) are performed immediately before the injection and weekly up to 2 weeks as indicated on the flowchart and used to evaluate changes at the back of the eye.
- Histology: Mice are sacrificed at 14 days post-laser treatment. The eyes enucleated (16 eyes from 8 mice per treatment group) for retinal pigment epithelium (RPE) flatmount are used to examine changes for markers of inflammation (CD11b, F4/80), markers of angiogenesis (CD31) and tissue apoptosis by TUNEL (Terminal deoxynucleotidyl transferase dUTP Nick End Labeling) staining (Kasagi, Gomyo et al. 1994).

The following groups are treated:
To assess prevention of subretinal angiogenesis after subretinal administration:
- Subretinal mNOX-E36 group: subretinal injection of 1 µL mNOX-E36 (10 mg/mL) in 5% (wt/vol) glucose to mice one day prior laser-induced CNV; mice are sacrificed at Day 14.
- Control group: subretinal injection of 1 µL revmNOX-E36 control (10 mg/mL) in 5% (wt/vol) glucose to mice one day prior laser-induced CNV; mice are sacrificed at Day 14.

To assess regression of subretinal angiogenesis after subretinal administration:
- Subretinal mNOX-E36 group: subretinal injection of 1 µL mNOX-E36 (10 mg/mL) in 5% (wt/vol) glucose to mice subjected to laser-induced CNV 3 days after laser treatment; mice are sacrificed at Day 14.
- Control group: subretinal injection of 1 µL revmNOX-E36 control (10 mg/mL) in 5% (wt/vol) glucose to mice subjected to laser-induced CNV 3 days after laser treatment; mice are sacrificed at Day 14.

To assess prevention of subretinal angiogenesis after systemic administration:
- Subcutaneous mNOX-E36 group: Subcutaneous injection of 20 mg/kg body weight mNOX-E36 in 5% (wt/vol) glucose to mice 1 day prior to laser-induced CNV, then every other day /Q2D until Day 14. Mice are sacrificed at Day 14.
- Control group for subcutaneous injection: Subcutaneous injection of 20 mg/kg body weight revmNOX-E36 control in 5% (wt/vol) glucose to mice 1 day prior to laser-induced CNV, then every other day /Q2D until Day 14. Mice are sacrificed at Day 14.

To assess regression of subretinal angiogenesis after systemic administration:
- Subcutaneous mNOX-E36 group: Subcutaneous injection of 20 mg/kg body weight mNOX-E36 in 5% (wt/vol) glucose to mice subjected to laser-induced CNV 3 days after laser treatment, then every other day /Q2D until Day 14. Mice are sacrificed at Day 14.
- Control group for subcutaneous injection: Subcutaneous injection of 20 mg/kg body weight revmNOX-E36 control in 5% (wt/vol) glucose to mice subjected to laser-induced CNV 3 days after laser treatment, then every other day /Q2D until Day 14. Mice are sacrificed at Day 14.

### Study flowchart:

| **Timeline** | **Subretinal prevention group** | **Subretinal regression group** | **Systemic prevention group** | **Systemic regression group** |
|---|---|---|---|---|
| **Day -1** | SR injection* | - | SC injection* Additional SC administration Q2D until end of in-life | - |
| **Day 0 (CNV)** | FFA/PS-OCT imaging at baseline Laser injury | | | |
| **Day 3** | - | SR injection* | - | SC injection* Additional SC administration Q2D until end of in-life |
| **Day 7** | FFA/PS-OCT imaging | | | |
| **Day 14** | FFA/PS-OCT imaging Eye enucleation, retinal flatmount | | | |

| | | | | |
|---|---|---|---|---|
| *, mNOX-E36 or revmNOX-E36 control FFA, fluorescein fundus angiography; PS-OCT, posterior segment optical coherence tomography; SR, subretinal; SC, subcutaneous; Q2D, every other day | | | | |

### Results:

Study results demonstrate that treatment with mNOX-E36 reduces pathological subretinal angiogenesis indicative of a clinically meaningful benefit.

### 3. Subretinal Fibrosis

Subretinal fibrosis is assessed in the mouse model of laser-induced choroidal neovascularization (CNV) (Tobe, Ortega et al. 1998). 6-8 week-old C67BL/6 mice are subjected to laser injury which causes rupture of Bruch's membrane and abnormal blood vessel formation from the choroid through the retinal pigmented epithelium into the subretinal space. Choroidal neovascularization reaches the highest level on day 14 following laser injury while the pathological process of subretinal fibrosis begins 7-14 days post laser and continues to expand beyond day 35 after laser injury.

The following examinations are performed:
- Posterior Segment Exam: Slit lamp and fluorescein fundus angiography/ posterior segment optical coherence tomography (PS-OCT; MICRON OCT system, Phoenix-Micron Inc, Bend, OR, USA) are performed immediately before the injection and weekly up to 5 weeks/35 days as indicated on the flowchart and used to evaluate changes at the back of the eye.
- Histology: Mice are sacrificed at 35 days post-laser treatment. The eyes enucleated (16 eyes from 8 mice per treatment group) for retinal pigment epithelium (RPE) flatmount are used to examine changes for markers of inflammation (CD11b, F4/80), markers of fibrosis (type I collagen, fibronectin) and tissue apoptosis by TUNEL (Terminal deoxynucleotidyl transferase dUTP Nick End Labeling) staining (Kasagi, Gomyo et al. 1994).

The following groups are treated:
To assess prevention of fibrosis after subretinal administration:
- Subretinal mNOX-E36 group: subretinal injection of 1 µL mNOX-E36 (10mg/mL) in 5% (wt/vol) glucose to mice subjected to laser-induced CNV on day 7 and day 21 following the treatment with the laser; mice are sacrificed at Day 35.
- Control group: subretinal injection of 1 µL revmNOX-E36 control (10 mg/mL) in 5% (wt/vol) glucose to mice subjected to laser-induced CNV on day 7 and day 21 following the treatment with the laser; mice are sacrificed at Day 35.

To assess regression of fibrosis after subretinal administration:
- Subretinal mNOX-E36 group: subretinal injection of 1 µL mNOX-E36 (10 mg/mL) in 5% (wt/vol) glucose to mice subjected to laser-induced CNV on day 14 and day 21 following the treatment with the laser; mice are sacrificed at Day 35.
- Control group: subretinal injection of 1 µL revmNOX-E36 control (10 mg/mL) in 5% (wt/vol) glucose to mice subjected to laser-induced CNV on day 14 and day 21 following the treatment with the laser; mice are sacrificed at Day 35.

To assess prevention of fibrosis after systemic administration:
- Subcutaneous mNOX-E36 group: Subcutaneous injection of 20 mg/kg body weight mNOX-E36 in 5% (wt/vol) glucose to mice on day 7, then every other day /Q2D until Day 35 following the treatment with the laser. Mice are sacrificed at Day 35.
- Control group: Subcutaneous injection of 20 mg/kg body weight revmNOX-E36 control in 5% (wt/vol) glucose to mice on day 7, then every other day /Q2D until Day 35 following the treatment with the laser. Mice are sacrificed at Day 35.

To assess regression of fibrosis after systemic administration:
- Subcutaneous mNOX-E36 group: Subcutaneous injection of 20 mg/kg body weight mNOX-E36 in 5% (wt/vol) glucose to mice on day 14, then every other day /Q2D until Day 35 following the treatment with the laser. Mice are sacrificed at Day 35.
- Control group: Subcutaneous injection of 20 mg/kg body weight revmNOX-E36 control in 5% (wt/vol) glucose to mice on day 14, then every other day /Q2D until Day 35 following the treatment with the laser. Mice are sacrificed at Day 35.

### Study flowchart:

| **Timeline** | **Subretinal prevention group** | **Subretinal regression group** | **Systemic prevention group** | **Systemic regression group** |
|---|---|---|---|---|
| **Day 0 (CNV)** | FFA/OCT imaging at baseline Laser injury | | | |
| **Day 7** | FFA/ PS-OCT imaging 1^{st} SR injection* | FFA/PS-OCT imaging | FFA/PS-OCT imaging 1^{st} SC injection* Additional SC administration Q2D until end of in-life | FFA/PS-OCT imaging |
| **Day 14** | FFA/PS-OCT imaging | FFA/PS-OCT imaging 1^{st} SR injection* | FFA/PS-OCT imaging | FFA/PS-OCT imaging 1^{st} SC injection* Additional SC administration Q2D until end of in-life |
| **Day 21** | FFA/PS-OCT imaging 2^{nd} SR injection* | FFA/PS-OCT imaging 2^{nd} SR injection* | FFA/PS-OCT imaging | FFA/PS-OCT imaging |
| **Day 28** | FFA/PS-OCT imaging | | | |
| **Day 35** | FFA/PS-OCT imaging Eye enucleation, retinal flatmount | | | |

| | | | | |
|---|---|---|---|---|
| *, mNOX-E36 or revmNOX-E36 control FFA, fluorescein fundus angiography; PS-OCT, posterior segment optical coherence tomography; SR, subretinal; SC, subcutaneous; Q2D, every other day | | | | |

### Results:

Study results demonstrate that treatment with mNOX-E36 reduces the pathological process of subretinal fibrosis indicative of a clinically meaningful benefit.

### Example 2: CCL2 inhibition with NOX-E36 (emapticap pegol) in a pig model of proliferative vitreoretinopathy

**Background:** Proliferative vitreoretinopathy (PVR) is a blinding condition that can occur secondary to penetrating ocular trauma, retinal detachment, or following surgery for retinal detachment repair *(*The classification of retinal detachment with proliferative vitreoretinopathy; Ophthalmology 1983, 90:121). In these conditions, a breach in the integrity of the retina introduces macrophages, retinal pigment epithelial (RPE) cells, glial cells, and fibroblasts into the vitreous, where they proliferate, incite inflammation and result in tissue scarring and obliteration of vision.

A representative model of PVR can be induced in the minipig (Umazume, Barak et al. 2012). The similarity in anatomy and physiology of the minipig eye with the human eye makes this an ideal animal model for testing therapeutics for PVR. In this study, the potential of NOX-E36 (INN, emapticap pegol; nucleotide sequence 5'-GCACGUCCCUCACCGGUGCAAGUGAAGCCGUGGCUC UGCG-3') which binds and inhibits human and porcine CCL2, to mitigate the severity of PVR in a minipig model is investigated. As control, the nonfunctional Spiegelmer revNOX-E36 (5'-GCGUCUCGGUGCCGAAGUGAACGUGGCCACUCCCUGCACG-3') is used. Doses for NOX-E36 and revmNOX-E36 always refer to the oligonucleotide part of the molecules.

Methods: The study involves the *in vivo* PVR minipig model with 2 study arms.
- Arm 1 (Control, revNOX-E36):
   Intravitreal injection of 100 µL control revNOX-E36, 13 mg/mL is given at the end of surgery on D1 and subsequently on D8 and D15; in addition, subcutaneous injection of Spiegelmer control revNOX-E36, 1mg/kg body weight is given at the end of surgery on D1 and subsequently on D4, D8, D11, D15 and D18.
- Arm 2 (NOX-E36):
   Intravitreal injection of 100 µL emapticap pegol (NOX-E36), 13 mg/mL is given at the end of surgery on D1 and subsequently on D8 and D15; in addition, subcutaneous injection of emapticap pegol, 1mg/kg body weight given at the end of surgery on D1 and subsequently on D4, D8, D11, D15 and D18.

A total of 12 eyes of 6 minipigs is used for this experiment. Minipigs are anesthetized with intramuscular injection of ketamine and xylazine (11-30 mg/kg of ketamine + 1-2 mg/kg of xylazine). For surgery, animals are further intubated and are maintained under isoflurane (1-3%). Six eyes from 3 pigs serve as intervention group (NOX-E36) and another 6 eyes from 3 pigs serve as control group (revNOX-E36).

RPE cell isolation: RPE cells are isolated from cadaveric pig eyes using dispase (Umazume, Barak et al. 2012) The cadaveric eyes are harvested within 2-4 hours from death to preservation (DTP) and kept at 4°C. The time from DTP to tissue processing is within 24-30 hours.

All the steps are performed under sterile conditions at room temperature. An initial incision is made in cornea of the eye, the cornea/iris complex is carefully cut off 1-2 mm posterior to the corneal limbus and the vitreous is removed. The posterior eye cup is dissected into 4 quadrants with a razor blade and eye cup is placed flat. The sensory retina is removed with an inoculating loop and the RPE/choroid layer from the sclera is gently peeled and placed in a holding buffer. The RPE-choroid sheets are incubated in 2% dispase solution for 30 min at 37°C. The dispase is rinsed off and the cells placed back in a holding buffer to stop the dispase activity. The RPE layer is peeled off in large sheets from the choroid with fine forceps and the crude RPE containing preparation is filtered through a 70-µm nylon mesh filter (Sonoda, Spee et al. 2009). RPE sheets retained in the 40-µm filter are placed into a 50 ml centrifuge tube in 25 ml holding buffer. RPE sheets are broken apart by gently pipetting slowly up and down, using a sterile 9-inch glass pipette. The resulting preparation is kept on ice in 10 % FBS supplemented Dulbecco's modified Eagle's medium (DMEM) and switched to serum-free DMEM just before use.

Platelet rich plasma (PRP) is prepared from minipig homologous blood. Venous blood is collected from the minipig's ear vein into citrate blood tubes (one part 3.8 % sodium citrate [wt/vol] to nine parts whole blood). The blood is centrifuged at 1,200 rotations per minute for 10 minutes, and the upper third of the supernatant PRP is aspirated. The platelet count is adjusted to 600,000 platelets per mm³ using an automated Coulter counter. Then, 0.1 mL of PRP are injected into the vitreous cavity at the end of surgery.

Induction of PVR: PVR is induced in both eyes based on the modified methodology of Umazume et al. (Umazume, Barak et al. 2012). Three-port 23-gauge pars plana vitrectomy (Alcon Constellation) is performed to complete posterior vitreous detachment after core vitrectomy, followed by shaving of the peripheral vitreous. Then, inferior retinal detachment is induced by injecting balanced salt solution (Alcon Laboratories, Fort Worth, TX: NaCl, 0.64%; KCl, 0.075%; CaCl2 • 2 H₂O, 0.048%; MgCl₂ • 6H₂O, 0.03%; Na-acetate •3 H₂O, 0.39%; Na-citrate • 2 H₂O, 0.17%; NaOH or NaCl to adjust pH, and water for injection. The pH is approximately 7.5. All amounts in wt/vol) into the subretinal space, using a 39-gauge angled cannula. Subretinal injections are performed 1- disc diameter away from the optic disc, and any visible blood vessels are avoided. Four to five subretinal injections, at least one in each quadrant, are made to cause an inferior retinal detachment, avoiding detachment of the area centralis (analogous to the macula region in the human eye). Finally, RPE cells harvested from cadaveric pig eyes in 0.1 mL of Dulbecco's modified Eagle's medium (DMEM) and 0.1 mL platelet rich plasma (PRP) are injected into the vitreous cavity.

Ocular injections: A Hamilton syringe with a 31-gauge needle is used to intravitreally inject 100 µl NOX-E36 or control, 4 mm from the limbus in the inferotemporal quadrant at the end of surgery (D1) and on D8 and D15 post-surgery.

Topical tobramycin 0.3% is administered 4 times a day for 5 days after ocular injections. Visual inspections are conducted daily, following surgery. The animals' eyes are inspected for status of injection site and evidence of infection.

Investigations and examination: The retinal status is examined with an indirect ophthalmoscope through a +30 D fundus lens on days 1, 2, 8, 15, 22 and 29 by two double-masked ophthalmologists. Proliferative vitreoretinopathy is graded according to the revised PVR classification:
Stage 0: Normal retina (A)
Stage 1: Surface wrinkling (B)
Stage 2: Mild pucker (C)
Stage 3: Severe pucker (D)
Stage 4: Elevated pucker (E)
Stage 5: Partial retinal detachment (F)
Stage 6: Low detachment (G)
Stage 7: Total detachment (H)

Fundus photography and Optical Coherence Tomography (OCT): Fundus photographs and OCT are taken using a spectral domain (SD)-OCT system. OCT is performed prior to surgery and then weekly post-surgery on Days 2, 8, 15, 22 and 29 till endpoint euthanasia.

Enucleation and euthanasia procedures: The minipigs are euthanized with intraperitoneal pentobarbitone (60- 150 mg/kg) and then have both eyes enucleated.

Pathology procedures: All eyes undergo histopathological evaluation post-enucleation. The eyes are fixed with 10% formalin for histology analysis.

The following stains are used: H&E staining, alpha smooth muscle actin (a-SMA,); cytokeratin 8, 18, and 19 (CK); glial fibrillary acidic protein (GFAP), vimentin and fibronectin. These stains allow to detect inner folding of the retina, myofibroblast activation and RPE dedifferentiation, which are characteristics of PVR.

In general, OCT and fundus images are used to determine the PVR score for each animal. A week-by-week comparison of the median PVR grade between all animals in the revNOX-E36 and NOX-E36 groups is then used to determine the success of the treatment. Histopathology staining is used qualitatively to support the PVR scoring.

### Study flowchart:

RPE Isolation from cadaveric pig
Platelet rich plasma (PRP) is prepared from minipig homologous blood
D1: Minipigs are anesthetized with intramuscular injection of ketamine and xylazine (11-
30 mg/kg of ketamine + 1-2 mg/kg of xylazine). For surgery, animals are further intubated
and are maintained under isoflurane (1-3%)
D1: PVR induction (including injection of RPE cells and PRP into the vitreous cavity) on
both eyes per pig
D1: Immediately post-surgery, 100 µl of NOX-E36 or control revNOX-E36 is injected
intravitreally using Hamilton syringe with a 31-gauge needle. 1 mg/kg body weight of
NOX-E36 or control revNOX-E36 is given subcutaneously.
D4: 1 mg/kg body weight of NOX-E36 or control revNOX-E36 is given subcutaneously
D8, another dose of 100 µl of NOX-E36 or control revNOX-E36 is injected intravitreally
using Hamilton syringe with a 31-gauge needle. 1 mg/kg body weight of NOX-E36 or
control revNOX-E36 is given subcutaneously.
D11: 1 mg/kg body weight of NOX-E36 or control revNOX-E36 is given subcutaneously
D15, another dose of 100 µl of NOX-E36 or control revNOX-E36 is injected intravitreally
using Hamilton syringe with a 31-gauge needle.
D18: 1 mg/kg body weight of NOX-E36 or control revNOX-E36 is given subcutaneously

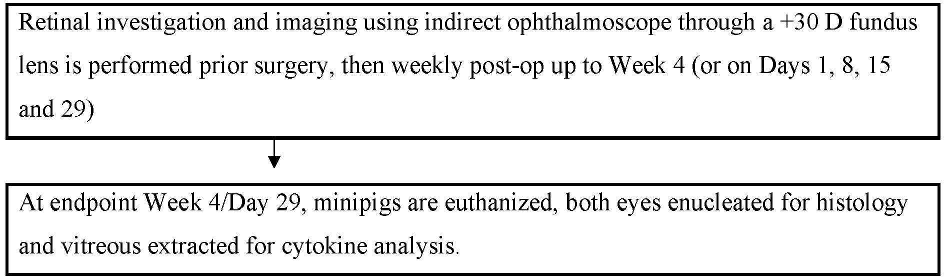
Retinal investigation and imaging using indirect ophthalmoscope through a +30 D fundus
lens is performed prior surgery, then weekly post-op up to Week 4 (or on Days 1, 8, 15
and 29)
At endpoint Week 4/Day 29, minipigs are euthanized, both eyes enucleated for histology
and vitreous extracted for cytokine analysis.

### Results:

Study results demonstrate that treatment with mNOX-E36 reduces pathological developments in the retina indicative of a clinically meaningful benefit.

### Example 3: Nucleic acids that bind human MCP-1

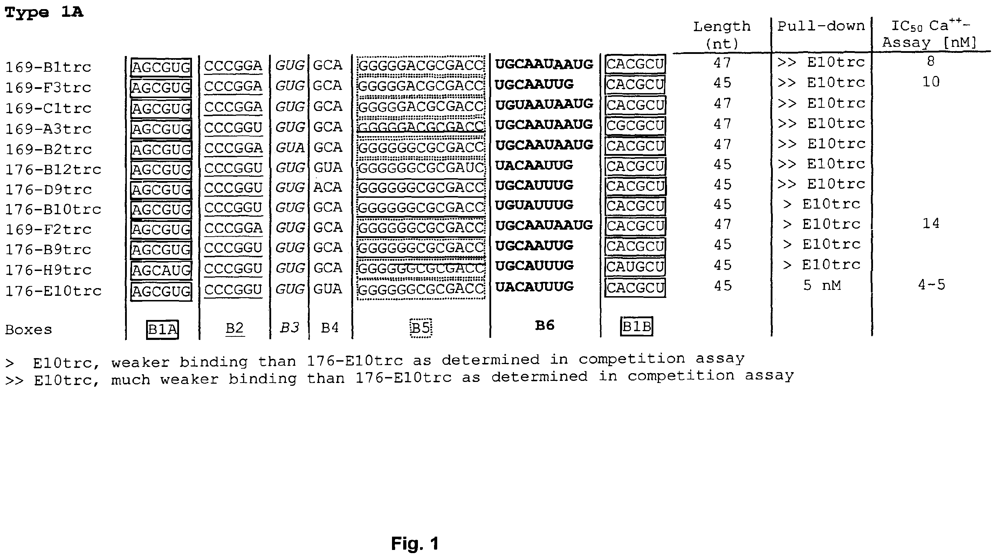

Using biotinylated human D-MCP-1 as a target, several nucleic acids that bind to human MCP-1 could be generated the nucleotide sequences of which are depicted in Figures 1 through 7. The nucleic acids were characterized on the aptamer, i. e. D-nucleic acid level using competitive or direct pull-down assays with biotinylated human D-MCP-1 (Example 6) or on the Spiegelmer level, i. e. L-nucleic acid with the natural configuration of MCP-1 (L-MCP) by surface plasmon resonance measurement using a Biacore 2000 instrument (Example 10), an *in vitro* cell culture Ca⁺⁺-release assay (Example 8), or an *in vitro* chemotaxis assay (Example 9).

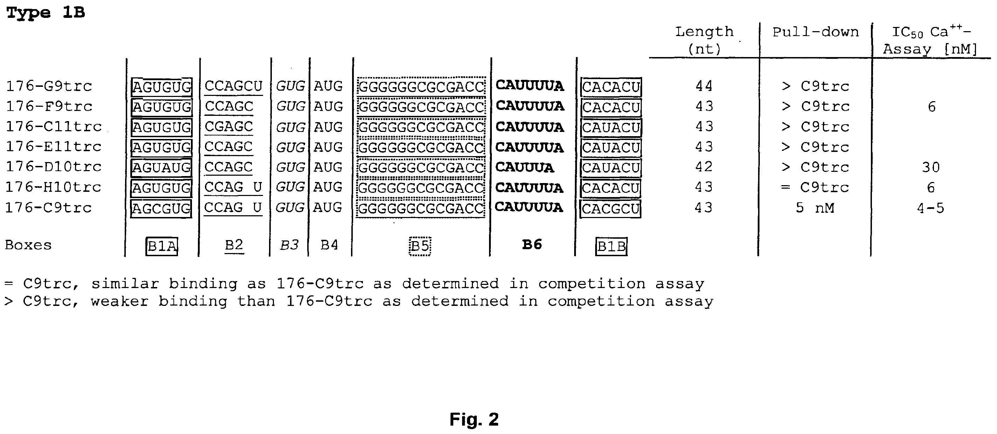

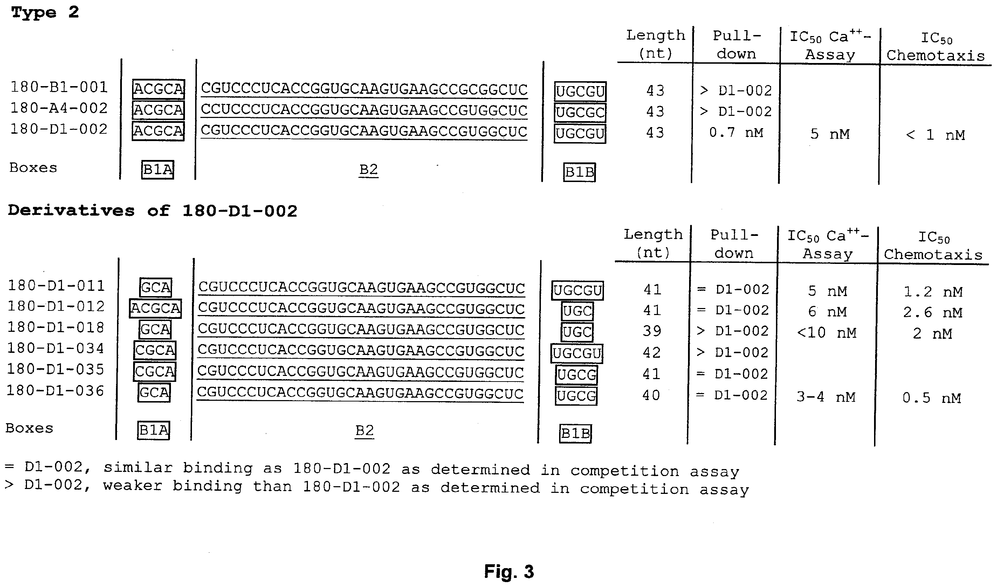

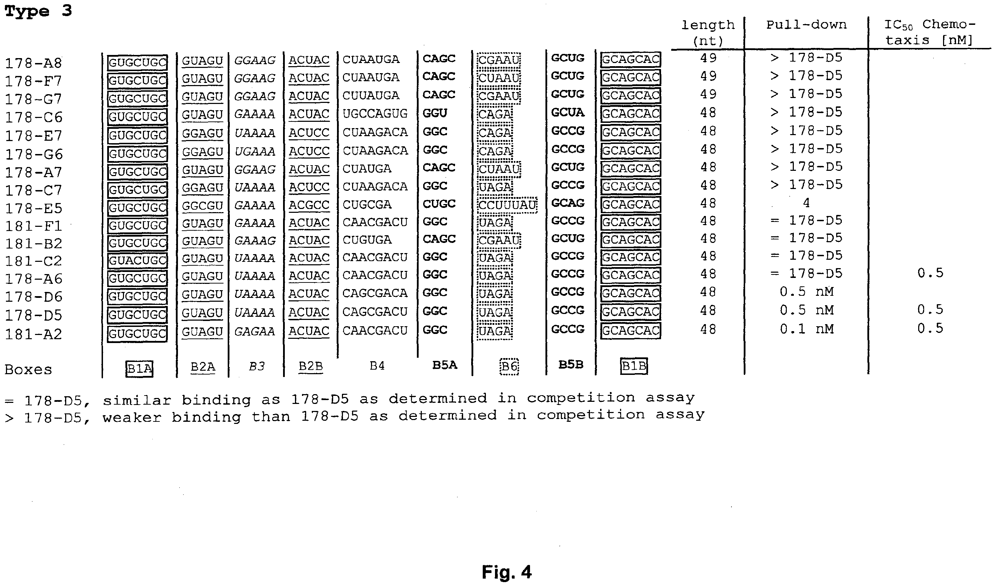

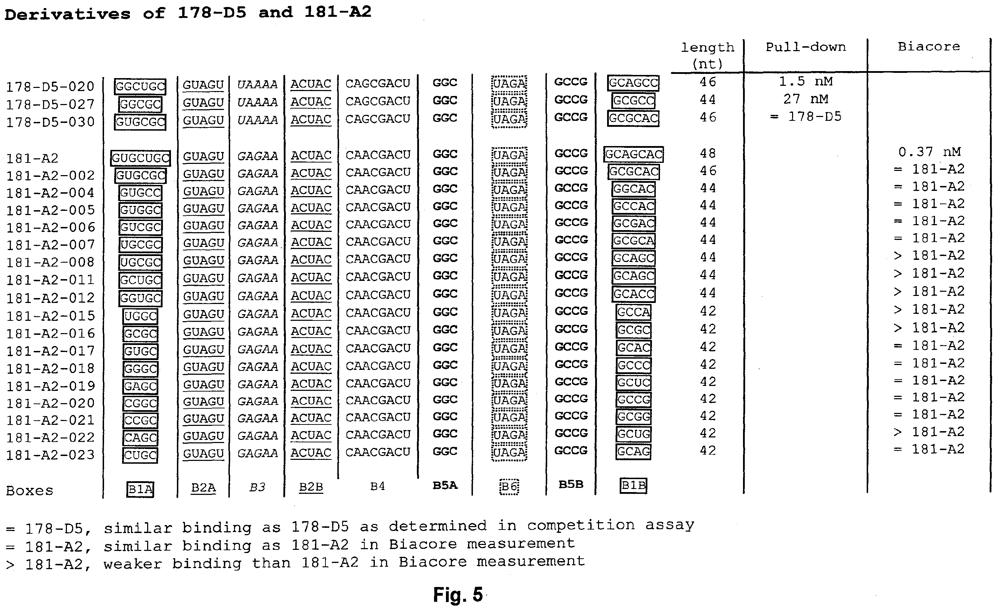

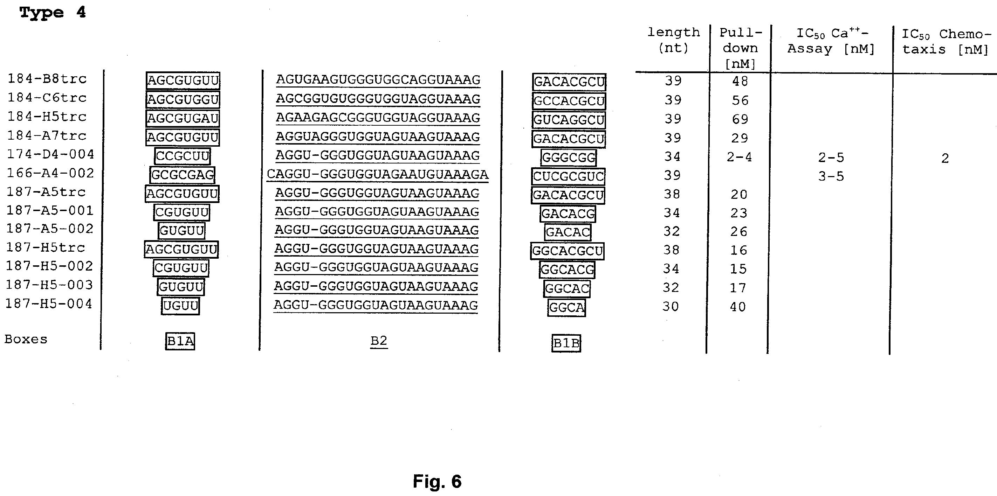

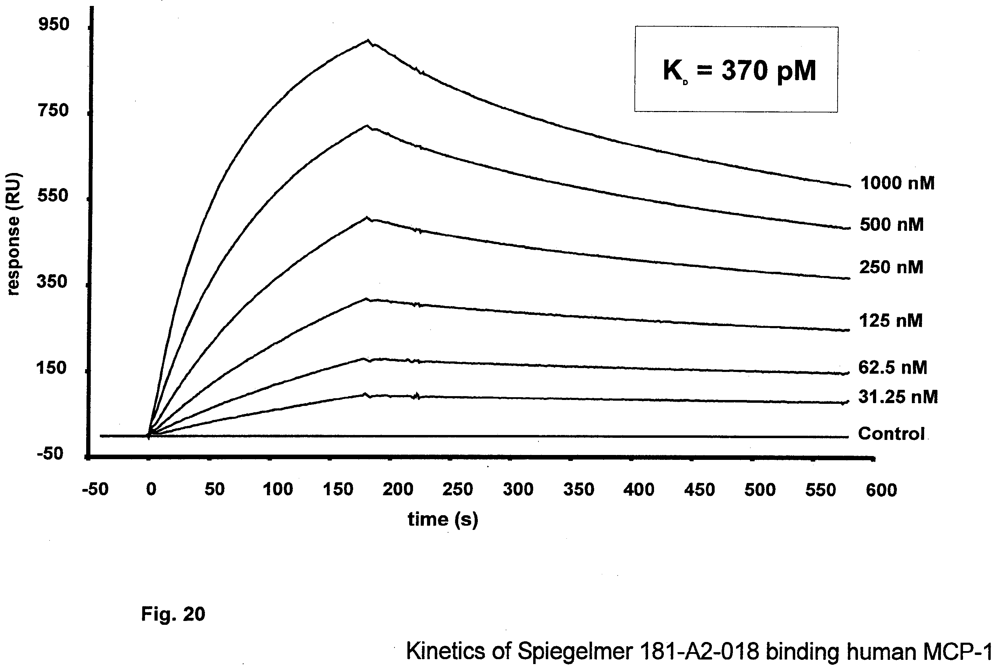

The nucleic acid molecules thus generated exhibit different sequence motifs, four main types are defined in Figs. 1 and 2 (Type 1A / 1B), Fig. 3 (Type 2), Figs. 4 and 5 (Type 3), and Fig. 6 (Type 4). Additional MCP-1 binding nucleic acids which cannot be related to each other and to the different sequence motifs described herein, are listed in Fig. 20. For definition of nucleotide sequence motifs, the IUPAC abbreviations for ambiguous nucleotides is used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches and boxes, respectively, is indicated in the 5' → 3' direction.

### Type 1A MCP-1 binding nucleic acids (Fig. 1)

As depicted in Fig. 1 all sequences of MCP-1 binding nucleic acids of Type 1A comprise several sequences stretches or boxes whereby boxes B1A and B1B are the 5'- and 3' terminal stretches that can hybridize with each other. However, such hybridization is not necessarily given in the molecule as actually present under physiological conditions. Boxes B2, *B3,* B4, B5 and box **B6** are flanked by box B1A and box B1B.

The nucleic acids were characterized on the aptamer level using direct and competitive pull-down assays with biotinylated human D-MCP-1 in order to rank them with respect to their binding behaviour (Example 6). Selected sequences were synthesized as Spiegelmer (Example 5) and were tested using the natural configuration of MCP-1 (L-MCP) in an *in vitro* cell culture Ca⁺⁺-release assay (Example 7).

The sequences of the defined boxes may be different between the MCP-1 binding nucleic acids of Type 1A which influences the binding affinity to MCP-1. Based on binding analysis of the different MCP-1 binding nucleic acids summarized as ***Type 1A MCP-1 binding nucleic acids,*** the boxes B1A, B2, *B3,* B4, B5, **B6** and B1B and their nucleotide sequences as described in the following are individually and more preferably in their entirety essential for binding to MCP-1:
- boxes B1A and B1B are the 5'- and 3' terminal stretches can hybridize with each other; where B1A is AGCRUG, preferably AGCGUG; and where B1B is CRYGCU, preferably CACGCU;
- box B2, which is CCCGGW, preferably CCCGGU;
- box B3, which is *GUR,* preferably *GUG;*
- box B4, which is RYA, preferably GUA;
- box B5, which is GGGGGRCGCGAYC, preferably GGGGGGCGCGACC,
- box **B6,** which is **UGCAAUAAUG** or **URYAWUUG,** preferably **UACAUUUG;**

As depicted in Fig. 1, the nucleic acid molecule referred to as 176-E10trc has the best binding affinity to MCP-1 (as aptamer in the pull-assay with a K_{D} of 5 nM as well as as Spiegelmer with an IC₅₀ of 4 - 5 nM in *in vitro* cell culture Ca⁺⁺-release assay) and therefore may constitute the optimal sequence and the optimal combination of sequence elements B1A, B2, *B3,* B4, B5, **B6** and B1B.

### Type 1B MCP-1 binding nucleic acids (Fig. 2)

As depicted in Fig. 2, all sequences of Type 1B comprise several sequences stretches or boxes whereby boxes B1A and B1B are the 5'- and 3' terminal stretches that can hybridize with each other and boxes B2, *B3,* B4, B5 and box **B6** are flanked by box B1A and box . However, such hybridization is not necessarily given in the molecule as actually present under physiological conditions.

The nucleic acids were characterized on the aptamer level using direct and competitive pull-down assays with biotinylated human D-MCP-1 in order to rank them with respect to their binding behavior (Example 3). Selected sequences were synthesized as Spiegelmer (Example 5) and were tested using the natural configuration of MCP-1 (L-MCP) in an *in vitro* cell culture Ca⁺⁺-release assay (Example 7).

The sequences of the defined boxes may be different between the MCP-1 binding nucleic acids of Type 1B which influences the binding affinity to MCP-1. Based on binding analysis of the different MCP-1 binding nucleic acids summarized as ***Type 1B MCP-1 binding nucleic acids,*** the boxes B1A, B2, *B3,* B4, B5, **B6** and B1B and their nucleotide sequences as described in the following are individually and more preferably in their entirety essential for binding to MCP-1:
- boxes B1A and B1B that can hybridize with each other; where B1A is AGYRUG, preferably AGCGUG; and where B1B is CAYRCU, preferably CACGCU;
- box B2, which is CCAGCU or CCAGY, preferably CCAGU;
- box *B3*, which is *GUG;*
- box B4, which is AUG;
- box B5, which is GGGGGGCGCGACC;
- box B6, which is CAUUUUA or CAUUUA, preferably CAUUUUA;

As depicted in Fig. 2, the nucleic acid referred to as 176-C9trc has the best binding affinity to MCP-1 (as aptamer in the pull-down assay with a K_{D} of 5 nM as well as as Spiegelmer with an IC₅₀ of 4 - 5 nM in *in vitro* cell culture Ca⁺⁺-release assay) and therefore may constitute the optimal sequence and the optimal combination of sequence elements B1A, B2, *B3*, B4, B5, **B6** and B1B.

### Type 2 MCP-1 binding nucleic acids (Fig. 3)

As depicted in Fig. 3, all sequences of Type 2 comprise several sequences stretches or boxes whereby boxes B1A and B1B are the 5'- and 3' terminal stretches that can hybridize with each other and box B2 is the central sequence element. However, such hybridization is not necessarily given in the molecule as actually present under physiological conditions.

The nucleic acids were characterized on the aptamer level using direct and competitive pull-down assays with biotinylated human D-MCP-1 in order to rank them with respect to their binding behaviour (Example 3). Selected sequences were synthesized as Spiegelmer (Example 5) and were tested using the natural configuration of MCP-1 (L-MCP) in *in vitro* cell culture Ca⁺⁺-release (Example 7) or *in vitro* chemotaxis assays (Example 8).

The sequences of the defined boxes may be different between the MCP-1 binding nucleic acids of Type 3 which influences the binding affinity to MCP-1. Based on binding analysis of the different MCP-1 binding nucleic acids summarized as ***Type 2 MCP-1 binding nucleic acids,*** the boxes B1A, B2, and B1B and their nucleotide sequences as described in the following are individually and more preferably in their entirety essential for binding to MCP-1:
- boxes B1A and B1B, 5'- and 3' terminal stretches that can hybridize with each other; where B1A is ACGCA and B1B is UGCGU, of B1A is CGCA and B1B is UGCG, or B1A is GCA and B1B is UGCG or UGC; preferably B1A is GCA and B1B is UGCG;
- box B2, CSUCCCUCACCGGUGCAAGUGAAGCCGYGGCUC, preferably CGUCCCUCACCGGUGCAAGUGAAGCCGUGGCUC

As depicted in Fig. 6, the nucleic acid referred to as 180-D1-002 as well as the derivatives of 180-D1-002 like 180-D1-011, 180-D1-012, 180-D1-035, and 180-D1-036 (= NOX-E36) have the best binding affinity to MCP-1 as aptamer in the pull-down or competitive pull-down assay with an K_{D} of < 1 nM and therefore may constitute the optimal sequence and the optimal combination of sequence elements B1A, B2, and .

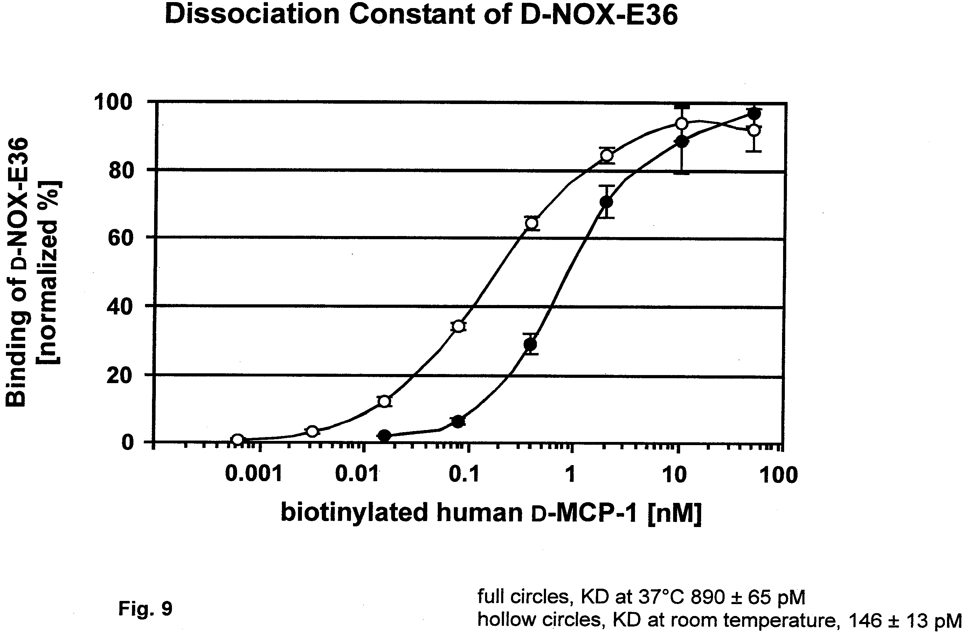

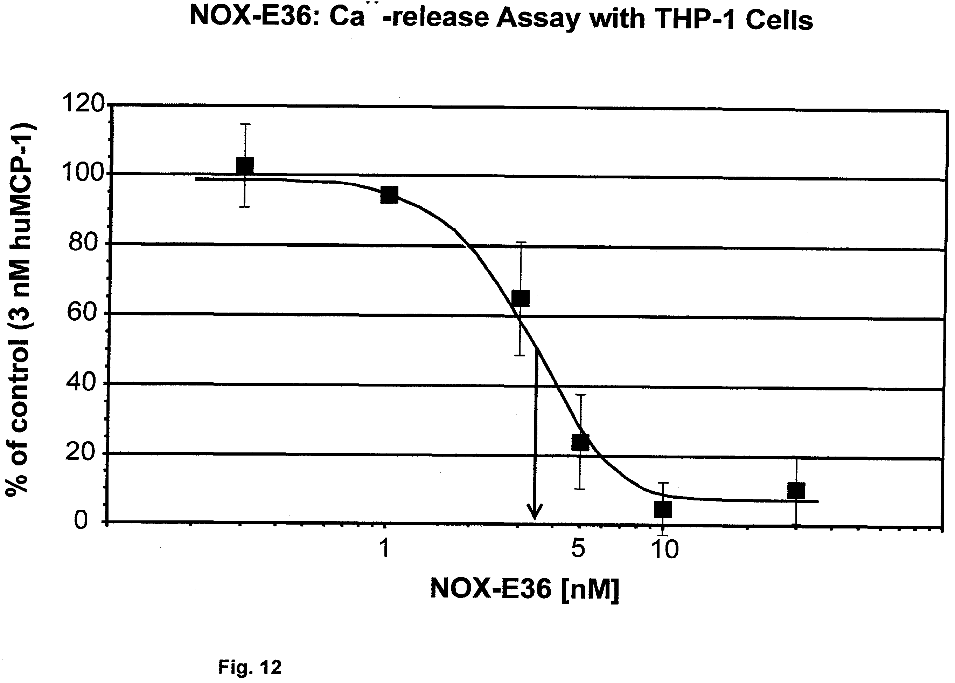

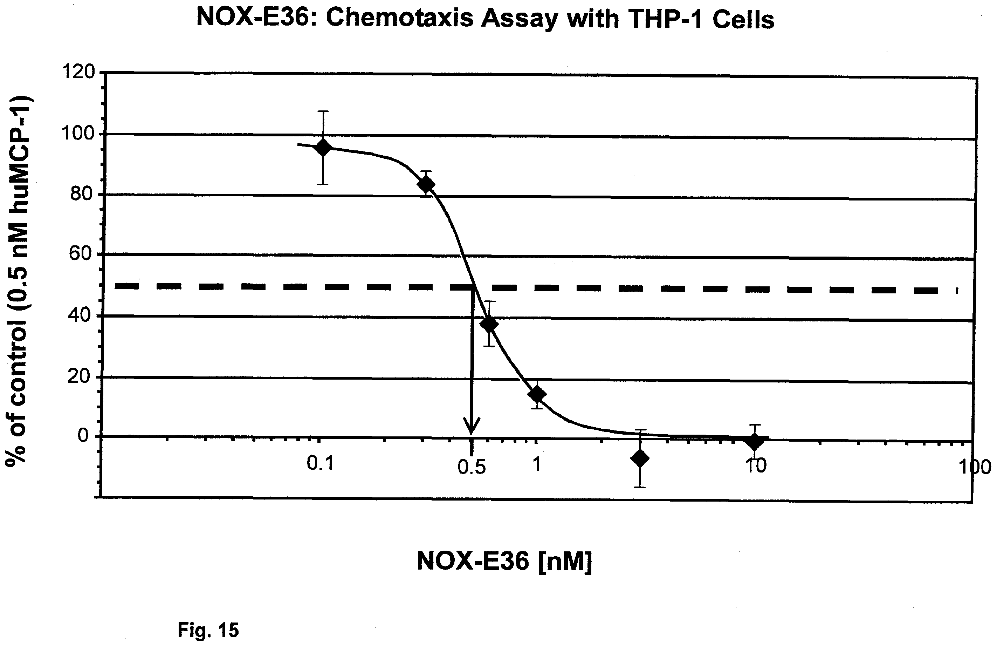

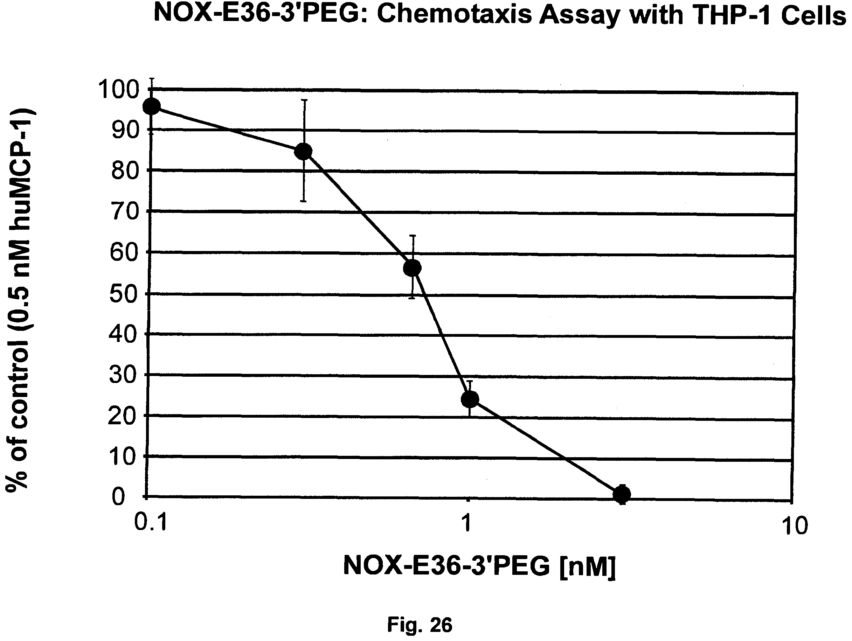

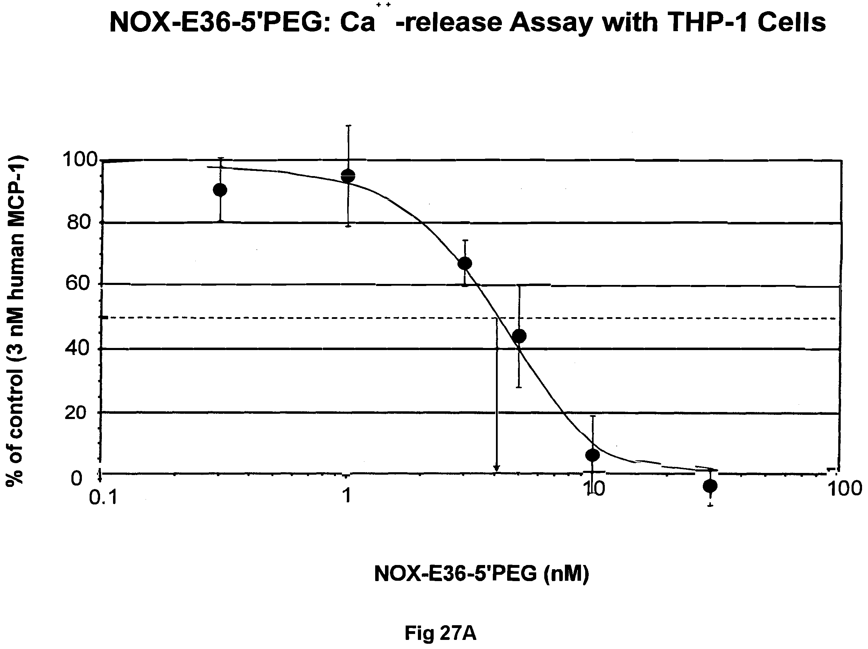

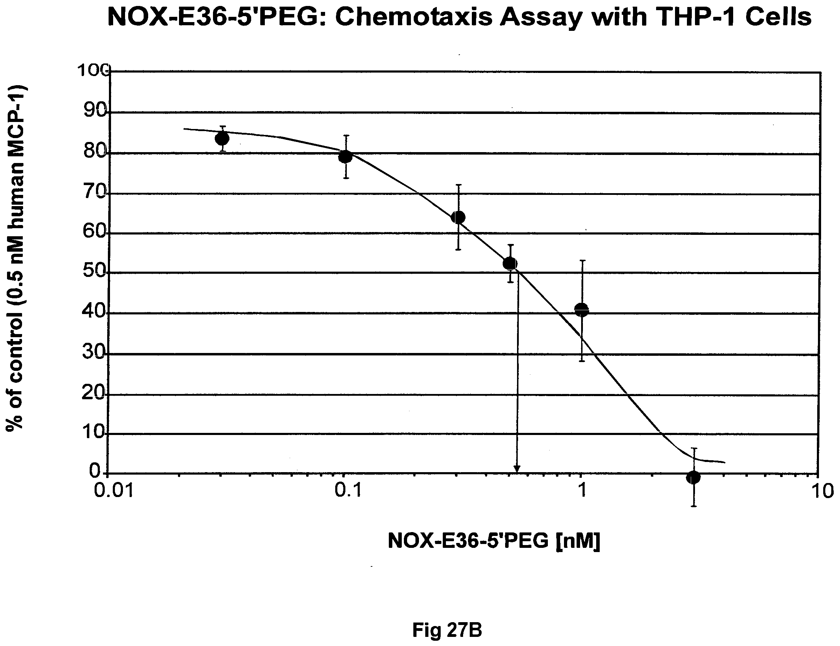

For nucleic acid molecule D-NOX-E36 (D-180-D1-036; SEQ.ID No. 159), a dissociation constant (K_{D}) of 890 ± 65 pM at room temperature (RT) and of 146 ± 13 pM at 37°C was determined (Example 6; Fig. 9). The respective Spiegelmer NOX-E36 (180-D1-036; SEQ.ID No. 37) exhibited an inhibitory concentration (IC₅₀) of 3 - 4 nM in an *in vitro* Ca⁺⁺-release assay (Example 7; Fig. 12) and of ca. 0.5 nM in an *in vitro* chemotaxis assay (Example 8; Fig. 15). For the PEGylated derivatives of NOX-E36, NOX-E36-3'PEG and NOX-E36-5'PEG, IC₅₀s of ca. 3 nM were determined in the Ca⁺⁺-release assay (Example 7, Fig. 38 and Fig. 40A) and < 1 nM in the chemotaxis assay (Example 8; Fig. 26 and Fig. 27B).

### Type 3 MCP-1 binding nucleic acids (Figs. 4+5)

As depicted in Figs. 4 and 5, all sequences of Type 3 comprise several sequence stretches or boxes whereby three pairs of boxes are characteristic for ***Type 3 MCP-1 binding nucleic acids.*** Both boxes B1A and B1B as well as boxes B2A and B2B as well as boxes **B5A** and **B5B** bear the ability to hybridize with each other. However, such hybridization is not necessarily given in the molecule as actually present under physiological conditions. Between these potentially hybridized sequence elements, non-hybridizing nucleotides are located, defined as box B3, box B4 and box B6.

The nucleic acids were characterized on the aptamer level using direct and competitive pull-down assays with biotinylated human D-MCP-1 in order to rank them with respect to their binding behavior (Example 6). Selected sequences were synthesized as Spiegelmer (Example 7) and were tested using the natural configuration of MCP-1 (L-MCP) in *in vitro* chemotaxis assays (Example 8) or via Biacore measurements (Example 9).

The sequences of the defined boxes may be different between the MCP-1 binding nucleic acids of Type 3 which influences the binding affinity to MCP-1. Based on binding analysis of the different MCP-1 binding nucleic acids summarized as ***Type 3 MCP-1 binding nucleic** acids,* the boxes B1A, B2A, *B3,* B2B, B4, **B5A,** B6, **B5B,** B1B and their nucleotide sequences as described in the following are individually and more preferably in their entirety essential for binding to MCP-1:
- boxes B1A and B1B, 5'- and 3' terminal stretches that can hybridize with each other; where B1A is GURCUGC and B1B is GCAGCAC; preferably B1A is GUGCUGC and B1B is GCAGCAC; or B1A is GKSYGC and B1B is |GCRSMC|; preferably B1A is GUGCGC and B1B is GCGCAC; or B1A is KBBSC and B1B is GSVVM; preferably B1A is KKSSC and B1B is GSSMM; or B1A is BNGC and B1B is GCNV; preferably B1A is SNGC and B1B is GCNS; most preferably B1A is GGGC and B1B is GCCC;
- boxes B2A and B2B, stretches that can hybridize with each other; where B2A is GKMGU and B2B is ACKMC; preferably B2A is GUAGU and B2B is ACUAC;
- box B3, which is *KRRAR,* preferably *UAAAA* or *GAGAA;*
- box B4, which is CURYGA or CUWAUGA or CWRMGACW or UGCCAGUG, preferably CAGCGACU or CAACGACU;
- **B5A** and **B5B,** stretches that can hybridize with each other; where **B5A** is **GGY** and **B5B** is **GCYR** whereas **GCY** can hybridize with the nucleotides of **B5A**; or **B5A** is **CWGC** and **B5B** is **GCWG;** preferably **B5A** is **GGC** and **B5B** is **GCCG;**
- box B6, which is: YAGA or CKAAU or CCUUUAU, preferably UAGA.

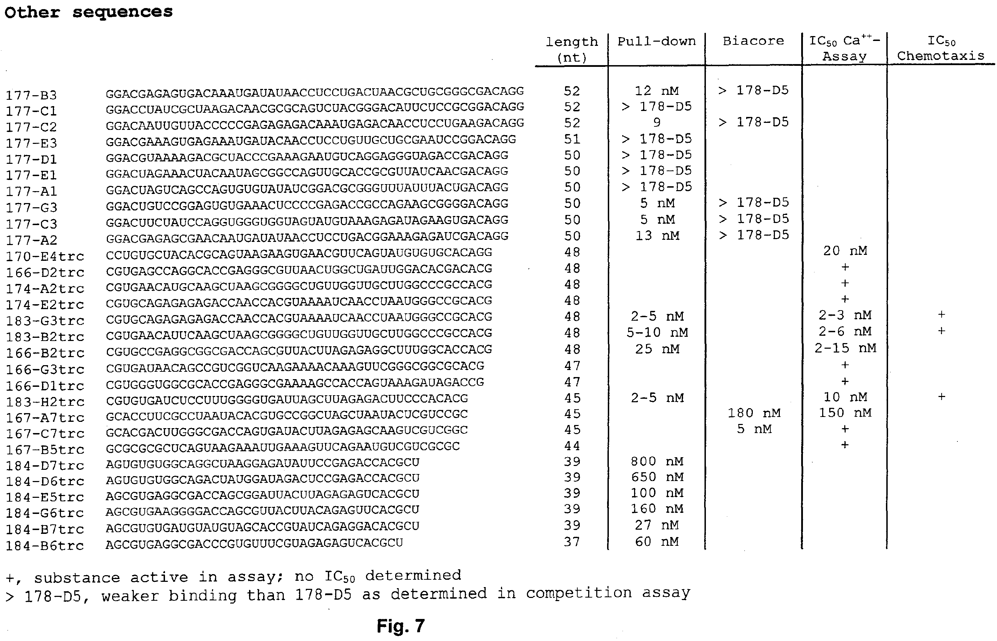

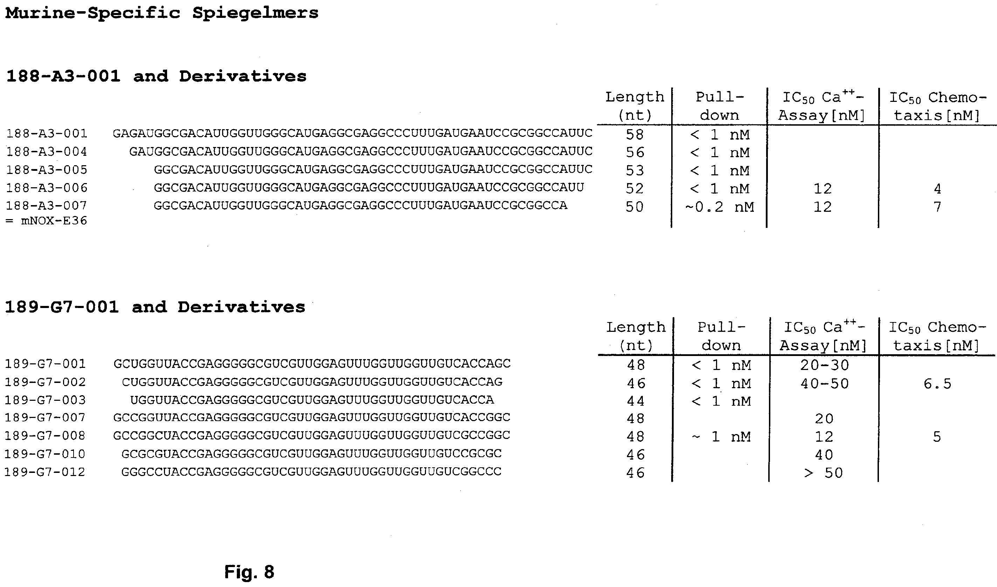

As depicted in Figs. 7 and 8, the nucleic acid referred to as 178-D5 and its derivative 178-D5-030 as well as 181-A2 with its derivatives 181-A2-002, 181-A2-004, 181-A2-005, 181-A2-006, 181-A2-007, 181-A2-017, 181-A2-018, 181-A2-019, 181-A2-020, 181-A2-021, and 181-A2-023 have the best binding affinity to MCP-1. 178-D5 and 178-D5-030 were evaluated as aptamers in direct or competitive pull-down assays (Example 6) with an K_{D} of approx. 500 pM. In the same experimental set-up, 181-A2 was determined with an K_{D} of approx. 100 pM. By Biacore analysis (Example 9), the K_{D} of 181-A2 and its derivatives towards MCP-1 was determined to be 200 - 300 pM. In Ca⁺⁺ release and chemotaxis assays with cultured cells (Example 7 and 8, respectively), for both 178-D5 and 181-A2, an IC₅₀ of approx. 500 pM was measured. Therefore, 178-D5 as well as 181-A2 and their derivatives may constitute the optimal sequence and the optimal combination of sequence elements B1A, B2A, *B3,* B2B, B4, **B5A,** B6, **B5B** and B1B.

### Type 4 MCP-1 binding nucleic acids (Fig. 9)

As depicted in Fig. 9, all sequences of Type 4 comprise several sequences, stretches or boxes whereby boxes B1A and B1B are the 5'- and 3' terminal stretches that can hybridize with each other and box B2 is the central sequence element.

The nucleic acids were characterized on the aptamer level using direct pull-down assays with biotinylated human D-MCP-1 in order to rank them with respect to their binding behavior (Example 6). Selected sequences were synthesized as Spiegelmer (Example 5) and were tested using the natural configuration of MCP-1 (L-MCP) in an *in vitro* cell culture Ca⁺⁺-release (Example 7) and/or chemotaxis assay (Example 8).

The sequences of the defined boxes may differ among the MCP-1 binding nucleic acids of Type 4 which influences the binding affinity to MCP-1. Based on binding analysis of the different MCP-1 binding nucleic acids summarized as ***Type 4 MCP-1 binding nucleic acids,*** the boxes , B2, and B1B and their nucleotide sequences as described in the following are individually and more preferably in their entirety essential for binding to MCP-1:
- boxes B1A and , 5'- and 3' terminal stretches that can hybridize with each other; where B1A is AGCGUGDU and B1B is GNCASGCU; or B1A is GCGCGAG and B1B is CUCGCGUC; or B1A is CSKSUU and B1B is GRSMSG; or B1A is GUGUU and B1B is GRCAC; or B1A is UGUU and B1B is GGCA; preferably B1A is CSKSUU and B1B is GRSMSG; mostly preferred B1A is CCGC and B1B is and
- box B2, which is AGNDRDGBKGGURGYARGUAAAG or
   AGGUGGGUGGUAGUAAGUAAAG or CAGGUGGGUGGUAGAAUGUAAAGA, preferably AGGUGGGUGGUAGUAAGUAAAG

As depicted in Fig. 6, the nucleic acid referred to as 174-D4-004 and 166-A4-002 have the best binding affinity to MCP-1 (as Spiegelmer with an IC₅₀ of 2 - 5 nM in *in vitro* cell culture Ca⁺⁺ release assay) and may, therefore, constitute the optimal sequence and the optimal combination of sequence elements B1A, B2, and .

Additionally, 29 other MCP-1 binding nucleic acids were identified which cannot be described by a combination of nucleotide sequence elements as has been shown for Types 1 - 4 of MCP-1 binding nucleic acids. These sequences are listed in Fig. 7.

It is to be understood that any of the sequences shown in Figs. 1 through 7 are nucleic acids suitable for use in the present invention, in particular the invention as defined in the claims, the aspects and any embodiment, including those truncated forms thereof but also including those extended forms thereof under the proviso, however, that the thus truncated and extended, respectively, nucleic acid molecules are still capable of binding to the target.

### Example 4: Nucleic acids that bind murine MCP-1

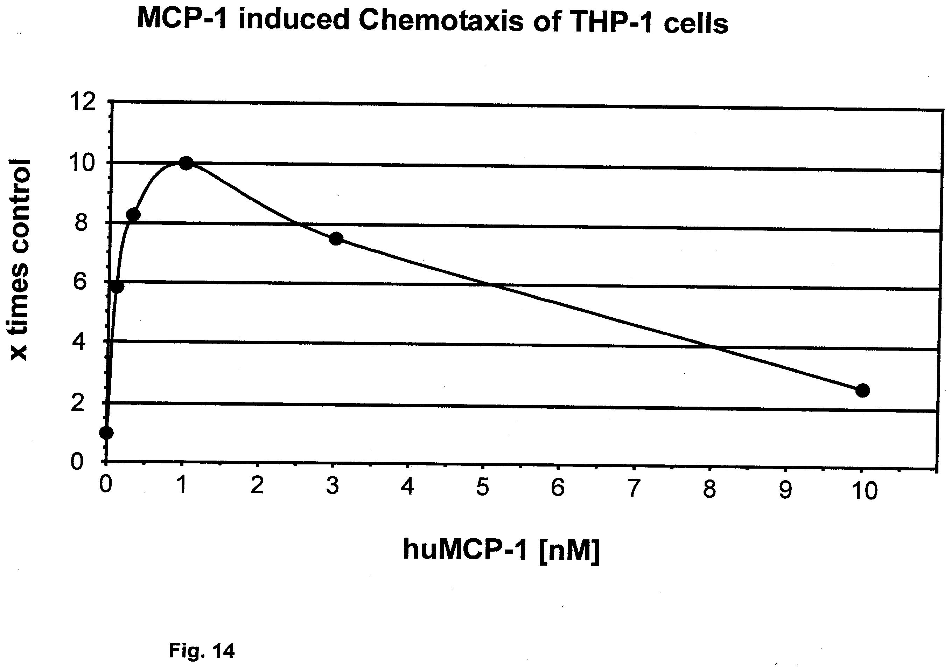

Using biotinylated murine D-MCP-1 as a target, several nucleic acid molecules binding thereto could be generated. The result of a sequence analysis of these nucleic acid molecules can be taken from Fig. 14.

The nucleic acids were characterized on the aptamer level using a pull-down assay using biotinylated murine D-MCP-1 in order to in order to rank them with respect to their binding behavior (Example 6). Selected sequences were synthesized as Spiegelmer (Example 5) and were tested using the natural configuration of MCP-1 (L-MCP) in an *in vitro* cell culture Ca⁺⁺-release (Example 7) and chemotaxis assay (Example 8).

As depicted in Fig. 14, D-188-A3-001 and D-189-G7-001 and their derivatives bind D-MCP-1 with sub-nanomolar K_{D} in the pull-down assay (Fig. 14).

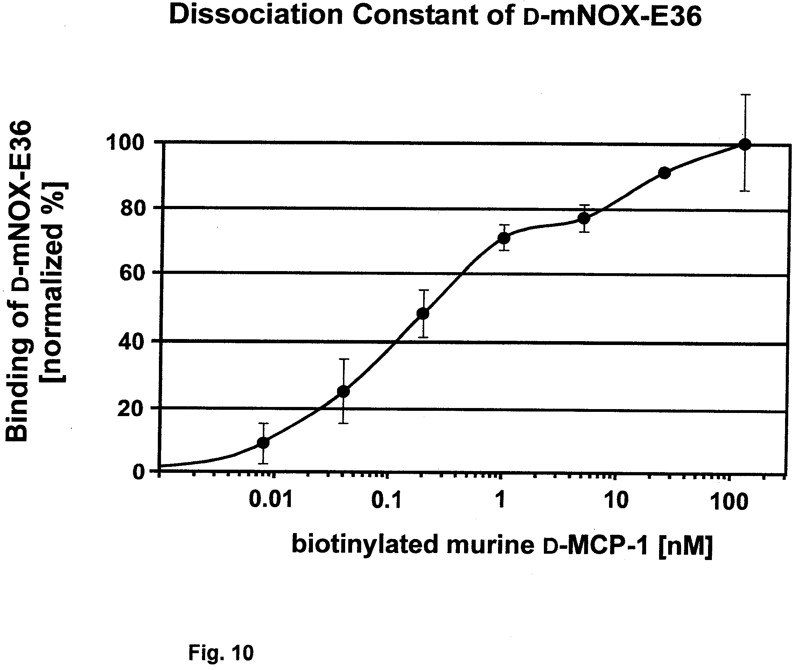

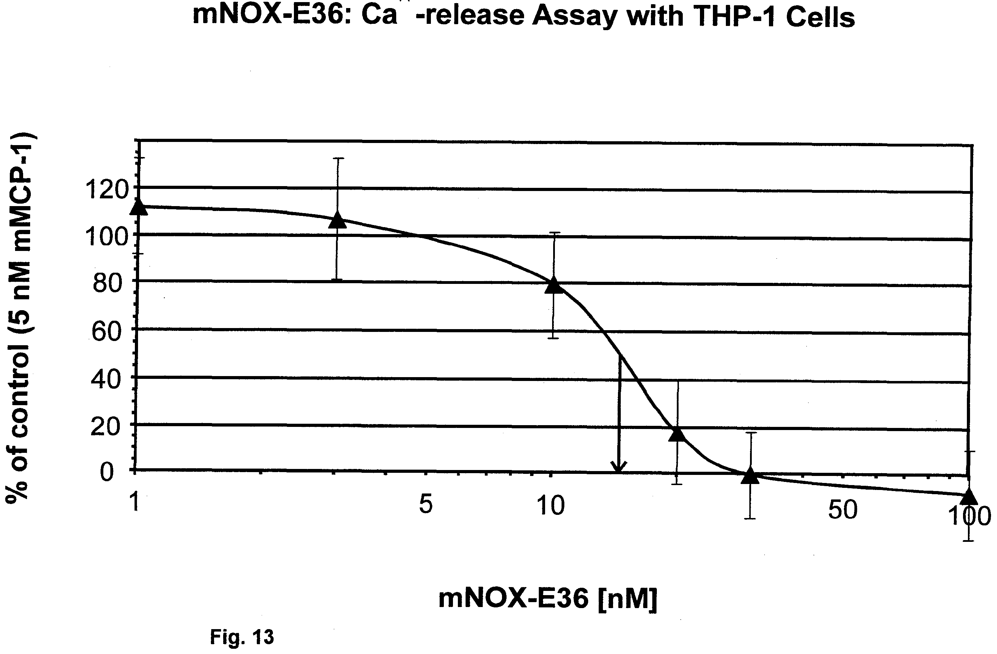

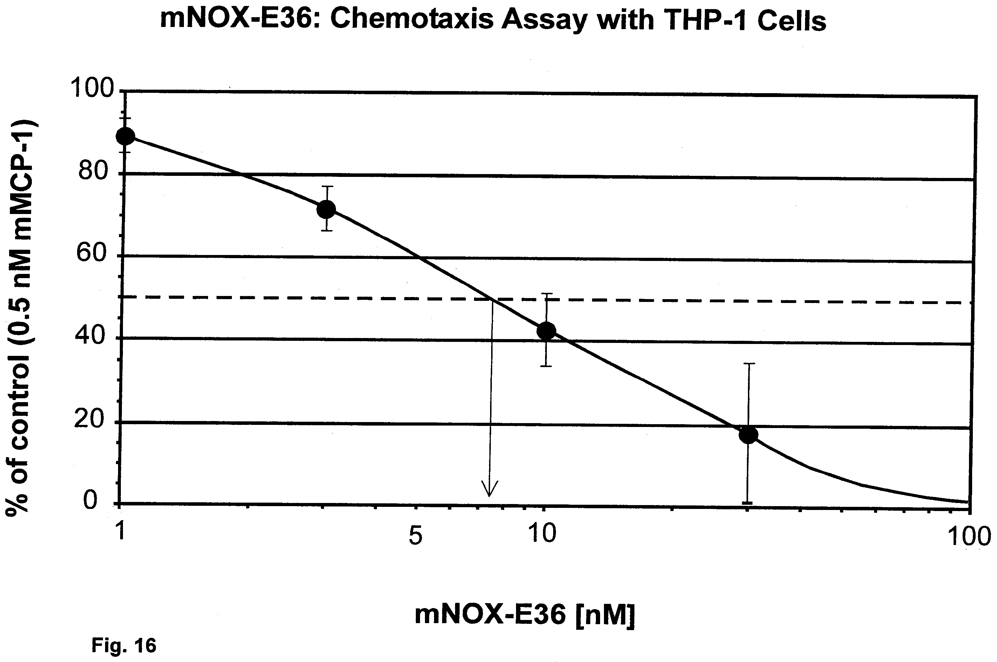

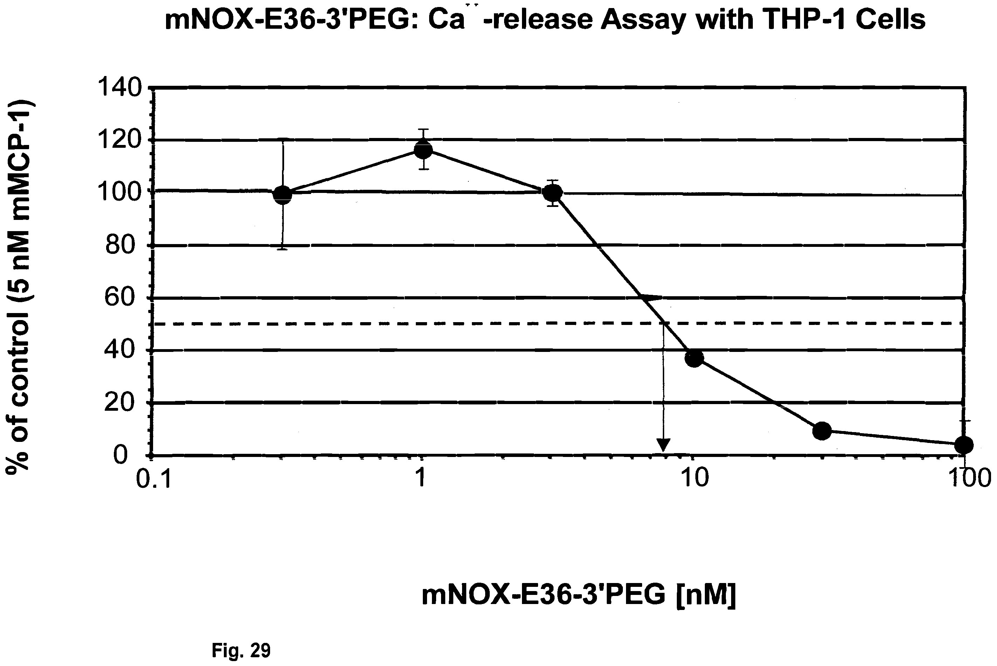

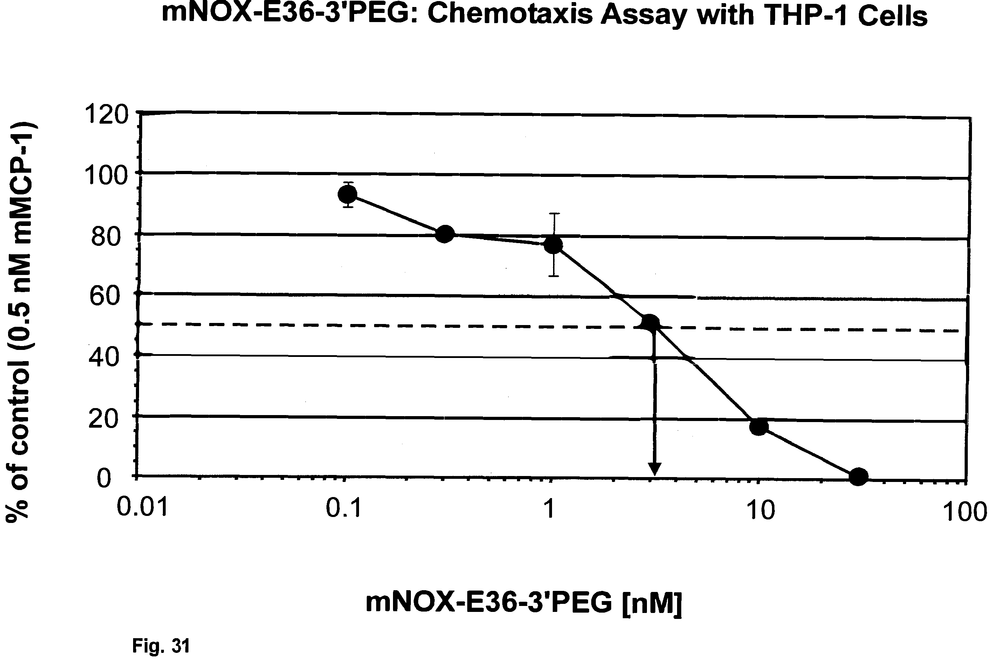

For D-mNOX-E36 (= D-188-A3-007; SEQ.ID No. 244), a dissociation constant (K_{D}) of 0.1 - 0.2 nM at 37°C was determined (Example 6; Fig. 10). The respective Spiegelmer mNOX-E36 (188-A3-007; SEQ.ID No. 122) exhibited an inhibitory concentration (IC₅₀) of approx. 12 nM in an *in vitro* Ca⁺⁺-release assay (Example 7; Fig. 13) and of approx. 7 nM in an *in vitro* chemotaxis assay (Example 8; Fig. 16). For the PEGylated derivative of mNOX-E36, mNOX-E36-3'PEG (SEQ.ID No. 254), IC₅₀'s of approx. 8 nM were determined in the Ca⁺⁺-release assay (Example 7, Fig. 29) and approx. 3 nM in the chemotaxis assay (Example 8; Fig. 31).

It is to be understood that any of the sequences shown in Fig. 14 are nucleic acids suitable for use in the present invention, in particular the invention as defined in the claims, the aspects and any embodiment, including those truncated forms thereof but also including those extended forms thereof under the proviso, however, that the thus truncated and extended, respectively, nucleic acid molecules are still capable of binding to the target.

### Example 5: Synthesis and derivatization of Aptamers and Spiegelmers

### Small scale synthesis

Aptamers and Spiegelmers were produced by solid-phase synthesis with an ABI 394 synthesizer (Applied Biosystems, Foster City, CA, USA) using 2'TBDMS RNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). rA(N-Bz)-, rC(Ac)-, rG(N-ibu)-, and rU- phosphoramidites in the D- and L-configuration were purchased from ChemGenes, Wilmington, MA. Aptamers and Spiegelmers were purified by gel electrophoresis.

### Large scale synthesis plus modification

Spiegelmer NOX-E36 was produced by solid-phase synthesis with an ÄktaPilot100 synthesizer (Amersham Biosciences; General Electric Healthcare, Freiburg) using 2'TBDMS RNA phosphoramidite chemistry (M.J. Damha, K.K. Ogilvie, Methods in Molecular Biology, Vol. 20 Protocols for oligonucleotides and analogs, ed. S. Agrawal, p. 81-114, Humana Press Inc. 1993). L-rA(N-Bz)-, L-rC(Ac)-, L-rG(N-ibu)-, and L-rU-phosphoramidites were purchased from ChemGenes, Wilmington, MA. The 5'-amino-modifier was purchased from American International Chemicals Inc. (Framingham, MA, USA). Synthesis of the unmodified Spiegelmer was started on L-riboG modified CPG pore size 1000 Å (Link Technology, Glasgow, UK); for the 3'-NH₂-modified Spiegelmer, 3'-Aminomodifier-CPG, 1000 Å (ChemGenes, Wilmington, MA) was used. For coupling (15 min per cycle), 0.3 M benzylthiotetrazole (CMS-Chemicals, Abingdon, UK) in acetonitrile, and 3.5 equivalents of the respective 0.1 M phosphoramidite solution in acetonitrile was used. An oxidation-capping cycle was used. Further standard solvents and reagents for oligonucleotide synthesis were purchased from Biosolve (Valkenswaard, NL). The Spiegelmer was synthesized DMT-ON; after deprotection, it was purified via preparative RP-HPLC (Wincott F. et al. (1995) Nucleic Acids Res 23:2677) using Source15RPC medium (Amersham). The 5'DMT-group was removed with 80% acetic acid (30 min at RT). Subsequently, aqueous 2 M NaOAc solution was added and the Spiegelmer was desalted by tangential-flow filtration using a 5 K regenerated cellulose membrane (Millipore, Bedford, MA).

### PEGylation of NOX-E36

In order to prolong the Spiegelmer's plasma residence time in vivo, Spiegelmer NOX-E36 was covalently coupled to a 40 kDa polyethylene glycol (PEG) moiety at the 3'-end or 5'-end.

### 3'-PEGylation of NOX-E36

For PEGylation (for technical details of the method for PEGylation see European patent application EP 1 306 382), the purified 3'-amino modified Spiegelmer was dissolved in a mixture of H₂O (2.5 ml), DMF (5 ml), and buffer A (5 ml; prepared by mixing citric acid • H₂O [7 g], boric acid [3.54 g], phosphoric acid [2.26 ml], and 1 M NaOH [343 ml] and adding H2O to a final volume of 11; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in four portions of 0.6 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), 4 ml buffer A, and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### 5'-PEGylation of NOX-E36

For PEGylation (for technical details of the method for PEGylation see European patent application EP 1 306 382), the purified 5'-amino modified Spiegelmer was dissolved in a mixture of H₂O (2.5 ml), DMF (5 ml), and buffer A (5 ml; prepared by mixing citric acid • H₂O [7 g], boric acid [3.54 g], phosphoric acid [2.26 ml], and 1 M NaOH [343 ml] and adding water to a final volume of 1 l; pH = 8.4 was adjusted with 1 M HCl).

The pH of the Spiegelmer solution was brought to 8.4 with 1 M NaOH. Then, 40 kDa PEG-NHS ester (Nektar Therapeutics, Huntsville, AL) was added at 37°C every 30 min in six portions of 0.25 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), and 4 ml buffer B (0.1 M triethylammonium acetate in H₂O) and heated to 95°C for 15 min. The PEGylated Spiegelmer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated Spiegelmer at 10 - 15% buffer C. Product fractions with a purity of >95% (as assessed by HPLC) were combined and mixed with 40 ml 3 M NaOAC. The PEGylated Spiegelmer was desalted by tangential-flow filtration (5 K regenerated cellulose membrane, Millipore, Bedford MA).

### Example 6: Determination of Binding Constants (Pull-Down Assay)

### Direct pull-down assay

The affinity of aptamers to D-MCP-1 was measured in a pull-down assay format at 20 or 37°C, respectively. Aptamers were 5'-phosphate labelled by T4 polynucleotide kinase (Invitrogen, Karlsruhe, Germany) using [γ-³²P]-labelled ATP (Hartmann Analytic, Braunschweig, Germany). The specific radioactivity of labeled aptamers was 200,000 - 800,000 cpm/pmol. Aptamers were incubated after de- and renaturation at 20 pM concentration at 37°C in selection buffer (20 mM Tris-HCl pH 7.4; 137 mM NaCl; 5 mM KCl; 1 mM MgCl₂; 1 mM CaCl₂; 0.1% [w/vol] Tween-20) together with varying amounts of biotinylated D-MCP-1 for 4 - 12 hours in order to reach equilibrium at low concentrations. Selection buffer was supplemented with 10 µg/ml human serum albumin (Sigma-Aldrich, Steinheim, Germany), and 10 µg/ml yeast RNA (Ambion, Austin, USA) in order to prevent adsorption of binding partners with surfaces of used plasticware or the immobilization matrix. The concentration range of biotinylated D-MCP-1 was set from 8 pM to 100 nM; total reaction volume was 1 ml. Peptide and peptide-aptamer complexes were immobilized on 1.5 µl Streptavidin Ultralink Plus particles (Pierce Biotechnology, Rockford, USA) which had been preequilibrated with selection buffer and resuspended in a total volume of 6 µl. Particles were kept in suspension for 30 min at the respective temperature in a thermomixer. Immobilized radioactivity was quantitated in a scintillation counter after detaching the supernatant and appropriate washing. The percentage of binding was plotted against the concentration of biotinylated D-MCP-1 and dissociation constants were obtained by using software algorithms (GRAFIT; Erithacus Software; Surrey U.K.) assuming a 1:1 stoichiometry.

### Competitive pull-down assay

In order to compare different D-MCP-1 binding aptamers, a competitive ranking assay was performed. For this purpose, the most affine aptamer available was radioactively labelled (see above) and served as reference. After de- and renaturation it was incubated at 37°C with biotinylated D-MCP-1 in 1 ml selection buffer at conditions that resulted in around 5 - 10 % binding to the peptide after immobilization and washing on NeutrAvidin agarose or Streptavidin Ultralink Plus (both from Pierce) without competition. An excess of de- and renatured non-labelled D-RNA aptamer variants was added to different concentrations (e.g. 2, 10, and 50 nM) with the labelled reference aptamer to parallel binding reactions. The aptamers to be tested competed with the reference aptamer for target binding, thus decreasing the binding signal in dependence of their binding characteristics. The aptamer that was found most active in this assay could then serve as a new reference for comparative analysis of further aptamer variants.

### Example 7: Determination of Inhibitory Concentration in a Ca⁺⁺-Release Assay

THP-1-cells (DSMZ, Braunschweig) were cultivated overnight at a cell density of 0.3 x 10⁶/ml at 37°C and 5% CO₂ in RPMI 1640 medium with GlutaMAX (Invitrogen) which contained in addition 10% fetal calf serum, 50 units/ml penicillin, 50 µg/ml streptomycin and 50 µM β-mercaptoethanol.

The Spiegelmers were incubated together with recombinant human MCP-1 (Bachem) in Hanks balanced salt solution (HBSS), containing 1 mg/ml bovine serum albumin, 5 mM probenecid and 20 mM HEPES (HBSS+) for 15 to 60 min at 37°C in a 0.2 ml low profile 96-tube plate ("stimulation solution").

For loading with the calcium indicator dye, cells were centrifuged at 300 x g for 5 min, resuspended in 4 ml indicator dye solution (10 µM fluo-4 [Molecular Probes], 0.08% pluronic 127 [Molecular Probes] in HBSS+) and incubated for 60 min at 37°C. Thereafter, 11 ml HBSS+ were added, and the cells were centrifuged as above, washed once with 15 ml HBSS+ and then resuspended in HBSS+ to give a cell density of 1.1 x 10⁶/ml. 90 µl of this cell suspension were added to each well of a black 96-well plate.

Measurement of fluorescence signals was done at an excitation wavelength of 485 nm and an emission wavelength of 520 nm in a Fluostar Optima multidetection plate reader (BMG). For parallel measurement of several samples, wells of one (perpendicular) row of a 96-well plate were recorded together. First three readings with a time lag of 4 sec were done for determination of the base line. Then the recording was interrupted, and the plate was moved from the instrument. Using a multi-channel pipette, 10 µl of the stimulation solution was added to the wells, then the plate was moved into the instrument again and the measurement was continued. In total, 20 recordings with time intervals of 4 seconds were performed.

For each well the difference between maximal fluorescence and base line value was determined and plotted against MCP-1 concentration or, in the experiments on the inhibition of calcium release by Spiegelmers, against concentration of Spiegelmer.

### Determination of half-maximal effective concentration (EC₅₀) for human MCP-1

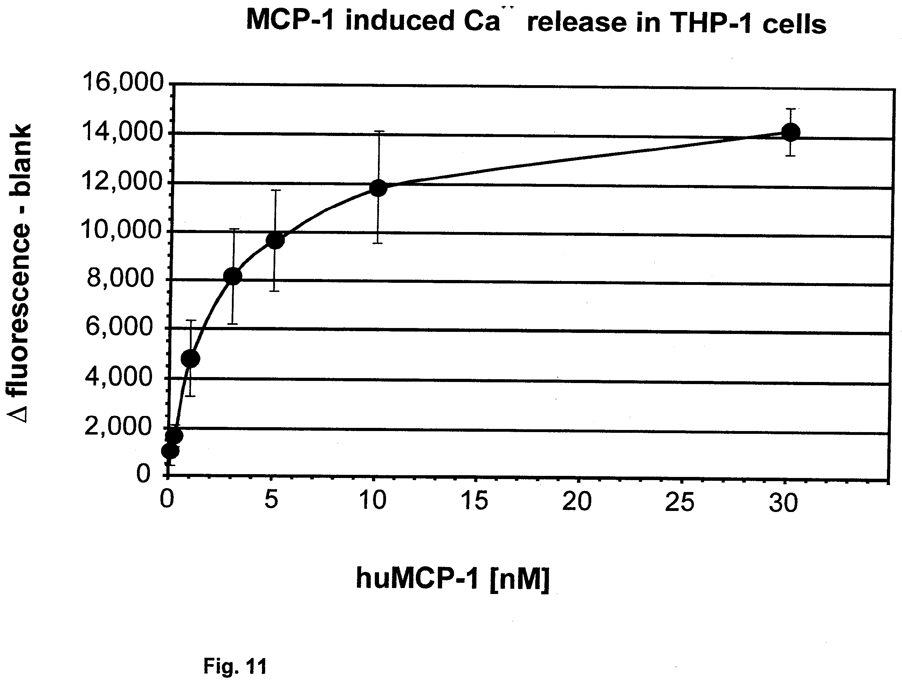

After stimulation of THP-1 cells with various hMCP-1 concentrations and plotting the difference between the maximal and the baseline signals, a dose-response curve for human MCP-1 was obtained, indicating a half effective concentration (EC₅₀) of about 2 - 4 nM (Fig. 11). This concentration was used for the further experiments on inhibition of Ca⁺⁺-release by Spiegelmers.

### Determination of half-maximal effective concentration (EC₅₀) for murine MCP-1

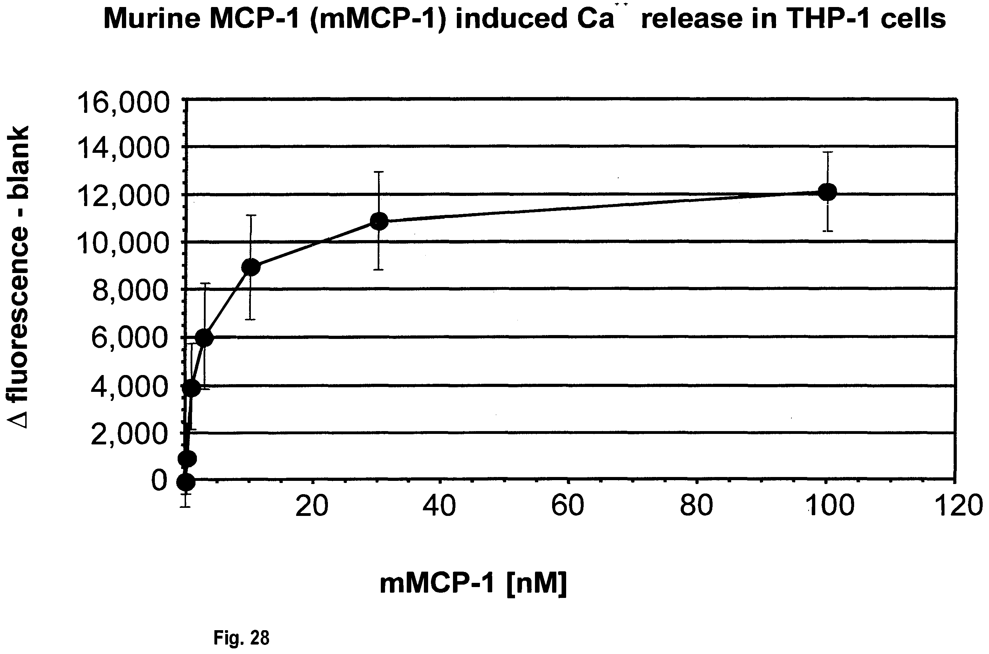

After stimulation of THP-1 cells with various mMCP-1 concentrations and plotting the difference between the maximal and the baseline signals, a dose-response curve for murine MCP-1 was obtained, indicating a half effective concentration (EC₅₀) of about 5 nM (Fig. 28). This concentration was used for the further experiments on inhibition of Ca⁺⁺-release by Spiegelmers.

### Example 8: Determination of Inhibitory Concentration in a Chemotaxis Assay

THP-1 cells grown as described above were centrifuged, washed once in HBH (HBSS, containing 1 mg/ml bovine serum albumin and 20 mM HEPES) and resuspended at 3 x 10⁶ cells/ml. 100 µl of this suspension were added to Transwell inserts with 5 µm pores (Corning, #3421). In the lower compartments MCP-1 was preincubated together with Spiegelmers in various concentrations in 600 µl HBH at 37°C for 20 to 30 min prior to addition of cells. Cells were allowed to migrate at 37°C for 3 hours. Thereafter the inserts were removed and 60 µl of 440 µM resazurin (Sigma) in phosphate buffered saline was added to the lower compartments. After incubation at 37°C for 2.5 hours, fluorescence was measured at an excitation wavelength of 544 nm and an emission wavelength of 590 nm in a Fluostar Optima multidetection plate reader (BMG).

### Determination of half-maximal effective concentration (EC₅₀) for human MCP-1

After 3 hours migration of THP-1 cells towards various human MCP-1 concentrations, a dose-response curve for human MCP-1 was obtained, indicating a maximal effective concentration of about 1 nM and reduced activation at higher concentrations (Fig. 14). For the further experiments on inhibition of chemotaxis by Spiegelmers a MCP-1 concentration of 0.5 nM was used.

### Determination of half-maximal effective concentration (EC₅₀) for murine MCP-1

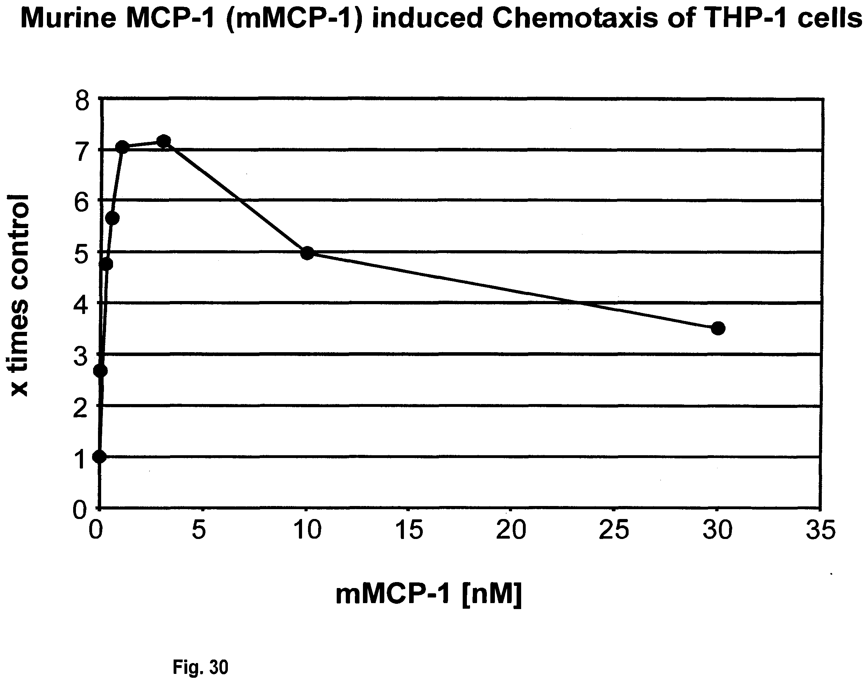

After 3 hours migration of THP-1 cells towards various murine MCP-1 concentrations, a dose-response curve for murine MCP-1 was obtained, indicating a maximal effective concentration of about 1 - 3 nM and reduced activation at higher concentrations (Fig. 30). For the further experiments on inhibition of chemotaxis by Spiegelmers a murine MCP-1 concentration of 0.5 nM was used.

### Example 9: Binding Analysis by Surface Plasmon Resonance Measurement

### 9.1 Specificity assessment of NOX-E36, 181-A2-018 and mNOX-E36

The Biacore 2000 instrument (Biacore AB, Uppsala, Sweden) was used to analyze binding of nucleic acids to human MCP-1 and related proteins. When coupling was to be achieved via amine groups, the proteins were dialyzed against water for 1 - 2 h (Millipore VSWP mixed cellulose esters; pore size, 0.025 µM) to remove interfering amines. PioneerF1 or CM4 sensor chips (Biacore AB) were activated before protein coupling by a 35-µl injection of a 1:1 dilution of 0.4 M NHS and 0.1 M EDC at a flow of 5 µl/min. Chemokine was then injected in concentrations of 0.1 - 1.5 µg/ml at a flow of 2 µl/min until the instrument's response was in the range of 1000 - 2000 RU (relative units). Unreacted NHS esters were deactivated by injection of 35 µl ethanolamine hydrochloride solution (pH 8.5) at a flow of 5 µl/min. The sensor chip was primed twice with binding buffer and equilibrated at 10 µl/min for 1 - 2 hours until the baseline appeared stable. For all proteins, kinetic parameters and dissociation constants were evaluated by a series of Spiegelmer injections at concentrations of 1000, 500, 250, 125, 62.5, 31.25, and 0 nM in selection buffer (Tris-HCl, 20 mM; NaCl, 137 mM; KCl, 5 mM; CaCl₂, 1 mM; MgCl₂, 1 mM; Tween20, 0.1% [w/v]; pH 7.4). In all experiments, the analysis was performed at 37°C using the Kinject command defining an association time of 180 and a dissociation time of 360 seconds at a flow of 10 µl/min. Data analysis and calculation of dissociation constants (K_{D}) was done with the BIAevaluation 3.0 software (BIACORE AB, Uppsala, Sweden) using the Langmuir 1:1 stochiometric fitting algorithm.

### 9.1.1 NOX-E36 and 181-A2-018 (human-MCP-1 specific nucleic acids)

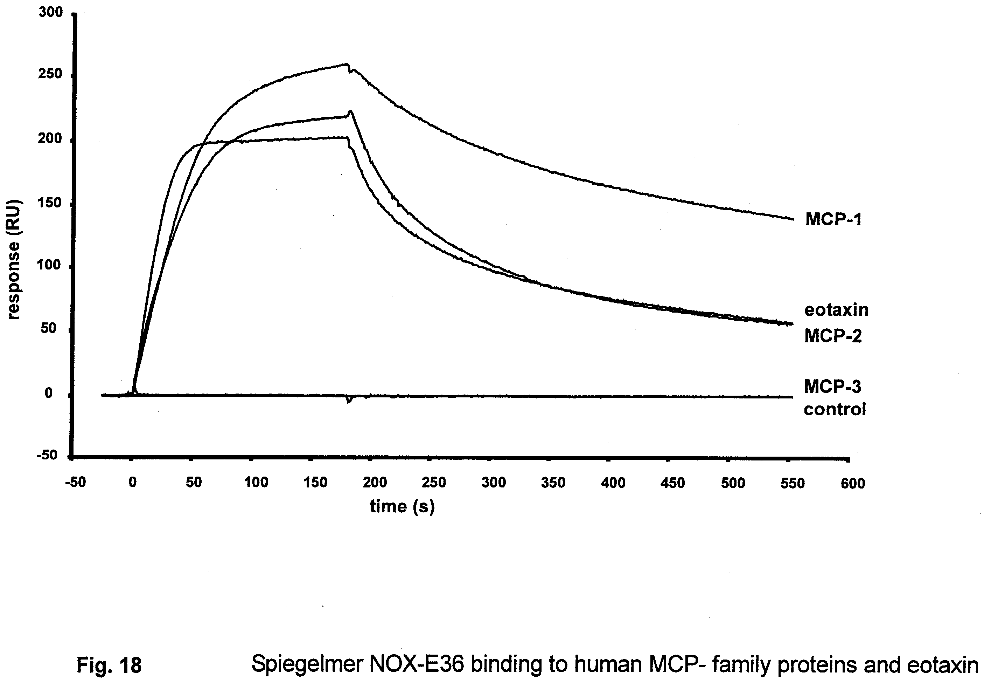

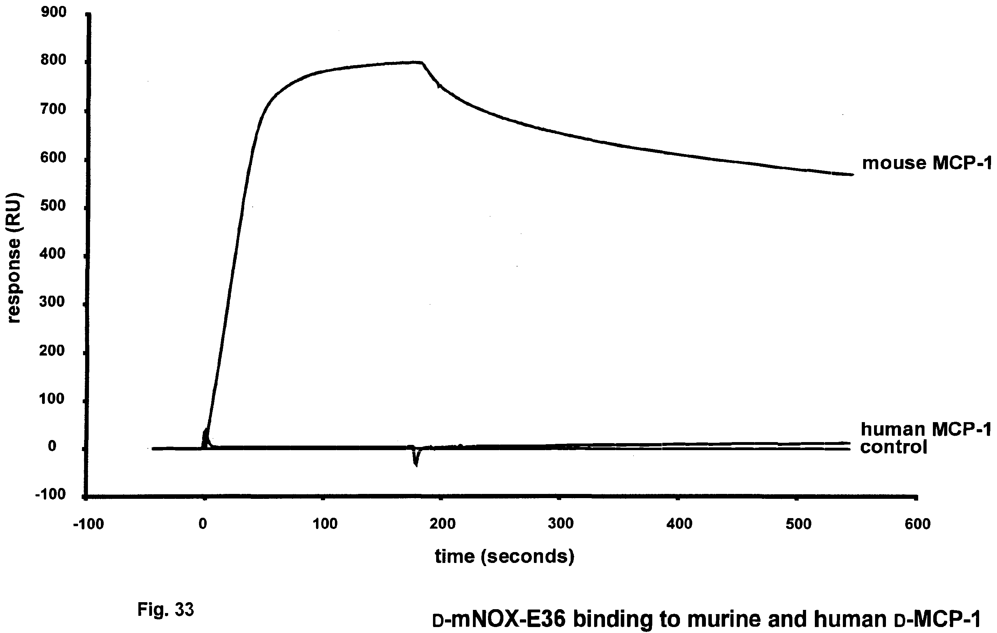

Only for human MCP-1 all sensorgrams are depicted (Figs 30 and 33, respectively); for the other proteins, only the sensorgram obtained with 125 nM Spiegelmer concentration is shown for sake of clarity (Figs. 18/19 and 21/22).

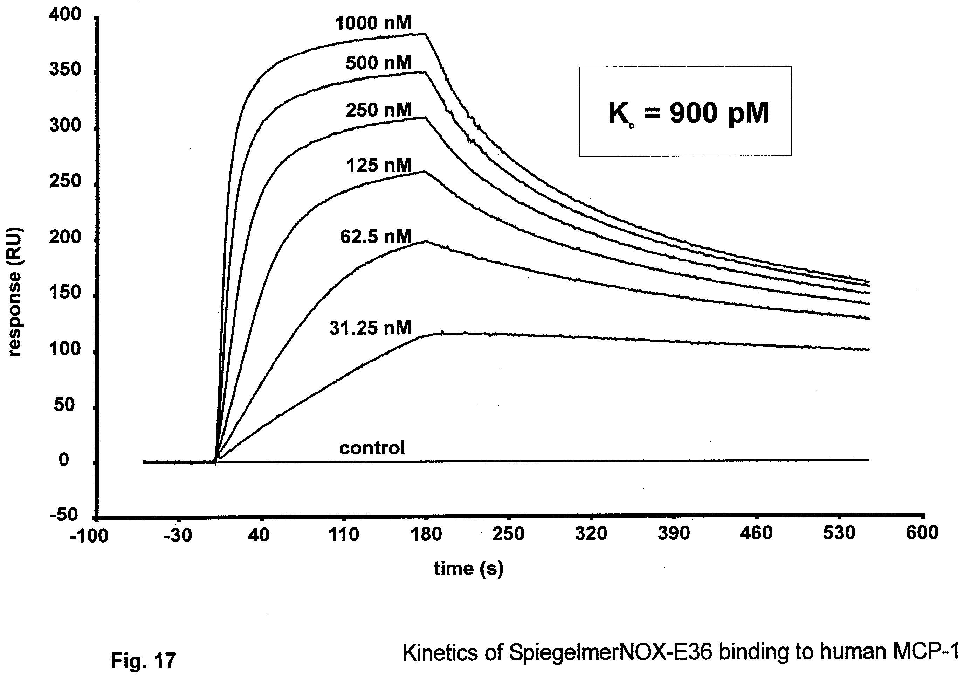

Analysis of the NOX-E36•hMCP-1 interaction: recombinant human MCP-1 was immobilized on a PioneerF1 sensor chip following the manufacturer's recommendations (amine coupling procedure) until an instrument response of 1381 RU (relative units) was established. The determined dissociation constant (K_{D}) for NOX-E36 binding to human MCP-1 was ca. 890 pM (Fig. 17).

Analysis of the 181-A2-018•hMCP-1 interaction: recombinant human MCP-1 was immobilized on a CM4 sensor chip following the manufacturer's recommendations (amine coupling procedure) until an instrument response of 3111 RU (relative units) was established. The determined dissociation constant (K_{D}) for 181-A2-018 binding to human MCP-1 was ca. 370 pM (Fig. 20).

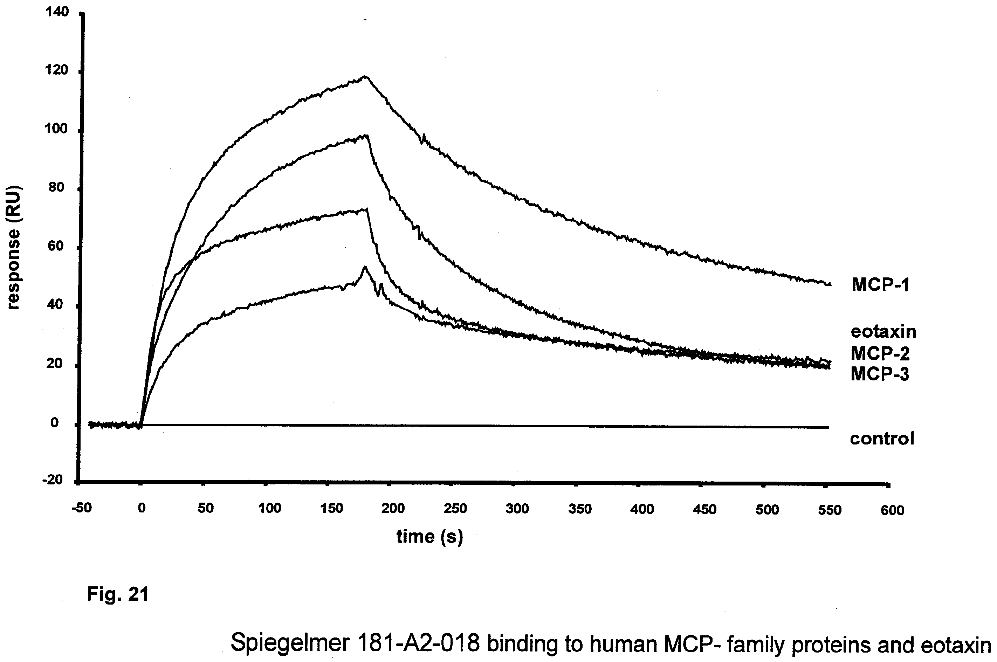

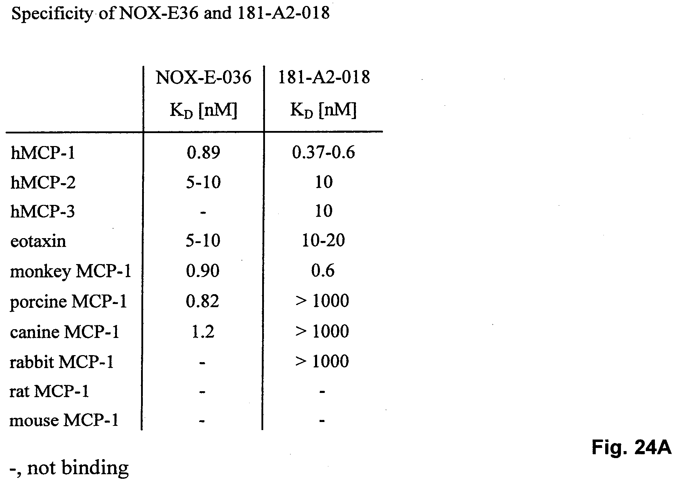

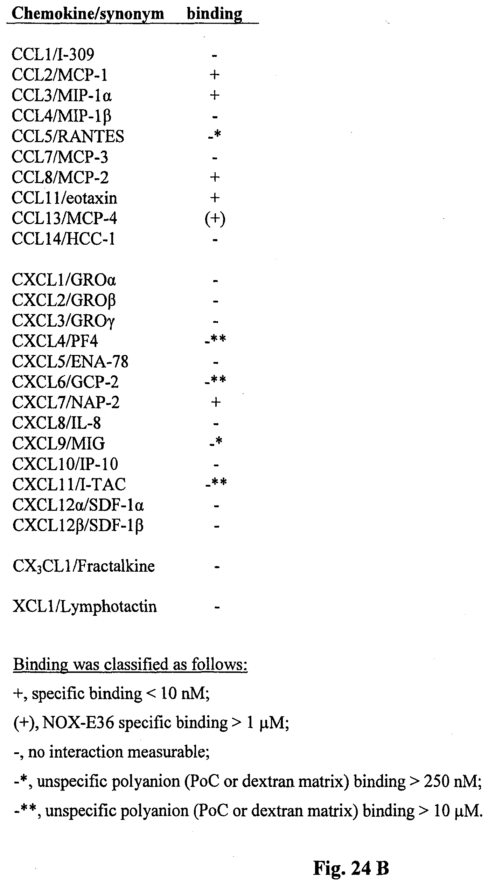

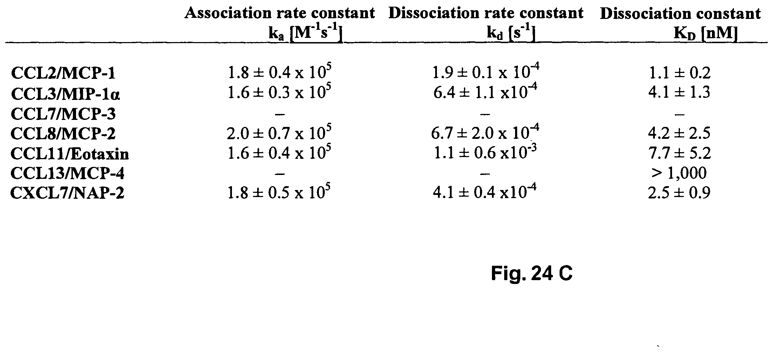

To determine the specificity of NOX-E36 and 181-A2-018, various human MCP-1 family proteins as well as human eotaxin were immobilized on a PioneerF1 and a CM4 sensor chip (hMCP-1, 1754 RU; hMCP-2, 1558 RU; hMCP-3, 1290 RU; eotaxin, 1523 RU). Kinetic analysis revealed that NOX-E36 binds to eotaxin and hMCP-2 with dissociation constants (K_{D}) of 5 - 10 nM; hMCP-3 was not recognized (Figs. 18 and 24A). 181-A2-018, in contrast, binds eotaxin, hMCP-2 and hMCP-3, but with slightly lower affinity (10 - 20 nM; Figs. 21 and 24A).

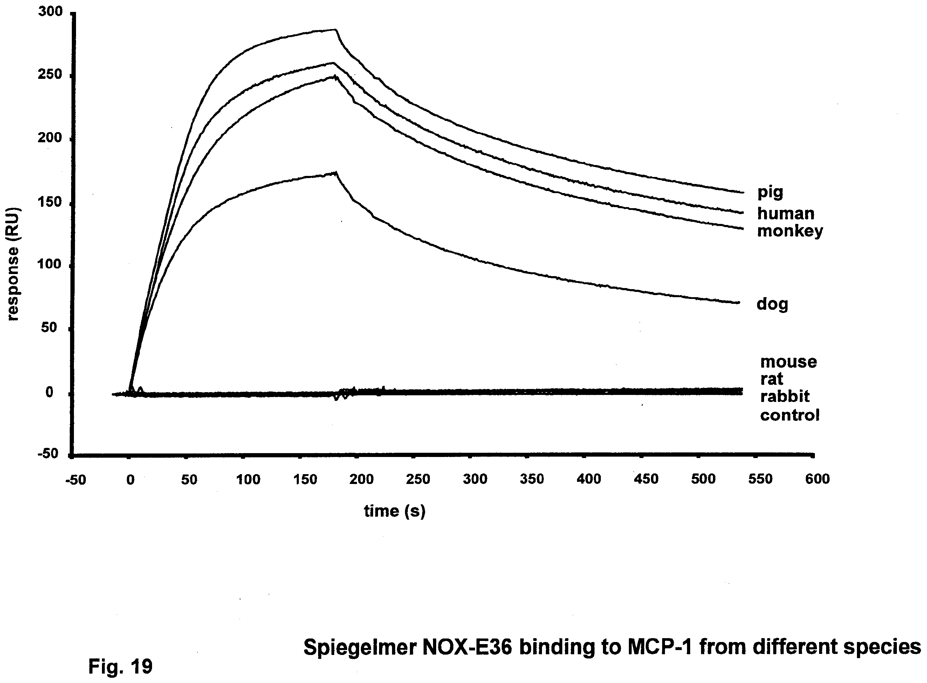

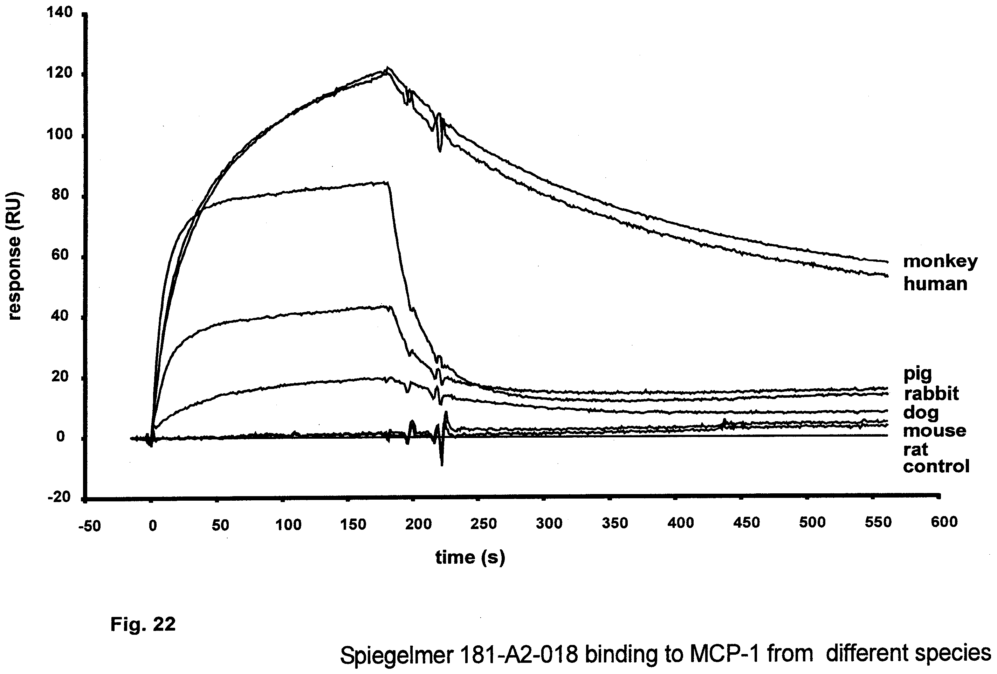

Interspecies cross-reactivity of NOX-E36 and 181-A2-018 was assessed using amino-coupling immobilized MCP-1 from human (1460 RU), monkey (1218 RU), pig (1428 RU), dog (1224 RU), rabbit (1244 RU), rat (1267 RU), and mouse (1361 RU) on a PioneerF1 and a CM4 sensor chip. Kinetic analysis revealed that NOX-E36 binds to human, monkey, porcine, and canine MCP-1 with comparable dissociation constants (K_{D}) of 0.89 - 1.2 nM whereas MCP-1 from mouse, rat and rabbit were not recognized (Figs. 19 and 24A). 181-A2-018 binds to human and monkey MCP-1 with comparable dissociation constants (K_{D}) of 0.5-0.6 nM, whereas porcine, rabbit and canine MCP-1 are bound with much lower affinity. Rat and mouse MCP-1 were not recognized by NOX-A2-018 (Figs. 22 and 24A).

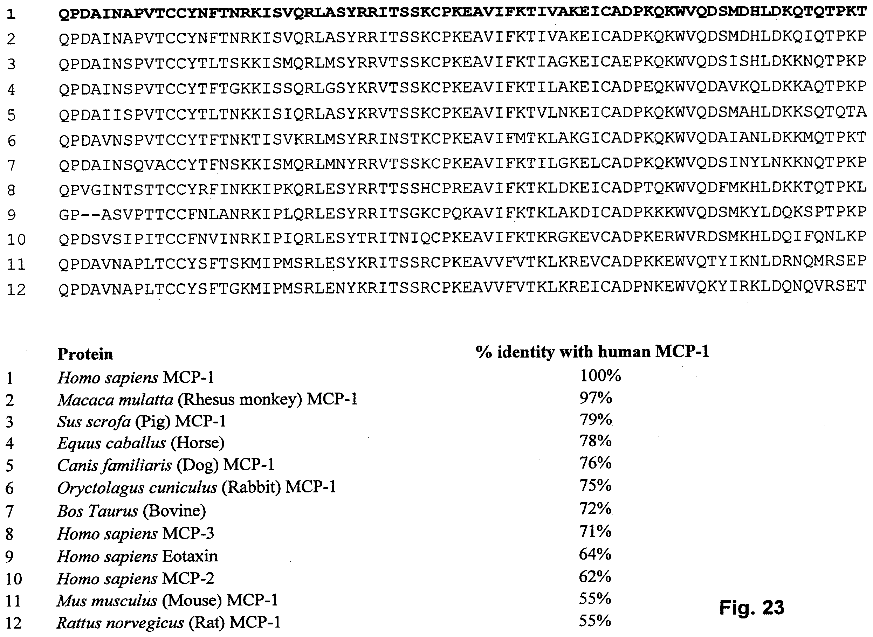

Sequences as well as degree of homology in percent identical amino acids between the MCP-1 protein from different species and closely related human proteins are depicted in Fig. 23; calculated KD values for NOX-E36 and 181-A2-018 are displayed in tabular format in Fig. 24A.

### 9.1.2 mNOX-E36 (murine MCP-1 specific nucleic acid)

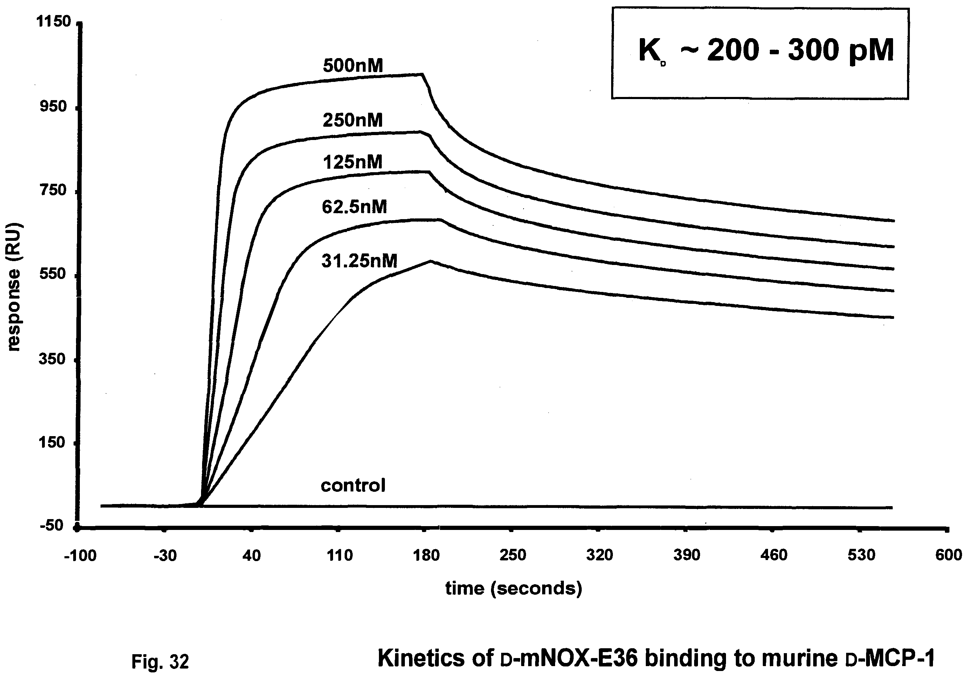

To analyze the binding behaviour of mNOX-E36, 3759 RU of synthetic biotinylated murine D-MCP-1 (flow cell 3) and 3326 RU of biotinylated human D-MCP-1 (flow cell 4) were immobilized on a Streptavidin conjugated sensor chip (Biacore AB, Freiburg, Germany), respectively. mNOX-E36 aptamer (D-RNA) solutions of 500, 250, 125, 62.5, 31.25, and 0 nM were injected using the Kinject command defining an association time of 180 sec and a dissociation time of 360 sec. Flow cell 1 was used as buffer and dextran matrix control (Biacore SA-Chip surface) whereas on flow cell 2, an unspecific D-peptide was immobilized to determine unspecific binding of the aptamer. Fig. 32 shows a sensorgram of the D-NOX-E36 kinetic for binding to murine D-MCP-1 with a calculated dissociation constant (K_{D}) of 200 - 300 pM. mNOX-E36 does not bind human D-MCP-1 (Fig. 33); for sake of clarity, only the sensorgram obtained with 125 nM Spiegelmer is shown.

### 9.2 Selectivity assessment of NOX-E36

Selectivity of NOX-E36 was assessed by surface plasmon resonance analysis by immobilizing 5'biotinylated NOX-E36 on a Streptavidin (SA-Chip). 352 RU of NOX-E36 on flowcell (FC) 1 and equal amount of 5'-terminal biotinylated non-functional control Spiegelmer (POC) on FC 2 were immobilized by streptavidin/biotin binding. FC3 was used as surface control to determine unspecific binding to the dextran-SA sensor surface.

100 nM of a panel of human chemokines from all four subgroups (CC, CXC, CX₃C, and XC) were injected for 360s and complexes were allowed to dissociate for 360s at a flow of 10µl/min and 37°C. Response units after association (Resp.1; degree of interaction) and after dissociation (Resp.2, affinity of interaction) were plotted. After each injection the chip surface was regenerated with a 240s of 1 M sodium chloride with 0,1% Tween; immobilized Spiegelmers were subsequently allowed to refold for 2 minutes at physiological conditions (running buffer). Injection of each chemokine was repeated 3 times. CXCL1, CXCL2, CXCL6 and CXCL9 showed unspecific binding to ribonucleic acids and chip dextran surface. Specific high-affinity binding to immobilized NOX-E36 could only be detected for CCL2/MCP-1, CCL8/MCP-2, CCL11/eotaxin, CCL3/MIP1α, and CXCL7/NAP-2 (Fig. 37B). The finding that MCP-2 and eotaxin are bound by NOX-E36 is not surprising due to the relatively high homology between these chemokines and MCP-1 of 62 and 70 %, for the unexpected positives CCL3/MIP-1α and CXCL7/NAP-2, *in vitro* tests for functional inhibition have been performed or are currently being established, respectively.

Finally, the kinetic parameters of interaction between NOX-E36 and CCL2/MCP-1, CCL8/MCP-2, CCL11/eotaxin, CCL3/MIP1α, CXCL7/NAP-2, CCL7/MCP-3 and CCL13/MCP-4 were determined in the "inverted" system. Here, the chemokines were immobilized and free NOX-E36 was injected (for the detailed protocol, see 9.1). Kinetic data are summarized in Fig. 37C.

### 9.3 Assessment of anti-MIP-1α Functionality in vitro

Biacore measurements had shown cross reactivity of NOX-E36 with MIP-1α. By employing a functional, cell culture-based *in vitro* assay it should be checked if mere Biacore binding of NOX-E36 to MIP-1α also translates to functionality, e.g. antagonism.

To achieve this, chemotaxis experiments with THP-1 cells were performed that can be stimulated by MIP-1 α. THP-1 cells grown as described above were centrifuged, washed once in HBH (HBSS, containing 1 mg/ml bovine serum albumin and 20 mM HEPES) and resuspended at 3 x 10⁶ cells/ml. 100 µl of this suspension were added to Transwell inserts with 5 µm pores (Corning, #3421). In the lower compartments MIP-1α was preincubated together with Spiegelmers in various concentrations in 600 µl HBH at 37°C for 20 to 30 min prior to addition of cells. Cells were allowed to migrate at 37°C for 3 hours. Thereafter the inserts were removed and 60 µl of 440 µM resazurin (Sigma) in phosphate buffered saline was added to the lower compartments. After incubation at 37°C for 2.5 hours, fluorescence was measured at an excitation wavelength of 544 nm and an emission wavelength of 590 nm in a Fluostar Optima multidetection plate reader (BMG).

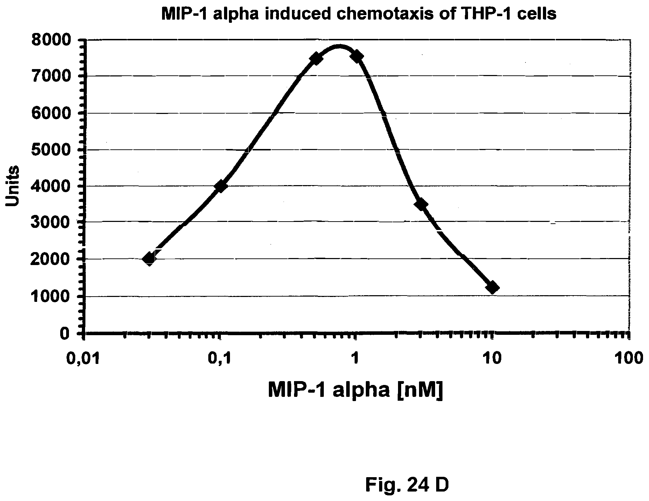

After 3 hours migration of THP-1 cells towards various human MIP-1α concentrations, a dose-response curve for human MIP-1α was obtained, indicating a half-maximal effective concentration of about 1 nM and reduced activation at higher concentrations (Fig. 24D). For the further experiments on inhibition of chemotaxis by Spiegelmers a MIP-1α concentration of 0.5 nM was used.

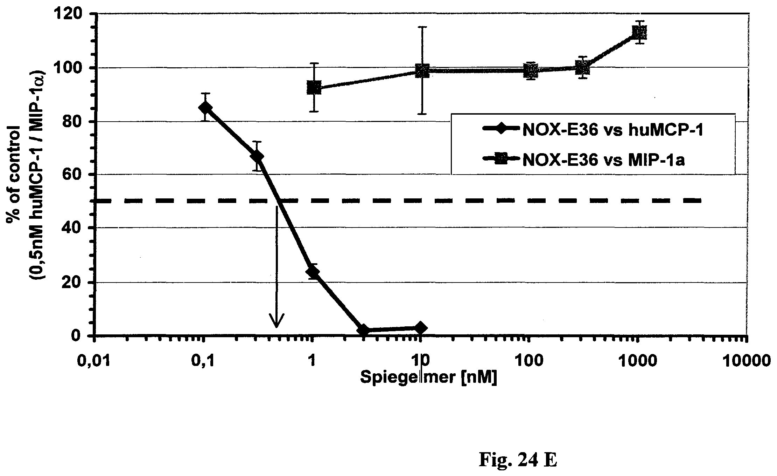

Experiments for determination of chemotaxis inhibition by NOX-E36 were performed with a stimulus of 0.5 nM MIP-1α. It could be clearly shown that NOX-E36 does not inhibit MIP-1α induced chemotaxis up to the highest tested concentration of 1 µM MIP-1α. As positive control, the respective experiment with MCP-1 as stimulus was performed in parallel (Fig. 24E).

### REFERENCES

The complete bibliographic data of the documents recited herein the disclosure of which is incorporated by reference is, if not indicated to the contrary, as follows.
(1983). "The classification of retinal detachment with proliferative vitreoretinopathy." Ophthalmology 90(2): 121-125.
(1992). "Vitrectomy with silicone oil or perfluoropropane gas in eyes with severe proliferative vitreoretinopathy: results of a randomized clinical trial. Silicone Study Report 2." Arch Ophthalmol 110(6): 780-792.
Aiello, L. P., R. L. Avery, P. G. Arrigg, B. A. Keyt, H. D. Jampel, S. T. Shah, L. R. Pasquale, H. Thieme, M. A. Iwamoto, J. E. Park and et al. (1994). "Vascular endothelial growth factor in ocular fluid of patients with diabetic retinopathy and other retinal disorders." N Engl J Med 331(22): 1480-1487.
Anguita, R., J. Roth, A. Makuloluwa, S. Shahid, M. Katta, H. Khalid and D. G. Charteris (2021). "Late Presentation of Retinal Detachment: Clinical Features and Surgical Outcomes." Retina 41(9): 1833-1838.
Antonetti, D. A., R. Klein and T. W. Gardner (2012). "Diabetic retinopathy." N Engl J Med 366(13): 1227-1239.
Apte, R. S. (2021). "Age-Related Macular Degeneration." N Engl J Med 385(6): 539-547. Apte, R. S., D. S. Chen and N. Ferrara (2019). "VEGF in Signaling and Disease: Beyond Discovery and Development." Cell 176(6): 1248-1264.
Armendariz, B. G. and U. Chakravarthy (2024). "Fibrosis in age-related neovascular macular degeneration in the anti-VEGF era." Eye (Lond) 38(17): 3243-3251.
Avery, R. L., J. Pearlman, D. J. Pieramici, M. D. Rabena, A. A. Castellarin, M. A. Nasir, M. J. Giust, R. Wendel and A. Patel (2006). "Intravitreal bevacizumab (Avastin) in the treatment of proliferative diabetic retinopathy." Ophthalmology 113(10): 1695 e1691-1615.
Bachmeier, I., B. G. Armendariz, S. Yu, R. J. Jager, A. Ebneter, C. Glittenberg, D. Pauleikhoff, S. R. Sadda, U. Chakravarthy and S. Fauser (2023). "Fibrosis in neovascular age-related macular degeneration: A review of definitions based on clinical imaging." Surv Ophthalmol 68(5): 835-848.
Ban, C. R. and S. M. Twigg (2008). "Fibrosis in diabetes complications: pathogenic mechanisms and circulating and urinary markers." Vasc Health Risk Manag 4(3): 575-596.
Bandello, F., C. Preziosa, G. Querques and R. Lattanzio (2014). "Update of intravitreal steroids for the treatment of diabetic macular edema." Ophthalmic Res 52(2): 89-96.
Berrocal, M. H., L. A. Acaba and A. Acaba (2016). "Surgery for Diabetic Eye Complications." Curr Diab Rep 16(10): 99.
Bhisitkul, R. B., T. S. Mendes, S. Rofagha, W. Enanoria, D. S. Boyer, S. R. Sadda and K. Zhang (2015). "Macular atrophy progression and 7-year vision outcomes in subjects from the ANCHOR, MARINA, and HORIZON studies: the SEVEN-UP study." Am J Ophthalmol 159(5): 915-924 e912.
Campochiaro, P. A. (1997). "Pathogenic mechanisms in proliferative vitreoretinopathy." Arch Ophthalmol 115(2): 237-241.
Charteris, D. G. (2020). "Proliferative vitreoretinopathy: revised concepts of pathogenesis and adjunctive treatment." Eye (Lond) 34(2): 241-245.
Charteris, D. G., P. Hiscott, H. L. Robey, Z. J. Gregor, S. L. Lightman and I. Grierson (1993). "Inflammatory cells in proliferative vitreoretinopathy subretinal membranes." Ophthalmology 100(1): 43-46.
Claes, C. and A. P. Lafeta (2014). "Proliferative vitreoretinopathy." Dev Ophthalmol 54: 188-195.
Coffee, R. E., L. Jiang and S. A. Rahman (2014). "Proliferative vitreoretinopathy: advances in surgical management." Int Ophthalmol Clin 54(2): 91-109.
Comparison of Age-related Macular Degeneration Treatments Trials Research, G., D. F. Martin, M. G. Maguire, S. L. Fine, G. S. Ying, G. J. Jaffe, J. E. Grunwald, C. Toth, M. Redford and F. L. Ferris, 3rd (2012). "Ranibizumab and bevacizumab for treatment of neovascular age-related macular degeneration: two-year results." Ophthalmology 119(7): 1388-1398.
Curtis, T. M., T. A. Gardiner and A. W. Stitt (2009). "Microvascular lesions of diabetic retinopathy: clues towards understanding pathogenesis?" Eye (Lond) 23(7): 1496-1508.
Daniel, E., W. Pan, G. S. Ying, B. J. Kim, J. E. Grunwald, F. L. Ferris, 3rd, G. J. Jaffe, C. A. Toth, D. F. Martin, S. L. Fine, M. G. Maguire and T. Comparison of Age-related Macular Degeneration Treatments (2018). "Development and Course of Scars in the Comparison of Age-Related Macular Degeneration Treatments Trials." Ophthalmology 125(7): 1037-1046.
Davis, M. D., R. E. Gangnon, L. Y. Lee, L. D. Hubbard, B. E. Klein, R. Klein, F. L. Ferris, S. B. Bressler, R. C. Milton and G. Age-Related Eye Disease Study (2005). "The Age-Related Eye Disease Study severity scale for age-related macular degeneration: AREDS Report No. 17." Arch Ophthalmol 123(11): 1484-1498.
Duh, E. J., J. K. Sun and A. W. Stitt (2017). "Diabetic retinopathy: current understanding, mechanisms, and treatment strategies." JCI Insight 2(14).
Eastlake, K., P. J. Banerjee, A. Angbohang, D. G. Charteris, P. T. Khaw and G. A. Limb (2016). "Muller glia as an important source of cytokines and inflammatory factors present in the gliotic retina during proliferative vitreoretinopathy." Glia 64(4): 495-506.
Ferris, F. L., M. D. Davis, T. E. Clemons, L. Y. Lee, E. Y. Chew, A. S. Lindblad, R. C. Milton, S. B. Bressler, R. Klein and G. Age-Related Eye Disease Study Research (2005). "A simplified severity scale for age-related macular degeneration: AREDS Report No. 18." Arch Ophthalmol 123(11): 1570-1574.
Franceschi, C., M. Bonafe, S. Valensin, F. Olivieri, M. De Luca, E. Ottaviani and G. De Benedictis (2000). "Inflamm-aging. An evolutionary perspective on immunosenescence." Ann N Y Acad Sci 908: 244-254.
Friedlander, M. (2007). "Fibrosis and diseases of the eye." J Clin Invest 117(3): 576-586.
Grunwald, J. E., M. Pistilli, G. S. Ying, M. G. Maguire, E. Daniel, D. F. Martin and G. Comparison of Age-related Macular Degeneration Treatments Trials Research (2015). "Growth of geographic atrophy in the comparison of age-related macular degeneration treatments trials." Ophthalmology 122(4): 809-816.
Guidry, C., K. M. Bradley and J. L. King (2003). "Tractional force generation by human muller cells: growth factor responsiveness and integrin receptor involvement." Invest Ophthalmol Vis Sci 44(3): 1355-1363.
Heier, J. S., D. M. Brown, V. Chong, J. F. Korobelnik, P. K. Kaiser, Q. D. Nguyen, B. Kirchhof, A. Ho, Y. Ogura, G. D. Yancopoulos, N. Stahl, R. Vitti, A. J. Berliner, Y. Soo, M. Anderesi, G. Groetzbach, B. Sommerauer, R. Sandbrink, C. Simader, U. Schmidt-Erfurth, View and V. S. Groups (2012). "Intravitreal aflibercept (VEGF trap-eye) in wet age-related macular degeneration." Ophthalmology 119(12): 2537-2548.
Heier, J. S., A. M. Khanani, C. Quezada Ruiz, K. Basu, P. J. Ferrone, C. Brittain, M. S. Figueroa, H. Lin, F. G. Holz, V. Patel, T. Y. Y. Lai, D. Silverman, C. Regillo, B. Swaminathan, F. Viola, C. M. G. Cheung, T. Y. Wong, Tenaya and L. Investigators (2022). "Efficacy, durability, and safety of intravitreal faricimab up to every 16 weeks for neovascular age-related macular degeneration (TENAYA and LUCERNE): two randomised, double-masked, phase 3, non-inferiority trials." Lancet 399(10326): 729-740.
Hogg, R., E. Curry, A. Muldrew, J. Winder, M. Stevenson, M. McClure and U. Chakravarthy (2003). "Identification of lesion components that influence visual function in age related macular degeneration." Br J Ophthalmol 87(5): 609-614.
Humphrey, M. F., I. J. Constable, Y. Chu and S. Wiffen (1993). "A quantitative study of the lateral spread of Muller cell responses to retinal lesions in the rabbit." J Comp Neurol 334(4): 545-558.
Idrees, S., J. Sridhar and A. E. Kuriyan (2019). "Proliferative Vitreoretinopathy: A Review." Int Ophthalmol Clin 59(1): 221-240.
Ishikawa, K., R. Kannan and D. R. Hinton (2016). "Molecular mechanisms of subretinal fibrosis in age-related macular degeneration." Exp Eye Res 142: 19-25.
Jaffe, G. J., G. S. Ying, C. A. Toth, E. Daniel, J. E. Grunwald, D. F. Martin, M. G. Maguire and G. Comparison of Age-related Macular Degeneration Treatments Trials Research (2019). "Macular Morphology and Visual Acuity in Year Five of the Comparison of Age-related Macular Degeneration Treatments Trials." Ophthalmology 126(2): 252-260.
Joachim, S. C. (2022). "Towards an Understanding of Retinal Diseases and Novel Treatment." Int J Mol Sci 23(14).
Kaplan, H. J. (2007). "Anatomy and function of the eye." Chem Immunol Allergy 92: 4-10.
Kasagi, N., Y. Gomyo, H. Shirai, S. Tsujitani and H. Ito (1994). "Apoptotic cell death in human gastric carcinoma: analysis by terminal deoxynucleotidyl transferase-mediated dUTP-biotin nick end labeling." Jpn J Cancer Res 85(9): 939-945.
Leasher, J. L., R. R. Bourne, S. R. Flaxman, J. B. Jonas, J. Keeffe, K. Naidoo, K. Pesudovs, H. Price, R. A. White, T. Y. Wong, S. Resnikoff, H. R. Taylor and S. Vision Loss Expert Group of the Global Burden of Disease (2016). "Global Estimates on the Number of People Blind or Visually Impaired by Diabetic Retinopathy: A Meta-analysis From 1990 to 2010." Diabetes Care 39(9): 1643-1649.
Levin, L. L. K., P. L.; Hartnett, M. E. (2024). Adler's Physiology of the Eye, Elsevier.
Liu, D., C. Zhang, J. Zhang, G. T. Xu and J. Zhang (2023). "Molecular pathogenesis of subretinal fibrosis in neovascular AMD focusing on epithelial-mesenchymal transformation of retinal pigment epithelium." Neurobiol Dis 185: 106250.
Machemer, R., T. M. Aaberg, H. M. Freeman, A. R. Irvine, J. S. Lean and R. M. Michels (1991). "An updated classification of retinal detachment with proliferative vitreoretinopathy." Am J Ophthalmol 112(2): 159-165.
Martin, F., J. C. Pastor, E. R. De La Rua, A. Mayo-Iscar, J. Garcia-Arumi, V. Martinez, N. Fernandez and M. A. Saornil (2003). "Proliferative vitreoretinopathy: cytologic findings in vitreous samples." Ophthalmic Res 35(4): 232-238.
McMeel, J. W. (1971). "Diabetic retinopathy: fibrotic proliferation and retinal detachment." Trans Am Ophthalmol Soc 69: 440-493.
Nakazawa, T., T. Hisatomi, C. Nakazawa, K. Noda, K. Maruyama, H. She, A. Matsubara, S. Miyahara, S. Nakao, Y. Yin, L. Benowitz, A. Hafezi-Moghadam and J. W. Miller (2007). "Monocyte chemoattractant protein 1 mediates retinal detachment-induced photoreceptor apoptosis." Proc Natl Acad Sci U S A 104(7): 2425-2430.
Pastor-Idoate, S., I. Rodriguez-Hernandez, J. Rojas, I. Fernandez, M. T. Garcia-Gutierrez, J. M. Ruiz-Moreno, A. Rocha-Sousa, Y. D. Ramkissoon, S. Harsum, R. E. MacLaren, D. G. Charteris, J. C. Van Meurs, R. Gonzalez-Sarmiento, J. C. Pastor and P. V. R. S. G. Genetics on (2015). "BAX and BCL-2 polymorphisms, as predictors of proliferative vitreoretinopathy development in patients suffering retinal detachment: the Retina 4 project." Acta Ophthalmol 93(7): e541-549.
Pastor, J. C., E. R. de la Rua and F. Martin (2002). "Proliferative vitreoretinopathy: risk factors and pathobiology." Prog Retin Eye Res 21(1): 127-144.
Pastor, J. C., J. Rojas, S. Pastor-Idoate, S. Di Lauro, L. Gonzalez-Buendia and S. Delgado-Tirado (2016). "Proliferative vitreoretinopathy: A new concept of disease pathogenesis and practical consequences." Prog Retin Eye Res 51: 125-155.
Querques, L., M. Parravano, E. Borrelli, A. Chiaravalloti, M. Tedeschi, R. Sacconi, I. Zucchiatti, F. Bandello and G. Querques (2020). "Anatomical and functional changes in neovascular AMD in remission: comparison of fibrocellular and fibrovascular phenotypes." Br J Ophthalmol 104(1): 47-52.
Roh, M., I. Lains, H. J. Shin, D. H. Park, S. Mach, D. G. Vavvas, I. K. Kim, J. W. Miller, D. Husain and J. B. Miller (2019). "Microperimetry in age-related macular degeneration: association with macular morphology assessed by optical coherence tomography." Br J Ophthalmol 103(12): 1769-1776.
Roy, S., S. Amin and S. Roy (2016). "Retinal fibrosis in diabetic retinopathy." Exp Eye Res 142: 71-75.
Rudolf, M., G. Malek, J. D. Messinger, M. E. Clark, L. Wang and C. A. Curcio (2008). "Subretinal drusenoid deposits in human retina: organization and composition." Exp Eye Res 87(5): 402-408.
Ryu, C. L., S. Al-Humaid, E. Rampakakis, I. J. Galic and J. C. Chen (2016). "Correlation of Visual Acuity with Fibrotic Scar Location in Treated Neovascular Age-Related Macular Degeneration Eyes." Retina 36(7): 1324-1330.
Salmon, J. F. (2024). Kanski's Clinical Ophthalmology, Elsevier.
Schranz, M., S. Sacu, G. S. Reiter, M. Baratsits, S. Desissaire, M. Pircher, G. Mylonas, C. Hitzenberger, U. Schmidt-Erfurth and P. K. Roberts (2024). "Structure-Function Correlation of Retinal Fibrosis in Eyes with Neovascular Age-Related Macular Degeneration." J Clin Med 13(4).
Seddon, J. M., J. Cote, N. Davis and B. Rosner (2003). "Progression of age-related macular degeneration: association with body mass index, waist circumference, and waist-hip ratio." Arch Ophthalmol 121(6): 785-792.
Seddon, J. M., S. George and B. Rosner (2006). "Cigarette smoking, fish consumption, omega-3 fatty acid intake, and associations with age-related macular degeneration: the US Twin Study of Age-Related Macular Degeneration." Arch Ophthalmol 124(7): 995-1001.
Seddon, J. M., W. C. Willett, F. E. Speizer and S. E. Hankinson (1996). "A prospective study of cigarette smoking and age-related macular degeneration in women." JAMA 276(14): 1141-1146.
Sene, A., D. Chin-Yee and R. S. Apte (2015). "Seeing through VEGF: innate and adaptive immunity in pathological angiogenesis in the eye." Trends Mol Med 21(1): 43-51.
Sethi, C. S., G. P. Lewis, S. K. Fisher, W. P. Leitner, D. L. Mann, P. J. Luthert and D. G. Charteris (2005). "Glial remodeling and neural plasticity in human retinal detachment with proliferative vitreoretinopathy." Invest Ophthalmol Vis Sci 46(1): 329-342.
Shu, D. Y., E. Butcher and M. Saint-Geniez (2020). "EMT and EndMT: Emerging Roles in Age-Related Macular Degeneration." Int J Mol Sci 21(12).
Singer, A. J. and R. A. Clark (1999). "Cutaneous wound healing." N Engl J Med 341(10): 738-746.
Sonoda, S., C. Spee, E. Barron, S. J. Ryan, R. Kannan and D. R. Hinton (2009). "A protocol for the culture and differentiation of highly polarized human retinal pigment epithelial cells." Nat Protoc 4(5): 662-673.
Stitt, A. W., T. M. Curtis, M. Chen, R. J. Medina, G. J. McKay, A. Jenkins, T. A. Gardiner, T. J. Lyons, H. P. Hammes, R. Simo and N. Lois (2016). "The progress in understanding and treatment of diabetic retinopathy." Prog Retin Eye Res 51: 156-186.
Tobe, T., S. Ortega, J. D. Luna, H. Ozaki, N. Okamoto, N. L. Derevjanik, S. A. Vinores, C. Basilico and P. A. Campochiaro (1998). "Targeted disruption of the FGF2 gene does not prevent choroidal neovascularization in a murine model." Am J Pathol 153(5): 1641-1646.
Umazume, K., Y. Barak, K. McDonald, L. Liu, H. J. Kaplan and S. Tamiya (2012). "Proliferative vitreoretinopathy in the Swine-a new model." Invest Ophthalmol Vis Sci 53(8): 4910-4916.
van Eeden, P. E., L. B. Tee, S. Lukehurst, C. M. Lai, E. P. Rakoczy, L. D. Beazley and S. A. Dunlop (2006). "Early vascular and neuronal changes in a VEGF transgenic mouse model of retinal neovascularization." Invest Ophthalmol Vis Sci 47(10): 4638-4645.
Wickham, L., G. O. Ho-Yen, C. Bunce, D. Wong and D. G. Charteris (2011). "Surgical failure following primary retinal detachment surgery by vitrectomy: risk factors and functional outcomes." Br J Ophthalmol 95(9): 1234-1238.
Wilke, G. A. and R. S. Apte (2024). "Complement regulation in the eye: implications for age-related macular degeneration." J Clin Invest 134(9).
Wong, W. L., X. Su, X. Li, C. M. Cheung, R. Klein, C. Y. Cheng and T. Y. Wong (2014). "Global prevalence of age-related macular degeneration and disease burden projection for 2020 and 2040: a systematic review and meta-analysis." Lancet Glob Health 2(2): e106-116.
Writing Committee for the Diabetic Retinopathy Clinical Research, N., J. G. Gross, A. R. Glassman, L. M. Jampol, S. Inusah, L. P. Aiello, A. N. Antoszyk, C. W. Baker, B. B. Berger, N. M. Bressler, D. Browning, M. J. Elman, F. L. Ferris, 3rd, S. M. Friedman, D. M. Marcus, M. Melia, C. R. Stockdale, J. K. Sun and R. W. Beck (2015). "Panretinal Photocoagulation vs Intravitreous Ranibizumab for Proliferative Diabetic Retinopathy: A Randomized Clinical Trial." JAMA 314(20): 2137-2146.
Yang, C. M., P. Y. Su, P. T. Yeh and M. S. Chen (2008). "Combined rhegmatogenous and traction retinal detachment in proliferative diabetic retinopathy: clinical manifestations and surgical outcome." Can J Ophthalmol 43(2): 192-198.
Yannuzzi, L. A., S. Negrao, T. Iida, C. Carvalho, H. Rodriguez-Coleman, J. Slakter, K. B. Freund, J. Sorenson, D. Orlock and N. Borodoker (2001). "Retinal angiomatous proliferation in age-related macular degeneration." Retina 21(5): 416-434.
Akahoshi T, Wada C, Endo H, Hirota K, Hosaka S, Takagishi K, Kondo H, Kashiwazaki S, Matsushima K (1993). Expression of monocyte chemotactic and activating factor in rheumatoid arthritis. Regulation of its production in synovial cells by interleukin-1 and tumor necrosis factor. Arthritis Rheum. 36:762
Alam R, York J, Moyars M, Stafford S, Grant JA, Lee J, Forsythe P, Sim T, Ida N (1996). Increased MCP-1, RANTES, and MIP-1α in bronchoalveolar lavage fluid of allergic asthmatic patients. Am. J. Respir. Crit. Care Med. 153:1398
Altschul SF, Gish W, Miller W, Myers EW, Lipman DJ (1990), Basic local alignment search tool. J Mol Biol. 215(3):403-10.
Altschul SF, Madden TL, Schaffer AA, Zhang J, Zhang Z, Miller W, Lipman DJ (1997). Gapped BLAST and PSI-BLAST: a new generation of protein database search programs. Nucleic Acids Res. Sep 1;25(17):3389-402.
Amann B, Tinzmann R, Angelkort B (2003). ACE inhibitors improve diabetic nephropathy through suppression of renal MCP-1. Diabetes Care 26:2421
Anders HJ, Vielhauer V, Frink M, Linde Y, Cohen CD, Blattner SM, Kretzler M, Strutz F, Mack M, Grone HJ, Onuffer J, Horuk R, Nelson PJ, Schlöndorff D (2002). A chemokine receptor CCR-1 antagonist reduces renal fibrosis after unilateral ureter ligation. J. Clin. Invest. 109:251
Anders HJ, Vielhauer V, Schlöndorff D (2003). Chemokines and chemokine receptors are involved in the resolution or progression of renal disease. Kidney Int. 63:401
Aurup H et al. (1994). Nucleic Acids Res 22:20
Austin HA 3rd, Muenz LR, Joyce KM, Antonovych TT, Balow JE (1984). Diffuse proliferative lupus nephritis: identification of specific pathologic features affecting renal outcome. Kidney Int. 25:689
Baggiolini M, Dewald B, Moser B (1994). Interleukin-8 and related chemotactic cytokines - CXC and CC chemokines. Adv. Immunol. 55:97
Baggiolini M (1998). Chemokines and leukocyte traffic. Nature 392:565
Banba N, Nakamura T, Matsumura M, Kuroda H, Hattori Y, Kasai K (2000). Possible relationship of monocyte chemoattractant protein-1 with diabetic nephropathy. Kidney Int. 58:684
Banisor I, Leist TP, Kalman B (2005). Involvement of β-chemokines in the development of inflammatory demyelination. J. Neuroinflammation 2:7
Bazan JF, Bacon KB, Hardiman G, Wang W, Soo K, Rossi D, Greaves DR, Zlotnik A, Schall TJ (1997). A new class of membrane-bound chemokine with a CX3C motif. Nature 385:640 Berkhout TA (1997). J Biol Chem 272:16404
Bohle A, Wehrmann M, Bogenschutz O, Batz C, Muller CA, Muller GA (1991). The pathogenesis of chronic renal failure in diabetic nephropathy. Investigation of 488 cases of diabetic glomerulosclerosis. Pathol. Res. Pract. 187:251
Boring L, Gosling J, Chensue SW, Kunkel SL, Farese RV Jr, Broxmeyer HE, Charo IF (1997). Impaired monocyte migration and reduced type 1 (Th1) cytokine responses in C-C chemokine receptor 2 knockout mice. J. Clin. Invest. 100:2552
Boring L, Gosling J, Cleary M, Charo IF (1998). Decreased lesion formation in CCR2-/- mice reveals a role for chemokines in the initiation of atherosclerosis. Nature 394:894
Boring L, Gosling J, Monteclaro FS, Lusis AJ, Tsou CL, Charo IF (1996). Molecular cloning and functional expression of murine JE (monocyte chemoattractant protein 1) and murine macrophage inflammatory protein 1alpha receptors: evidence for two closely linked C-C chemokine receptors on chromosome 9. J. Biol. Chem. 271:7551
Bossink AW, Paemen L, Jansen PM, Hack CE, Thijs LG, Van Damme J (1995). Plasma levels of the chemokines monocyte chemotactic proteins-1 and -2 are elevated in human sepsis. Blood 86:3841
Bower G, Brown DM, Steffes MW, Vernier RL, Mauer SM (1980). Studies of the glomerular mesangium and the juxtaglomerular apparatus in the genetically diabetic mouse. Lab. Invest. 43:333
Charo IF, Myers SJ, Herman A, Franci C, Connolly AJ, Coughlin SR (1994). Molecular cloning and functional expression of two monocyte chemoattractant protein 1 receptors reveals alternative splicing of the carboxyl-terminal tails. Proc. Natl Acad. Sci. USA 91:2752
Chow FY, Nikolic-Paterson DJ, Ma FY, Ozols E, Rollins BJ, Tesch GH (2007). Monocyte chemoattractant protein-1-induced tissue inflammation is critical for the development of renal injury but not type 2 diabetes in obese db/db mice. Diabetologica 50:471
Chow FY, Nikolic-Paterson DJ, Ozols E, Atkins RC, Rollin BJ, Tesch GH (2006). Monocyte chemoattractant protein-1 promotes the development of diabetic renal injury in streptozotocin-treated mice. Kidney Int. 69:73
Chow F, Ozols E, Nikolic-Paterson DJ, Atkins RC, Tesch GH (2004). Macrophages in mouse type 2 diabetic nephropathy: Correlation with diabetic state and progressive renal injury. Kidney Int. 65:116
Cockwell P, Howie AJ, Adu D, Savage CO (1998). In situ analysis of C-C chemokine mRNA in human glomerulonephritis. Kidney Int. 54:827
Cohen CD, Gröne HJ, Gröne EF, Nelson PJ, Schlöndorff D, Kretzler M (2002). Laser microdissection and gene expression analysis on formaldehyde-fixed archival tissue. Kidney Int. 61:125
Cummins LL et al. (1995). Nucleic Acids Res 23:2019
Dalla Vestra M, Mussap M, Gallina P, Bruseghin M, Cernigoi AM, Saller A, Plebani M, Fioretto P (2005). Acute-phase markers of inflammation and glomerular structure in patients with type 2 diabetes. J. Am. Soc. Nephrol. 16 Suppl 1:S78
Dawson J, Miltz W, Mir AK, Wiessner C (2003). Targeting monocyte chemoattractant protein-1 signalling in disease. Expert Opin. Ther. Targets 7:35
De Bleecker JL, De Paepe B, Vanwalleghem IE, Schroder JM (2002). Differential expression of chemokines in inflammatory myopathies. Neurology 58:1779
Drolet DW, Nelson J, Tucker CE, Zack PM, Nixon K, Bolin R, Judkins MB, Farmer JA, Wolf JL, Gill SC, Bendele RA (2000). Pharmacokinetics and safety of an anti-vascular endothelial growth factor aptamer (NX1838) following injection into the vitreous humor of rhesus monkeys. Pharm. Res. 17:1503
Eaton BE et al. (1995). Chem Biol 2:633
Eaton BE, Gold L, Hicke BJ, Janjic N, Jucker FM, Sebosta DP, Tarasow TM, Willis MC, Zichi DA (1997). Bioorg Med Chem 5:1087
Economou E, Tousoulis D, Katinioti A, Stefanadis C, Trikas A, Pitsavos C, Tentolouris C, Toutouza MG, Toutouzas P (2001). Chemokines in patients with ischaemic heart disease and the effect of coronary angioplasty. Int. J. Cardiol. 80:55
Egashira K, Zhao Q, Kataoka C, Ohtani K, Usui M, Charo IF, Nishida K, Inoue S, Katoh M, Ichiki T, Takeshita A (2002). Importance of monocyte chemoattractant protein-1 pathway in neointimal hyperplasia after periarterial injury in mice and monkeys. Circ. Res. 90:1167
Fujinaka H, Yamamoto T, Takeya M, Feng L, Kawasaki K, Yaoita E, Kondo D, Wilson CB, Uchiyama M, Kihara I (1997). Suppression of anti-glomerular basement membrane nephritis by administration of anti-monocyte chemoattractant protein-1 antibody in WKY rats. J. Am. Soc. Nephrol. 8:1174
Furuichi K, Wada T, Iwata Y, Kitagawa K, Kobayashi K-I, Hashimoto H, Ishiwata Y, Tomosugi N, Mukaida N, Matsushima K, Egashira K, Yokoyama H (2003). Gene therapy expressing amino-terminal truncated monocyte chemoattractant protein-1 prevents renal ischemia-reperfusion injury. J. Am. Soc. Nephrol. 14:1066
Furuta T, Saito T, Ootaka T, Soma J, Obara K, Abe K, Yoshinaga K (1993). The role of macrophages in diabetic glomerulosclerosis. Am. J. Kidney Dis. 21:480
Galasso JM, Liu Y, Szaflarski J, Warren JS, Silverstein FS (2000). Monocyte chemoattractant protein-1 is a mediator of acute excitotoxic injury in neonatal rat brain. Neuroscience 101:737 Galkina E, Ley K (2006). Leukocyte recruitment and vascular injury in diabetic nephropathy. J. Am. Soc. Nephrol. 17:368-377
Gao JL, Kuhns DB, Tiffany HL, McDermott D, Li X, Francke U, Murphy PM (1993). Structure and functional expression of the human macrophage inflammatory protein 1 alpha/RANTES receptor. J. Exp. Med. 177:1421
Garcia-Zepeda EA, Combadiere C, Rothenberg ME, Sarafi MN, Lavigne F, Hamid Q, Murphy PM, Luster AD (1996). Human monocyte chemoattractant protein (MCP)-4 is a novel CC chemokine with activities on monocytes, eosinophils, and basophils induced in allergic and nonallergic inflammation that signals through the CC chemokine receptors (CCR)-2 and -3. J. Immunol. 157:5613
Gerard C, Rollins, BJ. Chemokines and disease. Nat. Immunol. 6:1182
Gong X, Gong W, Kuhns DB, Ben-Baruch A, Howard OM, Wang JM (1997). Monocyte chemotactic protein-2 (MCP-2) uses CCR1 and CCR2B as its functional receptors. J. Biol. Chem. 272:11682
Gonzalo JA, Lloyd CM, Wen D, Albar JP, Wells TNC, Proudfoot A, Martinez-A C, Dorf M, Bjerke T, Coyle AJ, Gutierrez-Ramos JC (1998). The coordinated action of CC chemokines in the lung orchestrates allergic inflammation and airway hyperresponsiveness. J. Exp. Med. 188:157
Gordillo GM, Onat D, Stockinger M, Roy S, Atalay M, Beck FM, Sen CK (2004). A key angiogenic role of moncyte chemoattractant protein-1 in hemangioendothelioma proliferation. Am. J. Physiol. Cell Physiol. 287:C866
Green LS et al. (1995). Chem Biol 2:683
Handel TM, Domaille PJ (1996). Heteronuclear (1H, 13C, 15N) NMR assignments and solution structure of the monocyte chemoattractant protein-1 (MCP-1) dimer. Biochemistry 35:6569
Harigai M, Hara M, Yoshimura T, Leonard EJ, Inoue K, Kashiwazaki S (1993). Monocyte chemoattractant protein-1 (MCP-1) in inflammatory joint diseases and its involvement in the cytokine network of rheumatoid synovium. Clin. Immunol. Immunopathol. 69:83
Hasegawa H, Kohno M, Sasaki M, Inoue A, Ito MR, Terada M, Hieshima K, Maruyama H, Miyazaki J, Yoshie O, Nose M, Fujita S (2003). Antagonist of monocyte chemoattractant protein 1 ameliorates the initiation and progression of lupus nephritis and renal vasculitis in MRL/lpr mice. Arthritis Rheum. 48:2555
Heath H, Qin S et al. (1997). Chemokine receptor usage by human eosinophils. The importance of CCR3 demonstrated using an antagonistic monoclonal antibody. J Clin Invest 99:178
Holdsworth SR, Kitching AR, Tipping PG (2000). Chemokines as therapeutic targets in renal disease. Curr. Opin. Nephrol. Hypertens. 9:505
Holgate ST, Bodey KS, Janezic A, Frew AJ, Kaplan AP, Teran LM (1997). Release of RANTES, MIP-1α, and MCP-1 into asthmatic airways following endobronchial allergen challenge. Am. J. Respir. Crit. Care Med. 156:1377
Hosaka S et al. (1994). Clin Exp Immunol 97:451
Huang DR, Wang J, Kivisakk P, Rollins BJ, Ransohoff RM (2001). Absence of monocyte chemoattractant protein 1 in mice leads to decreased local macrophage recruitment and antigen-specific T helper cell type 1 immune response in experimental autoimmune encephalomyelitis. J. Exp. Med. 193:713
Hulkower K, Brosnan CF, Aquino DA, Cammer W, Kulshrestha S, Guida MP, Rapoport DA, Berman JW (1993). Expression of CSF-1, c-fms, and MCP-1 in the central nervous system of rats with experimental allergic encephalomyelitis. J. Immunol. 150:2525
Humbert M, Ying S, Corrigan C, Menz G, Barkans J, Pfister R, Meng Q, Van Damme J, Opdenakker G, Durham SR, Kay AB (1997). Bronchial mucosal expression of the genes encoding chemokines RANTES and MCP-3 in symptomatic atopic and nonatopic asthmatics: relationship to the eosinophil-active cytokines interleukin (IL)-5, granulocyte macrophage-colony-stimulating factor, and IL-3. Am J Respir Cell Mol Biol 16:1
Ihm CG, Park JK, Hong SP, Lee TW, Cho BS, Kim MJ, Cha DR, Ha H (1998). A high glucose concentration stimulates the expression of monocyte chemotactic peptide 1 in human mesangial cells. Nephron 79:33
Iyonaga K, Takeya M, Saita N, Sakamoto O, Yoshimura T, Ando M, Takahashi K (1994). Monocyte chemoattractant protein-1 in idiopathic pulmonary fibrosis and other interstitial lung diseases. Hum. Pathol. 25:455
Johrer K, Zelle-Rieser C, Perathoner A, Moser P, Hager M, Ramoner R, Gander H, Holtl L, Bartsch G, Greil R, Thurnher M (2005). Up-regulation of functional chemokine receptor CCR3 in human renal cell carcinoma. Clin Cancer Res 11:2459
Jolicoeur C, Lemay A, Akoum A (2001). Comparative effect of danazol and a GnRH agonist on monocyte chemotactic protein-1 expression by endometriotic cells. Am. J. Reprod. Immunol. 45:86
Jose PJ, Griffiths-Johnson DA, Collins PD, Walsh DT, Moqbel R, Totty NF, Truong O, Hsuan JJ, Williams TJ. Eotaxin: a potent eosinophil chemoattractant cytokine detected in a guinea pig model of allergic airways inflammation. J. Exp. Med. 179:881
Kaburagi Y, Shimada Y, Nagaoka T, Hasegawa M, Takehara K, Sato S (2001). Enhanced production of CC-chemokines (RANTES, MCP-1, MIP-1α, MIP-1β, and eotaxin) in patients with atopic dermatitis. Arch. Dermatol. Res. 293:350
Kawasaki AM et al. (1993). J Med Chem 36:831
Kennedy KJ, Strieter RM, Kunkel SL, Lukacs NW, Karpus WJ (1998). Acute and relapsing experimental autoimmune encephalomyelitis are regulated by differential expression of the CC chemokines macrophage inflammatory protein-1α and monocyte chemotactic protein-1. J. Neuroimmunol. 91:98
Kim JS, Gautam SC, Chopp M, Zaloga C, Jones ML, Ward PA, Welch KM (1995). Expression of monocyte chemoattractant protein-1 and macrophage inflammatory protein-1 after focal cerebral ischemia in the rat. J. Neuroimmunol. 56:127
Kitamoto S, Egashira K (2003). Anti-monocyte chemoattractant protein-1 gene therapy for cardiovascular diseases. Expert Rev. Cardiovasc. Ther. 1:393
Kleinhans M, Tun-Kyi A, Gilliet M, Kadin ME, Dummer R, Burg G, and Nestle FO (2003). Functional expression of the eotaxin receptor CCR3 in CD30+ cutaneous T-cell lymphoma. Blood 101:1487
Koch AE, Kunkel SL, Harlow LA, Johnson B, Evanoff HL, Haines GK, Burdick MD, Pope RM, Strieter RM (1992). Enhanced production of monocyte chemoattractant protein-1 in rheumatoid arthritis. J. Clin. Invest. 90:772
Kouno J, Nagai H, Nagahata T, Onda M, Yamaguchi H, Adachi K, Takahashi H, Teramoto A, and Emi M (2004). Up-regulation of CC chemokine, CCL3L1, and receptors, CCR3, CCR5 in human glioblastoma that promotes cell growth. J Neurooncol 70:301
Kurihara T, Warr G, Loy J, Bravo R (1997). Defects in macrophage recruitment and host defense in mice lacking the CCR2 chemokine receptor. J. Exp. Med. 186:1757
Kusser W (2000). J Biotechnol 74:27-38
Kuziel WA, Morgan SJ, Dawson TC, Griffin S, Smithies O, Ley K, Maeda N (1997). Severe reduction in leukocyte adhesion and monocyte extravasation in mice deficient in CC chemokine receptor 2. Proc. Natl Acad. Sci. U S A 94:12053
Lesnik EA et al. (1993). Biochemistry 32:7832
Lloyd CM, Minto AW, Dorf ME, Proudfoot A, Wells TNC, Salant DJ, Gutierrez-Ramos JC (1997). RANTES and monocyte chemoattractant protein-1 (MCP-1) play an important role in the inflammatory phase of crescentic nephritis, but only MCP-1 is involved in crescent formation and interstitial fibrosis. J. Exp. Med. 185:1371
Lu BB, Rutledge BJ, Gu L, Fiorillo J, Lukacs NW, Kunkel SL, North R, Gerard C, Rollins BJ (1998). Abnormalities in monocyte recruitment and cytokine expression in monocyte chemoattractant protein-1 deficient mice. J. Exp. Med. 187:601
Lubkowski J, Bujacz G, Boque L, Domaille PJ, Handel TM, Wlodawer A (1997). The structure of MCP-1 in two crystal forms provides a rare example of variable quaternary interactions. Nat Struct Biol 4:64
Mack M, Cihak J, Simonis C, Luckow B, Proudfoot AE, Plachy J, Bruhl H, Frink M, Anders HJ, Vielhauer V, Pfirstinger J, Stangassinger M, Schlöndorff D (2001). Expression and characterization of the chemokine receptors CCR2 and CCR5 in mice. J. Immunol. 166:4697
Martinelli R, Sabroe I, LaRosa G, Williams TJ, Pease JE. The CC chemokine eotaxin (CCL11) is a partial agonist of CC chemokine receptor 2b. J Biol Chem 276:42957
Matsushima K, Morishita K, Yoshimura T, Lavu S, Kobayashi Y, Lew W, Appella E, Kung HF, Leonard EJ, Oppenheim JJ (1989). Molecular cloning of a human monocyte-derived neutrophil chemotactic factor (MDNCF) and the induction of MDNCF mRNA by interleukin 1 and tumor necrosis factor. J. Exp. Med. 167:1883
McGinnis S, Madden TL (2004). BLAST: at the core of a powerful and diverse set of sequence analysis tools. Nucleic Acids Res. 32(Web Server issue):W20-5.
Meyer TW (2003). Immunosuppression for diabetic glomerular disease? Kidney Int. 63:377
Miller MD, Krangel MS (1992). Biology and biochemistry of the chemokines: a family of chemotactic and inflammatory cytokines. Crit. Rev. Immunol. 12:17
Miller LE et al. (1993). J Physiol 469:213
Mora C, Navarro JF (2005). The role of inflammation as a pathogenic factor in the development of renal disease in diabetes. Curr. Diab. Rep. 5:399
Morii T, Fujita H, Narita T, Shimotomai T, Fujishima H, Yoshioka N, Imai H, Kakei M, Ito S (2003). Association of monocyte chemoattractant protein-1 with renal tubular damage in diabetic nephropathy. J. Diabetes Complications 17:11
Murphy PM, Baggiolini M, Charo IF, Hebert CA, Horuk R, Matsushima K, Miller LH, Oppenheim JJ, Power CA (2000). International union of pharmacology. XXII. Nomenclature for chemokine receptors. Pharmacol. Rev. 52:145
Nakamura H, Weiss ST, Israel E, Luster AD, Drazen JM, Lilly CM (1999). Eotaxin and impaired lung function in asthma. Am J Respir Crit Care Med 160:1952
Nakazawa T, Hisatomi T, Nakazawa C, Noda K, Maruyama K, She H, Matsubara A, Miyahara S, Nakao S, Yin Y, Benowitz L, Hafezi-Moghadam A, Miller JW (2007). Monocyte chemoattractant protein 1 mediated retinal detachment-induced photoreceptor apoptosis. Proc Natl. Acad. Sci. U S A 104:2425
Navarro JF, Mora C, Maca M, Garca J (2003). Inflammatory parameters are independently associated with urinary albumin in type 2 diabetes mellitus. Am. J. Kidney Dis. 42:53
Myers SJ, Wong LM, Charo IF (1995). Signal transduction and ligand specificity of the human monocyte chemoattractant protein-1 receptor in transfected embryonic kidney cells. J. Biol. Chem. 270:5786
Needleman & Wunsch (1970), A general method applicable to the search for similarities in the amino acid sequence of two proteins. J Mol Biol. 48(3):443-53.
Nelken NA, Coughlin SR, Gordon D, Wilcox JN (1991). Monocyte chemoattractant protein-1 in human atheromatous plaques. J. Clin. Invest. 88:1121
Neote K, DiGregorio D, Mak JY, Horuk R, Schall TJ (1993). Molecular cloning, functional expression, and signaling characteristics of a C-C chemokine receptor. Cell 72:415
Ninichuk V, Gross O, Reichel C, Khandoga A, Pawar RD, Ciubar R, Segerer S, Belemezova E, Radomska E, Luckow B, de Lema GP, Murphy PM, Gao JL, Henger A, Kretzler M, Horuk R, Weber M, Krombach F, Schlondorff D, Anders HJ (2005). Delayed chemokine receptor 1 blockade prolongs survival in collagen 4A3-deficient mice with Alport disease. J. Am. Soc. Nephrol. 16:977
Ogata H, Takeya M, Yoshimura T, Takagi K, Takahashi K (1997). The role of monocyte chemoattractant protein-1 (MCP-1) in the pathogenesis of collagen-induced arthritis in rats. J. Pathol. 182:106
Okuno T, Andoh A, Bamba S, Araki Y, Fujiyama Y, Fujiyama M, Bamba T (2002). Interleukin-1β and tumor necrosis factor-α induce chemokine and matrix metalloproteinase gene expression in human colonic subepithelial myofibroblasts. Scand. J. Gastroenterol. 37:317
Oppenheim JJ, Zachariae CO, Mukaida N, Matsushima K (1991). Properties of the novel proinflammatory supergene "intercrine" cytokine family. Annu. Rev. Immunol. 9:617
Pawar RD, Patole PS, Zecher D, Segerer S, Kretzler M, Schlöndorff D, Anders HJ (2006). Toll-like receptor-7 modulates immune complex glomerulonephritis. J. Am. Soc. Nephrol. 17:141
Pearson & Lipman (1988), Improved tools for biological sequence comparison. Proc. Nat'l. Acad. Sci. USA 85: 2444
Perez de Lema G, Maier H, Franz TJ, Escribese M, Chilla mS, Segerer S, Camarasa N, Schmid H, Banas B, Kalaydjiev S, Busch DH, Pfeffer K, Mampaso F, Schlöndorff D, Luckow B (2005). Chemokine receptor CCR2 deficiency reduces renal disease and prolongs survival in MRL/lpr lupus-prone mice. J. Am. Soc. Nephrol. 16:3592
Perez de Lema G, Maier H, Nieto E, Vielhauer V, Luckow B, Mampaso F, Schlöndorff D. Chemokine expression precedes inflammatory cell infiltration and chemokine receptor and cytokine expression during the initiation of murine lupus nephritis. J. Am. Soc. Nephrol. 12:1369
Ponath PD, Qin S, Ringler DJ, Clark-Lewis I, Wang J, Kassam N, Smith H, Shi X, Gonzalo JA, Newman W, Gutierrez-Ramos JC, Mackay CR (1996a). Cloning of the human eosinophil chemoattractant, eotaxin. Expression, receptor binding, and functional properties suggest a mechanism for the selective recruitment of eosinophils. J. Clin. Invest. 97:604
Ponath PD, Qin S, Post TW, Wang J, Wu L, Gerard NP, Newman W, Gerard C, Mackay CR (1996b). Molecular cloning and characterization of a human eotaxin receptor expressed selectively on eosinophils. J. Exp. Med. 183:2437
Power CA, Meyer A, Nemeth K, Bacon KB, Hoogewerf AJ, Proudfoot AE, Wells TN (1995). Molecular cloning and functional expression of a novel CC chemokine receptor cDNA from a human basophilic cell line. J. Biol. Chem. 270:19495
Qi Z, Whitt I, Mehta A, Jin J, Zhao M, Harris RC, Fogo AB, Breyer MD (2004). Serial determination of glomerular filtration rate in conscious mice using FITC-inulin clearance. Am. J. Physiol. Renal Physiol. 286:F590
Qin S, LaRosa G, Campbell JJ, Smith-Heath H, Kassam N, Shi X, Zeng L, Buthcher EC, Mackay CR (1996). Expression of monocyte chemoattractant protein-1 and interleukin-8 receptors on subsets of T cells: correlation with transendothelial chemotactic potential. Eur. J. Immunol. 26:640
Ransohoff RM et al. (1993). FASEB J 7:592
Raport CJ, Gosling J, Schweickart VL, Gray PW, Charo IF (1996). Molecular cloning and functional characterization of a novel human CC chemokine receptor (CCR5) for RANTES, MIP-1β, and MIP-1α. J. Biol. Chem. 271:17161
Ritz E, Rychlik I, Locatelli F, Halimi S (1999). End-stage renal failure in type 2 diabetes: A medical catastrophe of worldwide dimensions. Am. J. Kidney Dis. 34:795-808
Rollins BJ, Stier P, Ernst T, Wong GG (1989). The human homolog of the JE gene encodes a monocyte secretory protein. Mol. Cell Biol. 9:4687
Rollins BJ (1996). Monocyte chemoattractant protein 1: a potential regulator of monocyte recruitment in inflammatory disease. Mol. Med. Today 2:198
Rovin BH, Rumancik M, Tan L, Dickerson J (1994). Glomerular expression of monocyte chemoattractant protein-1 in experimental and human glomerulonephritis. Lab. Invest. 71:536
Ruffing N, Sullivan N, et al. (1998). CCR5 has an expanded ligand-binding repertoire and is the primary receptor used by MCP-2 on activated T cells. Cell Immunol 189:160
Salcedo R, Ponce ML, Young HA, Wasserman K, Ward JM, Keinman HK, Oppenheim JJ, Murphy WJ (2000). Human endothelial cells express CCR2 and respond to MCP-1: direct role of MCP-1 in angiogenesis and tumor progression. Blood 96:34
Samson M, Labbe O, Mollereau C, Vassart G, Parmentier M (1996). Molecular cloning and functional expression of a new human CC-chemokine receptor gene. Biochemistry 35:3362
Schall TJ, Bacon KB (1994). Chemokines, leukocyte trafficking, and inflammation. Curr. Opin. Immunol. 6:865
Schneider A, Panzer U, Zahner G, Wenzel U, Wolf G, Thaiss F, Helmchen U, Stahl RA (1999). Monocyte chemoattractant protein-1 mediates collagen deposition in experimental glomerulonephritis by transforming growth factor-beta. Kidney Int. 56:135
Schwarting A, Paul K, Tschirner S, Menke J, Hansen T, Brenner W, Kelly VR, Relle M, Galle PR (2005). Interferon-beta: a therapeutic for autoimmune lupus in MRL-Faslpr mice. J. Am. Soc. Nephrol. 16:3264
Schwartz CJ, Valente AJ, Sprague EA (1993). A modern view of atherogenesis. Am. J. Cardiol. 71:9B
Segerer S, Nelson PJ, Schlöndorff D (2000). Chemokines, chemokine receptors, and renal disease: from basic science to pathophysiologic and therapeutic studies. J. Am. Soc. Nephrol. 11:152
Shimizu S, Nakashima H, Masutani K, Inoue Y, Miyake K, Akahoshi M, Tanaka Y, Egashira K, Hirakata H, Otsuka T, Harada M (2004). Anti-monocyte chemoattractant protein-1 gene therapy attenuates nephritis in MRL/lpr mice. Rheumatology (Oxford) 43:1121
Smith & Waterman (1981), Adv. Appl. Math. 2: 482
Springer TA (1995). Traffic signals on endothelium for lymphocyte recirculation and leukocyte emigration. Annu. Rev.Physiol. 57:827
Steinman L (2004). Immune therapy for autoimmune diseases. Science 305:212
Svensson M, Sundkvist G, Arnqvist HJ, Bjork E, Blohme G, Bolinder J, Henricsson M, Nystrom L, Torffvit O, Waernbaum I, Ostman J, Eriksson JW (2003). Signs of nephropathy may occur early in young adults with diabetes despite modern diabetes management: Results from the nationwide population-based Diabetes Incidence Study in Sweden (DISS). Diabetes Care 26:2903
Takebayashi K, Matsumoto S, Aso Y, Inukai T (2006). Association between circulating monocyte chemoattractant protein-1 and urinary albumin excretion in nonobese Type 2 diabetic patients. J. Diabetes Complications 20:98
Takeya M, Yoshimura T, Leonard EJ, Takahashi K (1993). Detection of monocyte chemoattractant protein-1 in human atherosclerotic lesions by an anti-monocyte chemoattractant protein-1 monoclonal antibody. Hum. Pathol. 24:534
Tang WW, Qi M, Warren JS (1996). Monocyte chemoattractant protein 1 mediates glomerular macrophage infiltration in anti-GBM Ab GN. Kidney Int. 50:665
Tashiro K, Koyanagi I, Saitoh A, Shimizu A, Shike T, Ishiguro C, Koizumi M, Funabiki K, Horikoshi S, Shirato I, Tomino Y (2002). Urinary levels of monocyte chemoattractant protein-1 (MCP-1) and interleukin-8 (IL-8), and renal injuries in patients with type 2 diabetic nephropathy. J. Clin. Lab. Anal. 16:1
Tesch GH, Maifert S, Schwarting A, Rollins BJ, Kelley VR (1999). Monocyte chemoattractant protein 1-dependent leukocytic infiltrates are responsible for autoimmune disease in MRL-Fas(lpr) mice. J. Exp. Med. 190:1813
Tuaillon N, Shen de F, Berger RB, Lu B, Rollins BJ, Chan CC (2002). MCP-1 expression in endotoxin-induced uveitis. Invest. Ophthalmol. Vis. Sci. 43:1493
Tuttle KR (2005). Linking metabolism and immunology: diabetic nephropathy is an inflammatory disease. J. Am. Soc. Nephrol. 16:1537
Uguccioni M, Mackay CR et al. (1997). High expression of the chemokine receptor CCR3 in human blood basophils. Role in activation by eotaxin, MCP-4, and other chemokines. J Clin Invest 100:1137
United States Renal Data System (2004). Annual data report: Incidence and prevalence 2004. Am. J. Kidney Dis. 45:S77
Utimura R, Fujihara CK, Mattar AL, Malheiros DM, Noronha IL, Zatz R (2003). Mycophenolate mofetil prevents the development of glomerular injury in experimental diabetes. Kidney Int. 63:209
Van Riper G, Siciliano S, Fischer PA, Meurer R, Springer MS, Rosen H (1993). Characterization and species distribution of high affinity GTP-coupled receptors for human rantes and monocyte chemoattractant protein 1. J. Exp. Med. 177:851
Venkatesan N et al. (2003). Curr Med Chem 10:1973
Vestergaard C, Just H, Baumgartner Nielsen J, Thestrup-Pedersen K, Deleuran M (2004). Expression of CCR2 on monocytes and macrophages in chronically inflamed skin in atopic dermatitis and psoriasis. Acta Derm. Venereol. 84:353
Viedt C, Orth SR (2002). Monocyte chemoattractant protein-1 (MCP-1) in the kidney: does it more than simply attract monocytes? Nephrol. Dial. Transplant. 17:2043
Wada T, Furuichi K, Segada-Takaeda C, Ahimizu M, Sakai N, Takeda SI, Takasawa K, Kida H, Kobayashi KI, Mukaida N, Ohmoto Y, Matsushima K, Yokoyama H (1999). MIP-1α and MCP-1 contribute to crescents and interstitial lesions in human crescentic glomerulonephritis. Kidney Int. 56:995
Wada T, Yokoyama H, Matsushima K, Kobayashi KI (2001). Chemokines in renal diseases. Int. Immunopharmacol. 1:637
Wada T, Yokoyama H, Furuichi K, Kobayashi KI, Harada K, Naruto M, Su SB, Akiyama M, Mukaida N, Matsushima K (1996). Intervention of crescentic glomerulonephritis by antibodies to monocyte chemotactic and activating factor (MCAF/MCP-1). FASEB J. 10:1418
Wang X, Yue TL, Barone FC, Feuerstein GZ (1995). Monocyte chemoattractant protein-1 messenger RNA expression in rat ischemic cortex. Stroke 26:661
Wenzel U, Schneider A, Valente AJ, Abboud HE, Thaiss F, Helmchen UM, Stahl RA (1997). Monocyte chemoattractant protein-1 mediates monocyte/macrophage influx in anti-thymocyte antibody-induced glomerulonephritis. Kidney Int. 51:770
Yamagishi S, Inagaki Y, Okamoto T, Amano S, Koga K, Takeuchi M, Makita Z (2002). Advanced glycation end product-induced apoptosis and overexpression of vascular endothelial growth factor and monocyte chemoattractant protein-1 in human-cultured mesangial cells. J. Biol. Chem. 277:20309
Ying S, Robinson DS, Meng Q, Rottman J, Kennedy R, Ringler DJ, Mackay CR, Daugherty BL, Springer MS, Durham SR, Williams TJ, Kay AB (1997). Enhanced expression of eotaxin and CCR3 mRNA and protein in atopic asthma. Association with airway hyperresponsiveness and predominant co-localization of eotaxin mRNA to bronchial epithelial and endothelial cells. Eur J Immunol 27:3507
Ying S, Meng Q, Zeibecoglou K, Robinson DS, Macfarlane A, Humbert M, Kay AB (1999). Eosinophil chemotactic chemokines (eotaxin, eotaxin-2, RANTES, monocyte chemoattractant protein-3 (MCP-3), and MCP-4), and C-C chemokine receptor 3 expression in bronchial biopsies from atopic and nonatopic (Intrinsic) asthmatics. J Immunol 163:6321
Yla-Herttuala S, Lipton BA, Rosenfeld ME, Sarkioja T, Yoshimura T, Leonard EJ, Witztum JL, Steinberg D (1991). Expression of monocyte chemoattractant protein 1 in macrophage-rich areas of human and rabbit atherosclerotic lesions. Proc. Natl Acad. Sci. U S A 88:5252
Yoshimura T, Robinson EA, Tanaka S, Appella E, Leonard EJ (1989). Purification and amino acid analysis of two human monocyte chemoattractants produced by phytohemagglutinin-stimulated human blood mononuclear leukocytes. J. Immunol. 142:1956
Yozai K, Shikata K, Sasaki M, Tone A, Ohga S, Usui H, Okada S, Wada J, Nagase R, Ogawa D, Shikata Y, Makino H (2005). Methotrexate prevents renal injury in experimental diabetic rats via anti-inflammatory actions. J. Am. Soc. Nephrol. 16:3326
Zimmet P, Alberti KG, Shaw J (2001). Global and societal implications of the diabetes epidemic. Nature 414:782

The features of the present invention disclosed in the specification, the claims and/or the drawings may both separately and in any combination thereof be material for realizing the invention in various forms thereof.

## Claims

1. An inhibitor of Monocyte Chemoattractant Protein-1 (CCL2) activity, for use in a method for treating a subject suffering from or being at risk of suffering from an eye disease, wherein the eye disease is a back of the eye disease selected from the group consisting of age-related macular degeneration (AMD), diabetic retinopathy (DR) and proliferative vitreoretinopathy (PVR), preferably the inhibitor of CCL2 activity is locally administered to the subject, systemically administered to the subject or locally and systemically administered to the subject.

2. The inhibitor of Monocyte Chemoattractant Protein-1 (CCL2) activity for use of claim 1, wherein local administration is intravitreal administration, suprachoroidal administration, subretinal administration or a combination thereof, preferably local administration is intravitreal administration, and systemic administration is selected from the group comprising subcutaneous administration and intravenous administration.

3. The inhibitor of Monocyte Chemoattractant Protein-1 (CCL2) activity for use of any one of claims 1 to 2, wherein the subject is suffering from or at risk of suffering from AMD, preferably AMD is selected from the group consisting of neovascular or wet AMD and atrophic dry AMD.

4. The inhibitor of Monocyte Chemoattractant Protein-1 (CCL2) activity for use of claim 3, wherein an agent is administered to the subject prior to, concomitantly with or subsequent to the administration of the inhibitor of CCL2 activity, wherein the agent is selected from the group comprising a VEGF inhibitor, an angiopoietin-2 inhibitor, a C3 complement inhibitor, a C5 complement inhibitor and a steroid.

5. The inhibitor of Monocyte Chemoattractant Protein-1 (CCL2) activity for use of any one of claims 1 to 2, wherein the subject is suffering from or at risk of suffering from DR, preferably DR is nonproliferative diabetic retinopathy (NPDR) or proliferative diabetic retinopathy (PDR).

6. The inhibitor of Monocyte Chemoattractant Protein-1 (CCL2) activity for use of claim 5, wherein DR goes along with or is associated with diabetic macular edema (DME).

7. The inhibitor of Monocyte Chemoattractant Protein-1 (CCL2) activity for use of any one of claims 5 to 6, wherein prior to the administration of the inhibitor of CCL2 activity the subject underwent a therapy selected from the group comprising anti-VEGF therapy, laser photoagglutination, vitreoretinal surgery or a combination thereof.

8. The inhibitor of Monocyte Chemoattractant Protein-1 (CCL2) activity for use of any one of claims 5 to 7, wherein an agent is administered to the subject prior to, concomitantly with or subsequent to the administration of the inhibitor of CCL2 activity, wherein the agent is selected from the group comprising an angiotensin converting enzyme inhibitor, a VEGF inhibitor, a CTGF inhibitor, a statin and a steroid, preferably the agent is concomitantly administered with the inhibitor of CCL2 activity.

9. The inhibitor of Monocyte Chemoattractant Protein-1 (CCL2) activity for use of any one of claims 1 to 2, wherein the subject is at risk of suffering from or is suffering from PVR, preferably the subject is at risk of suffering from PVR.

10. The inhibitor of Monocyte Chemoattractant Protein-1 (CCL2) activity for use of claim 9, wherein the subject is suffering from an ocular trauma, preferably the ocular trauma is retinal detachment repair surgery, wherein the subject undergoes the retinal detachment repair surgery prior to, concomitantly with or subsequent to administration of the inhibitor of CCL2 activity.

11. The inhibitor of Monocyte Chemoattractant Protein-1 (CCL2) activity for use of any one of claims 9 to 10, wherein an agent is administered to the subject prior to, concomitantly with or subsequent to the administration of the inhibitor of CCL2 activity, wherein the agent is selected from the group comprising an anti-inflammatory agent, an agent inhibiting cell proliferation and an anti-fibrotic agent, preferably the agent is selected from the group comprising a corticosteroid and methotrexate.

12. The inhibitor of Monocyte Chemoattractant Protein-1 (CCL2) activity for use of any one of claims 1 to 11, wherein the inhibitor of CCL2 activity is anti-fibrotic.

13. The inhibitor of Monocyte Chemoattractant Protein-1 (CCL2) activity for use of any one of claims 1 to 12, wherein the inhibitor of CCL2 activity is a nucleic acid molecule binding to CCL2, wherein the nucleic acid molecule comprises a nucleotide sequence of SEQ IQ NO: 37 or a nucleotide sequence having an identity of at least 85 % to the nucleotide sequence of SEQ ID NO: 37, preferably the nucleic acid comprises a modification, more preferably the modification comprises a polyethylene glycol (PEG) molecule having a molecular weight of about 40 kDa.

14. The inhibitor of Monocyte Chemoattractant Protein-1 (CCL2) activity for use of any one of claims 1 to 12, wherein the inhibitor of CCL2 activity is NOX-E36.
